(19) Europäisches Patentamt
European Patent Office
Office européen des brevets

(11) Publication number: **0 582 261 A1**

(12) **EUROPEAN PATENT APPLICATION**

(21) Application number: **93112374.9**

(51) Int. Cl.[5]: **C07D 501/00**, A61K 31/545

(22) Date of filing: **02.08.93**

(30) Priority: **01.08.92 KR 1388392**
**01.08.92 KR 1388492**

(43) Date of publication of application:
**09.02.94 Bulletin 94/06**

(84) Designated Contracting States:
**AT BE CH DE DK ES FR GB GR IE IT LI LU MC NL PT SE**

(71) Applicant: **LUCKY LTD.**
**20, Yoido-dong,**
**Yongdungpo-ku**
**Seoul 150-721(KR)**

(72) Inventor: **Kim, Yong Zu**
**Lucky Yeolip 203, 388-11, Doryong-Dong**
**Youseong-Ku, Daejeon(KR)**
Inventor: **Oh, Hun Seung**
**LuckyYeonlip 303, 388-11, Doryong-Dong**
**Youseong-Ku, Daejeon(KR)**
Inventor: **Nahm, Kee Pyung**
**Lucky Apt. 6-102, 381-42, Doryong-Dong**
**Youseong-Ku, Daejeon(KR)**
Inventor: **Yeo, Jae Hong**
**118-1, Shinsong-Dong**
**Youseong-Ku, Daejeon(KR)**
Inventor: **Lim, Jong Chan**
**Lucky Apt. A-104, 386-4, Doryong-Dong**
**Youseong-Ku, Daejeon(KR)**
Inventor: **Bang, Chan Sik**
**Lucky Dorm 210, 386-4, Doryong-Dong**
**Youseong-Ku, Daejeon(KR)**
Inventor: **Seo, Mi Kyeong**
**Lucky Dorm 424, 386-1, Doryong-Dong**
**Youseong-Ku, Daejeon(KR)**

(74) Representative: **Patentanwälte Grünecker, Kinkeldey, Stockmair & Partner**
**Maximilianstrasse 58**
**D-80538 München (DE)**

(54) **Cephalosporin compounds and processes for the preparation thereof.**

(57) The present invention relates to a cephalosporin compound including the pharmaceutically acceptable non-toxic salts, physiologically hydrolyzable esters, hydrates and solvates thereof of the formula(I) and its isomers:

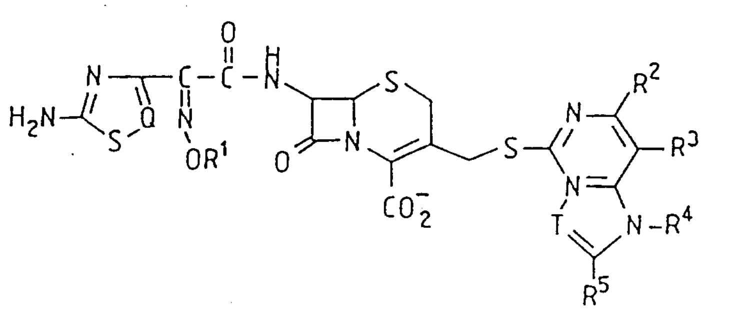

(I)

wherein:

$R^1$ is a $C_{1-4}$ alkyl, $C_{3-4}$ alkenyl, $C_{3-4}$ alkynyl or $-C(R^a)(R^b)-COOH$ wherein $R^a$ and $R^b$ are, independently of each other, a hydrogen or a $C_{1-4}$ alkyl group, respectively, or jointly form a $C_{3-7}$ cycloalkyl group together with the carbon atoms to which they are attached;

$R^2$ is a hydrogen or an amino which may be substituted or unsubstituted, alkoxy or heterocyclic group which is linked with a nitrogen atom;

EP 0 582 261 A1

| | |
|---|---|
| $R^3$ is | a hydrogen or a $C_{1-4}$ alkyl, amino, aminomethyl, hydroxy, hydroxymethyl, carboxy or carboxymethyl group; |
| $R^4$ is | a hydrogen or a $C_{1-4}$ alkyl, amino, hydroxy, hydroxyalkl, acetamide, carboxyalkyl or sulfonylethyl group; |
| $R^5$ is | a hydrogen or a $C_{1-4}$ alkyl, amino, carboxy, carboxyalkyl or hydroxy group; and |
| Q and T are | independently CH or N, provided that $R^2$ is an amino group when T is N. |

## Field of the Invention

The present invention relates to novel cephalosporin compounds, pharmaceutically acceptable non-toxic salts thereof, physiologically hydrolyzable esters, hydrates and solvates thereof and isomers thereof which possess potent and broad antibacterial activities. The invention also relates to processes for preparing these compounds and to pharmaceutical compositions containing them as active ingredients.

## Description of the Prior Art

Antibiotics of cephalosporin series are widely used in therapy for treatment of diseases which are caused by general pathogenic bacteria in human beings and animals. It has been known that such antibiotics are useful for the treatment of diseases caused by bacteria exhibiting resistance to other antibiotics, e.g., penicillin-resistant bacteria, and for the treatment of penicillin-sensitive patients.

In most circumstances, it is desirable to employ antibiotics possessed with broad antibacterial activities, e.g., against both Gram-positive and Gram-negative bacteria. In this regard, there were many studies made in developing a variety of cephalosporin antibiotics with such broad-spectrum antibiotic activities.

For example, GB Patent No. 1,399,086 discloses 7β-acylamido-ceph-3-em-4-carboxylic acids having the formula (A):

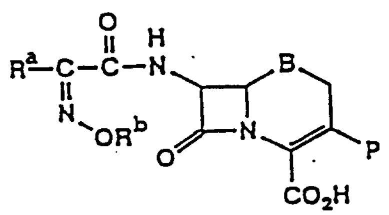

(A)

wherein:

$R^a$ is a hydrogen or an organic group;
$R^b$ is an etherifying monovalent organic group linked to the oxygen atom through a carbon atom;
B is S or S → O; and
P is an organic group.

Stimulated by the discovery of theses compounds, there followed many attempts to develop antibiotic compounds having improved properties with respect to certain microorganisms, especially against Gram-negative bacteria. Such efforts resulted in the development of, e.g., those compounds disclosed in GB Patent No. 1,522,140, which have the following formula(B) and exist as syn isomers or as a mixture of syn and anti isomers wherein the syn isomers are present in at least 90%:

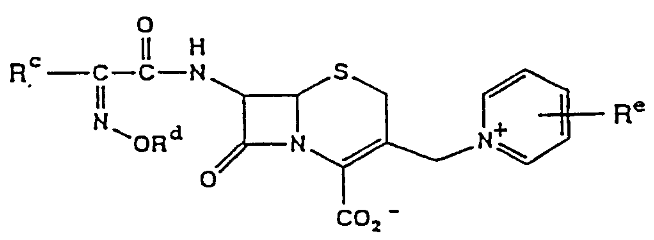

(B)

wherein:

$R^c$ is a furyl or thienyl group;
$R^d$ is a $C_{1-4}$ alkyl, $C_{3-7}$ cycloalkyl, furylmethyl or thienylmethyl group; and
$R^e$ is a hydrogen or a carbamoyl, carboxy, carboxymethyl, sulfonyl or methyl group.

Recently, further efforts were made to prepare new and improved antibiotics, typically by way of introducing an acylamido group into the 7-position and certain other groups into the 3-position of the cephem nucleus as shown in the foregoing formula(B).

For example, BE Patent No. 852,427 discloses cephalosporin compounds prepared by substituting $R^a$ in the formula(A) with various organic group including 2-aminothiazol-4-yl and by way of attaching an aliphatic hydrocarbon, which hydrocarbon may further be substituted with a carboxy group, to the oxygen atom in the oxyimino group.

European Patent Application No. 47,977 discloses cephalosporin compounds having the following formula(C):

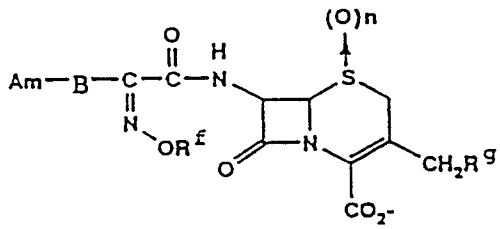

(C)

wherein:

n is 0 or 1;
Am is an optionally substituted amino group;
B is a thiadiazolyl group (which is attached to its neighbor group via its two carbon atoms);
$R^f$ is a hydrogen or a cycloalkyl, optionally substituted alkyl or carbamoyl group; and
$R^g$ is an optionally substituted thiazolium or pyrazolium,
$R^g$ is an optionally substituted thiazolium or pyrazolium, tri-(lower)alkyl ammonium, or pyridinium group having the formula of

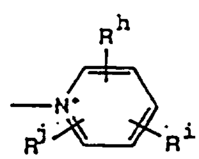

wherein:

$R^h$ is an (lower)alkyl substituted with cycloalkyl, methyl, hydroxy, alkoxy, halogen, cyano, carbamoyl, carboxy or sulfonyl, (lower)alkenyl or (lower) alkylthio optionally substituted with carboxy, amino optionally monosubstituted by (lower)alkyl, (lower)alkanoyl or aminobenzenesulfonyl, di(lower)-alkylamino, carbamoyl substituted with (lower)alkyl, hydroxy(lower)alkyl, (lower)alkoxy, hydroxy or cyano, di(lower)alkylcarbamoyl, thiocarbamoyl, cycloalkyl, phenyl, hydroxy, (lower)alkoxy, halogen, (lower)alkoxycarbonyl, (lower)alkanoyloxy, (lower)alkanoyl, carboxy, sulfocyano, nitro or hydroxysulfo(lower)alkyl group;
$R^i$ is a hydrogen or a carbamoyl or the same as $R^h$; and
$R^j$ is a hydrogen or the same as $R^h$.

U.S. Patent No. 4,390,534 discloses the cephem compounds of the formula(D):

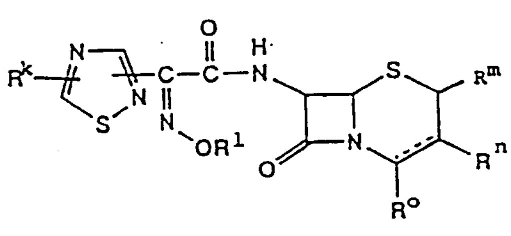

(D)

wherein:

$R^k$ is an amino or protected amino group;
$R^l$ is a hydrogen or an acyl, optionally substituted aryl, substituted alkyl, alkenyl, alkynyl, optionally

substituted cycloalkyl, cycloalkynyl or 5-membered heterocyclic ring containing oxygen or sulfur substituted with hydroxy;

$R^m$ is a hydrogen or an alkyl group;

$R^n$ is a hydrogen or an acyloxyalkyl, alkylthioalkyl, optionally substituted pyridinium alkyl, optionally substituted heterocyclic thioalkyl, alkyl, halogen, hydroxy or optionally substituted thiazolium alkyl group;

$R^o$ is a carboxy or protected carboxy group, provided that $R^o$ is a carboxy if $R^n$ is an optionally substituted pyridinium alkyl or optionally substituted thiazolium alkyl group; and the dotted line represents a single or double bond.

European Patent Appln. No. 150,507 describes cephalosporin compounds having the following formula-(E):

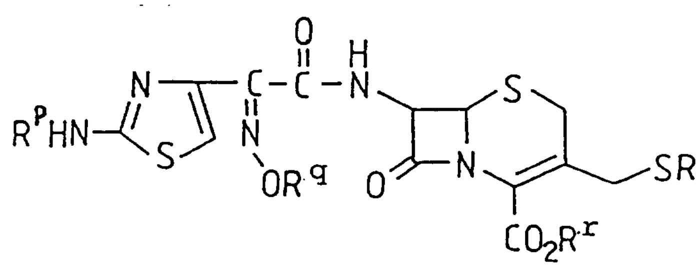

(E)

wherein:

R is a heterocyclic group, preferably triazolopyrimidyl or thiadiazolopyrimidyl group;

$R^p$ is a hydrogen or an amino protecting group;

$R^q$ is a hydrogen or a methyl, hydroxy protecting group or acyl group; and

$R^r$ is a hydrogen or a carboxy protecting group.

Further, Korean Patent Appln. Nos. 89-6431, 89-7827, 89-7828, 89-10755 and 90-1351 of the present inventor describe cephem compounds of formula(F):

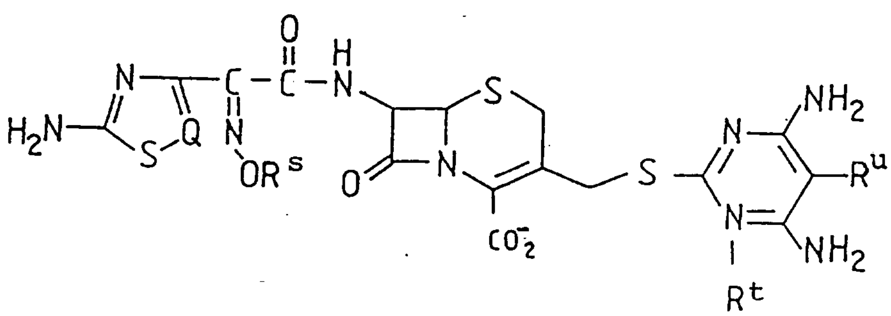

(F)

wherein,

$R^s$ is a $C_{1-4}$ alkyl, $C_{3-4}$ alkenyl, $C_{3-4}$ alkynyl or -C(R')(R'')COOH group wherein R' and R'', which may be the same or different, are independently a hydrogen or a $C_{1-4}$ alkyl group or form a $C_{3-7}$ cycloalkyl group together with the carbon atoms to which they are attached;

$R^t$ is a $C_{1-4}$ alkyl, $C_{3-4}$ alkenyl, $C_{3-4}$ alkynyl, $C_{3-4}$ cycloalkyl, substituted or unsubstituted amino or substituted or unsubstituted phenyl group;

$R^u$ is a hydrogen or a $C_{1-4}$ alkyl group; and

Q is CH or N.

However, these patent applications disclose a pyriminidium ring which is a single heterocycle, and not a heterocycle fused with two or more nuclei as a substitutent in the 3 position of the cephem.

## Summary of the Invention

The present inventors have carried out extensive research in search for a cephalosporin compound which has a broad spectrum of antibiotic activities; and, as a result, have discovered that cephalosporin compounds with a (1,2,8-substituted-7-amino[1,2,4] triazolo[1,5-c] pyrimidinium-5-yl)thiomethyl or (1,2,8-substituted-7-amino([1,3]-imidazo[1,2-c]pyrimidinium-5-yl)thiomethyl group in the 3 position and a specific group in the 7$\beta$ position of the cephem nucleus exhibit strong antibacterial activities in a broad spectrum.

Accordingly, a primary object of the present invention is to provide said novel cephalosporin compounds.

It is another object of the present invention to provide processes for preparing such compounds.

It is still another object of the present invention to provide pharmaceutical compositions containing same as active ingredients.

In accordance with one aspect of the present invention, there is provided with novel cephalosporin compounds of formula(I):

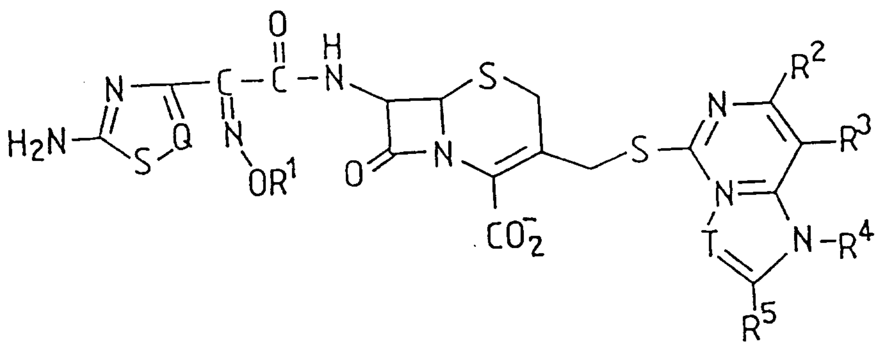 (I)

wherein:

- $R^1$ is a $C_{1-4}$ alkyl, $C_{3-4}$ alkenyl, $C_{3-4}$ alkynyl or $-C(R^a)(R^b)-COOH$ wherein $R^a$ and $R^b$ are, independently of each other, a hydrogen or a $C_{1-4}$ alkyl group, respectively, or jointly form a $C_{3-7}$ cycloalkyl group together with the carbon atoms to which they are attached;
- $R^2$ is a hydrogen or an amino which may be substituted or unsubstituted, alkoxy or heterocyclic group which is linked with a nitrogen atom;
- $R^3$ is a hydrogen or a $C_{1-4}$ alkyl, amino, aminomethyl, hydroxy, hydroxymethyl, carboxy or carboxymethyl group;
- $R^4$ is a hydrogen or a $C_{1-4}$ alkyl, amino, hydroxy, hydroxyalkyl, acetamide, carboxyalkyl or sulfonylethyl group;
- $R^5$ is a hydrogen or a $C_{1-4}$ alkyl, amino, carboxy, carboxyalkyl or hydroxy group; and
- Q and T are independently CH or N, provided that $R^2$ is an amino group when T is N.

In accordance with another aspect of the present invention, there is provided with processes for preparing the cephalosporin compounds of formula(I).

In accordance with a further aspect of the present invention, there is provided with pharmaceutically acceptable non-toxic salts, physiologically hydrolyzable esters, hydrates and solvates of the compounds of formula(I) and processes for preparing these compounds.

In accordance with a still further aspect of the present invention, there is provided with pharmaceutical compositions comprising one or more of the cephalosporin compounds represented by formula(I) and their pharmaceutically acceptable derivatives as an active ingredient.

## Detailed Description of the Invention

The novel cephalosporin compounds of formula(I) include both syn isomers and mixtures of syn and anti isomers, with respect to the radical, $-O-C(R^a)(R^b)COOH$, which mixtures contain at least 90% of the syn isomer or not more than 10% of the anti isomer.

The compounds of formula(I) also include the diastereomeric isomers and mixtures thereof when $R^a$ and $R^b$ are different from each other.

In addition, the compounds of formula(I) in accordance with the present invention may exist in tautomeric forms and such tautomers are also included within the scope of the invention. Namely, when $R^2$ is an amino group, $R^5$ is a hydroxy group and T is N, as shown below, 2-hydroxy[1,2,4]triazolo[1,5-c]-pyrimidinium-5-yl group undergoes tautomerism to form [1,2,4]triazol-3-one[1,5-c]pyrimidinium-5-yl group, its tautomer, as follows:

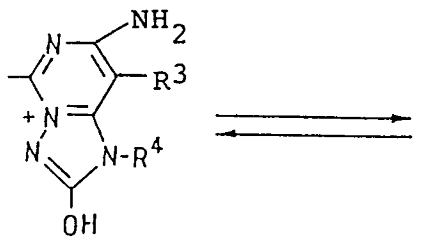

When $R^2$ is any group other than amino group, $R^5$ is a hydroxy group and T is CH, 2-hydroxy[1,3]imidazo-[1,2-c]pyriminium-5-yl group forms 2-hydroxy[1,3]imidazol-4-one[1,2-c]pyrimidinium-5-yl group, as its tautomer, as follows:

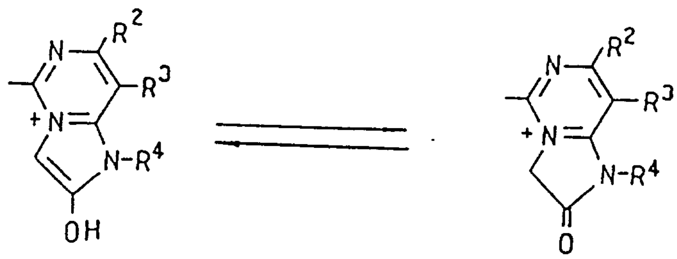

Among the compounds of the present invention, preferred are those wherein: $R^1$ is a methyl, ethyl, allyl, propargyl or -C($R^a$)($R^b$)COOH group wherein $R^a$ and $R^b$, which may be the same or different, are a hydrogen or a methyl or ethyl group or jointly form a cyclopentyl group together with the carbon atoms to which they are attached; $R^2$ is a hydrogen or an amino group; $R^3$ is a hydrogen or a methyl or amino group; $R^4$ is a methyl, ethyl, amino or carboxymethyl group; and $R^5$ is a hydrogen or a methyl, amino or hydroxy group.

More preferred compounds of formula (I) included in the present invention are those listed in Table 1.

Table 1: Preferred Species of Formula(I) compounds

| $R^1$ | $R^2$ | $R^3$ | $R^4$ | $R^5$ | Q | T |
|---|---|---|---|---|---|---|
| $-C(CH_3)_2CO_2H$ | $NH_2$ | -H | $-CH_3$ | -H | -CH | N |
| $-C(CH_3)_2CO_2H$ | $NH_2$ | -H | $-CH_3$ | $-NH_2$ | -CH | N |
| $-C(CH_3)_2CO_2H$ | $NH_2$ | $-CH_3$ | $-CH_3$ | $-NH_2$ | -CH | N |
| $-C(CH_3)_2CO_2H$ | $NH_2$ | $-CH_3$ | $-CH_3$ | -H | -CH | N |
| (S) $-\overset{*}{C}H(CH_3)CO_2H$ | $NH_2$ | -H | $-CH_3$ | -H | -CH | N |
| (R) $-\overset{*}{C}H(CH_3)CO_2H$ | $NH_2$ | -H | $-CH_3$ | $-NH_2$ | -CH | N |
| $-CH_2CH_3$ | $NH_2$ | -H | $-CH_3$ | $-NH_2$ | -CH | N |
| $-CH_3$ | $NH_2$ | -H | $-CH_3$ | -H | -CH | N |
| $-CH_3$ | $NH_2$ | -H | $-CH_2CO_2H$ | -H | -CH | N |
| $-CH_3$ | $NH_2$ | -H | $-CH_2CO_2H$ | $-NH_2$ | -CH | N |
| $-CH_2CH_3$ | $NH_2$ | -H | $-CH_3$ | -H | -N | N |
| 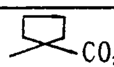 $CO_2H$ | $NH_2$ | -H | $-CH_3$ | -H | -CH | N |
| (R) $-\overset{*}{C}H(CH_2CH_3)CO_2H$ | $NH_2$ | -H | $-CH_3$ | -H | -CH | N |
| (R) $-\overset{*}{C}H(CH_2CH_3)CO_2H$ | $NH_2$ | -H | $-CH_3$ | $-NH_2$ | -CH | N |
| $-\overset{*}{C}CH_3(CH_2CH_3)CO_2H$ | $NH_2$ | -H | $-CH_3$ | $-NH_2$ | -CH | N |

Table 1(continued)

| $R^1$ | $R^2$ | $R^3$ | $R^4$ | $R^5$ | Q | T |
|---|---|---|---|---|---|---|
| $-C(CH_3)_2CO_2H$ | $NH_2$ | H | $CH_3$ | H | CH | CH |
| $-C(CH_3)_2CO_2H$ | $NHCH_3$ | H | $CH_3$ | H | CH | CH |
| $-C(CH_3)_2CO_2H$ | $NH_2$ | $CH_3$ | $CH_3$ | H | CH | CH |
| (S) $-\overset{*}{C}H(CH_3)CO_2H$ | $NH_2$ | H | $CH_3$ | H | CH | CH |
| (R) $-\overset{*}{C}H(CH_3)CO_2H$ | $NH_2$ | H | $CH_3$ | H | CH | CH |
| $-CH_2CO_2H$ | $NH_2$ | H | $CH_3$ | H | CH | CH |
| $-CH_3$ | $NH_2$ | H | $CH_3$ | H | CH | CH |
| $-CH_3$ | $NH_2$ | $CH_3$ | $CH_3$ | H | CH | CH |
| $-CH_2CH_3$ | $NH_2$ | H | $CH_3$ | H | CH | CH |
| (R) $-\overset{*}{C}H(CH_2CH_3)CO_2H$ | $NH_2$ | H | $CH_3$ | H | CH | CH |
| 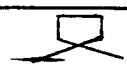 $CO_2H$ | $NH_2$ | H | $CH_3$ | H | CH | CH |
| $-\overset{*}{C}CH_3(CH_2CH_3)CO_2H$ | $NH_2$ | H | $CH_3$ | H | CH | CH |
| (S) $-\overset{*}{C}H(CH_3)CO_2H$ | $NH_2$ | $CH_3$ | $CH_3$ | H | CH | CH |

Furthermore, the present invention encompasses, within its scope, those pharmaceutically acceptable non-toxic salts, physiologically hydrolyzable esters, solvates and hydrates of the compounds of formula(I). Suitable pharmaceutically acceptable salts of the cephalosporin compounds(I) are conventional non-toxic salts and may include inorganic acid salts(e.g., hydrochloride, hydrobromide, sulfate, phosphate, etc.); organic carboxylic or sulfonic acid salts(e.g., formate, trifluoroacetate, citrate, acetate, maleate, tartrate, oxalate, succinate, benzoate, fumarate, mandelate, ascorbate, malate, methanesulfonate, p-toluenesulfonate, etc.); and also, depending on $R^1$, inorganic base salts such as alkali metal hydroxides(e.g., sodium hydroxide, potassium hydroxide, etc.), alkaline earth metal hydroxides (e.g., calcium hydroxide etc., ),

sodium bicarbonate, potassium bicarbonate, sodium carbonate, potassium carbonate, calcium carbonate, etc. or organic base salts including amino acids.

The pharmaceutically acceptable non-toxic salts may be prepared in accordance with known methods, e.g., by reacting the compounds of the formula(I) with one to four equivalents of corresponding acids or bases to the salts mentioned above in the presence of a solvent which may be water, or a mixture of water and water-miscible solvent (e.g., methanol, ethanol, acetonitrile, acetone, etc.).

The physiologically hydrolyzable esters of the compounds(I) may include, for example, indanyl, phthalidyl, methoxymethyl, pivaloyloxymethyl, glycyloxymethyl, phenylglycyloxymethyl or 5-methyl-2-oxo-1,3-dioxolan-4-yl esters, and other physiologically hydrolyzable esters which have been widely used in the technical fields of penicillin and cephalosporin antibiotics.

These esters can be prepared in accordance with known methods, e.g., by reacting the compounds of formula(I) with corresponding alkyl halides(e.g., methoxymethyl chloride) in the presence of a base(e.g., triethylamine, pyridine or sodium bicarbonate).

Exemplary solvates of the cephalosporin compounds of formula(I) may include solvates with water-miscible solvents, e.g., methanol, ethanol, acetone or acetonitrile; and more preferably, ethanol.

The present invention also includes within its scope pharmaceutical compositions comprising one or more of the compounds(I) as active ingredients, in association with pharmaceutically acceptable carriers, excipients or other additives, if necessary.

The antibiotic compounds(I) of the invention may be formulated for administration in unit dose or multi-dose containers. The compositions may take various forms such as solution, suspension or emulsion in an oily or aqueous vehicle, which may contain conventional additivies such as a dispersant, suspending agent, stabilizer and the like. Alternatively, the active ingredient may be formed into a dried powder that can be normally dissolved in an aqueous solution of sterile pyrogen-free water before use. The compounds(I) may be also formulated into suppositories containing conventional suppository bases such as cocoa butter or other glycerides.

The pharmaceutical compositions in a unit dose form may preferably comprise about 50 to 1,500mg of the active ingredient, depending on the age and body weight of the patient, the nature and severity of the illness, and so on. In general, it has been shown advantageous to administer the active compounds in an amount ranging from 500 to 5,000mg per day in order to achieve the desired results, depending on the routes and frequency of administration. In case of intramuscular or intravenous administration for adult human treatment, the dosage of about 150 to 3,000mg per day is thought to be sufficient, although it may vary in case of treatment for specific infections caused by certain strains.

If desired, the compounds(I) can be administered in combination with other antibiotics such as penicillin or other cephalosporins.

The compounds of the present invention, as described above, exhibit potent and broad antibacterial activities against Gram-positive bacteria and a variety of Gram-negative bacteria as well, particularly against Pseudomonas. Also, these compounds have high stability to $\beta$-lactamases produced by a number of Gram-negative bacteria.

A compound of formula(I) may be prepared by reacting a compound of formula(II) in the presence of a solvent, with a compound of formula(III) and removing the amino or carboxy protecting groups or reducing $S \rightarrow (O)_n$, if necessary:

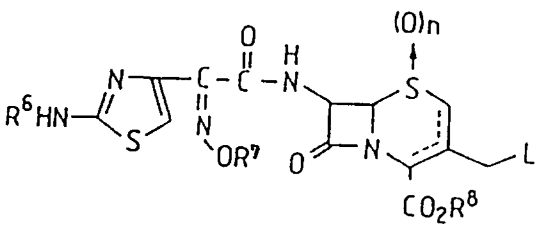

(II)

wherein:

n is 0 or 1;

$R^6$ is a hydrogen or an amino protecting group;

$R^7$ is a $C_{1-4}$ alkyl, $C_{3-4}$ alkenyl, $C_{3-4}$ alkynyl or $-C(R^a)(R^b)-COOR^c$ wherein $R^a$ and $R^b$, which may be the same or different, are independently a hydrogen or a $C_{1-4}$ alkyl group or form a $C_{3-7}$ cycloalkyl group together with the carbon atoms to which they are attached, and $R^c$ is a

hydrogen or a carboxy protecting group;

$R^8$ is a hydrogen or a carboxy protecting group; and

L is a leaving group; and

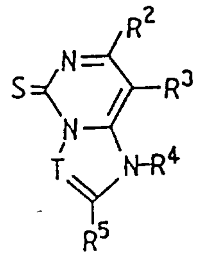

(III)

wherein: $R^2$, $R^3$, $R^4$, $R^5$ and T have the same meanings as defined above.

The amino protecting group in $R^6$ above may be any group which can be readily removed under the conventional mild conditions to allow the formation of free amino groups; and may include acyl, substituted or unsubstituted aryl(lower)alkyl(e.g., benzyl, diphenylmethyl and triphenylmethyl), (lower)alkoxyaryl(e.g., 4-methoxybenzyl), halo(lower)alkyl(e.g., trichloromethyl and trichloroethyl), tetrahydropyranyl, substituted phenylthio, substituted alkylidene, substituted aralkylidene or substituted cycloalkylidene. The acyl group appropriate for an amino protecting group may include, for example, $C_{1-6}$ alkanoyl(e.g., formyl and acetyl), $C_{2-6}$ alkoxycarbonyl(e.g., methoxycarbonyl and ethoxycarbonyl), (lower)alkanesulfonyl (e.g., methanesulfonyl and ethanesulfonyl), or aryl(lower) alkoxycarbonyl(e.g., benzyloxycarbonyl), where the acyl group can be substituted with 1-3 substituent(s) such as halogen, hydroxy, cyano or nitro. In addition, the amino protecting group may include reaction products obtained from amino groups and silane, boron or phosphorus compounds.

The carboxy protecting group of $R^c$ or $R^8$ may be any group which can be readily removed under the conventional mild conditions to allow the formation of free carboxy groups; and may include for example, (lower)alkylesters (e.g., methylester and t-butylester), (lower)alkenylesters(e.g., vinylester and allylester), (lower)alkoxy (lower)alkylesters(e.g., methoxymethylester), (lower) alkylthio(lower)alkylesters(e.g., methylthiomethylester), halo(lower)alkylesters(e.g.,2,2,2-trichloroethylester), substituted or unsubstituted aralkylesters(e.g., benzylester and p-nitrobenzylester), (lower) alkoxyaryl esters(e.g., p-methoxybenzylester) or silylesters, which can be selected by considering the chemical property of the desired compounds(I).

The leaving group L in formula(II) may include, for example, a halogen such as chlorine or fluorine, a (lower) alkanoyloxy group such as acetoxy, a (lower) alkanesulfonyloxy group such as methanesulfonyloxy, an arenesulfonyloxy group such as p-toluenesulfonyloxy, an alkoxycarbonyloxy group and the like.

The term "lower" as used hereinabove and elsewhere in this specification, for example, in reference to "lower alkyl", encompasses groups having 1 to 6 carbon atoms, more preferably, 1 to 4 carbon atoms.

The starting materials of the compounds(II) are known intermediates conventionally employed for the preparation of cephalosporin compounds. The dotted line of formula(II) represents a single or double bond; and, therefore, the compounds of formula(II) may be the compounds of formula(II-a), or the compounds of formula(II-b), or mixtures thereof:

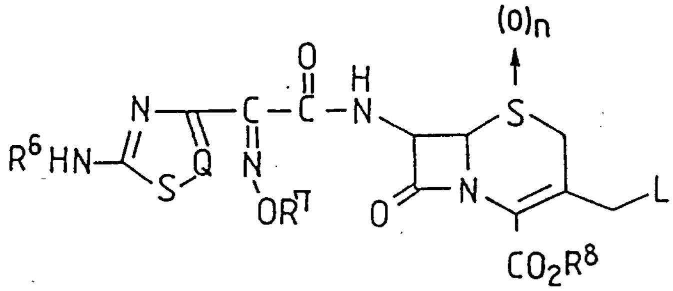

(II-a)

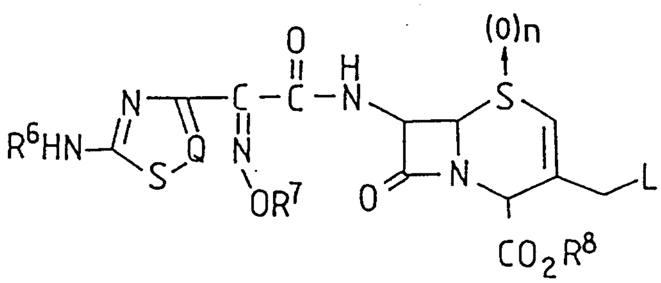

(II-b)

wherein:
n, $R^6$, $R^7$, $R^8$ and L have the same meanings as defined above.

The compounds of formula(II) may be prepared by activating the compounds of formula(IV) or salts thereof with an acylating agent and then reacting with the compounds of formula(V) in accordance with the following scheme(A).

Scheme A

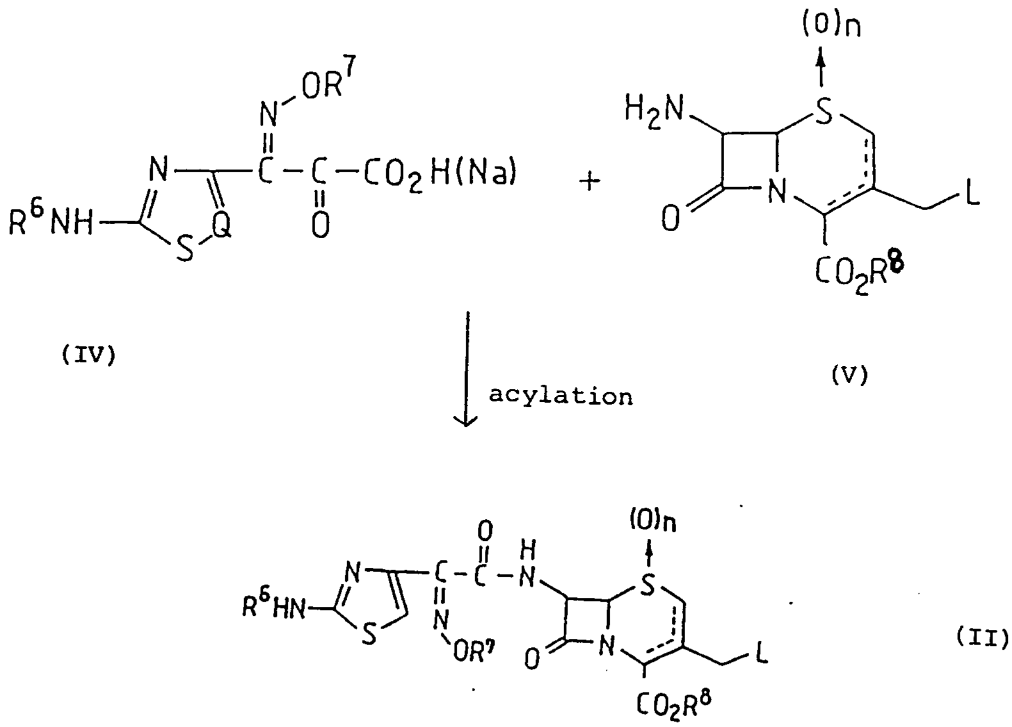


(IV) (V) (II)

wherein:
n, $R^6$, $R^7$, $R^8$ and L have the same meanings as defined above.

The dotted line of formula(V) represents a single or double bond; and therefore, the compounds of formula(V) may be the compounds of formula(V-a) or the compounds of formula (V-b), or mixtures thereof:

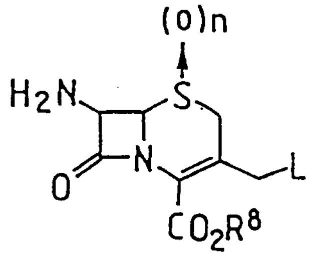

(V-a)

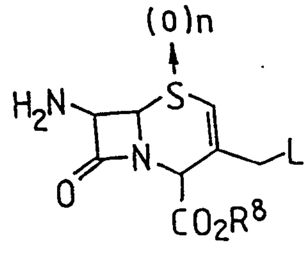

(V-b)

Another starting materials of compounds(III) may be prepared by the following scheme(B):

Scheme B

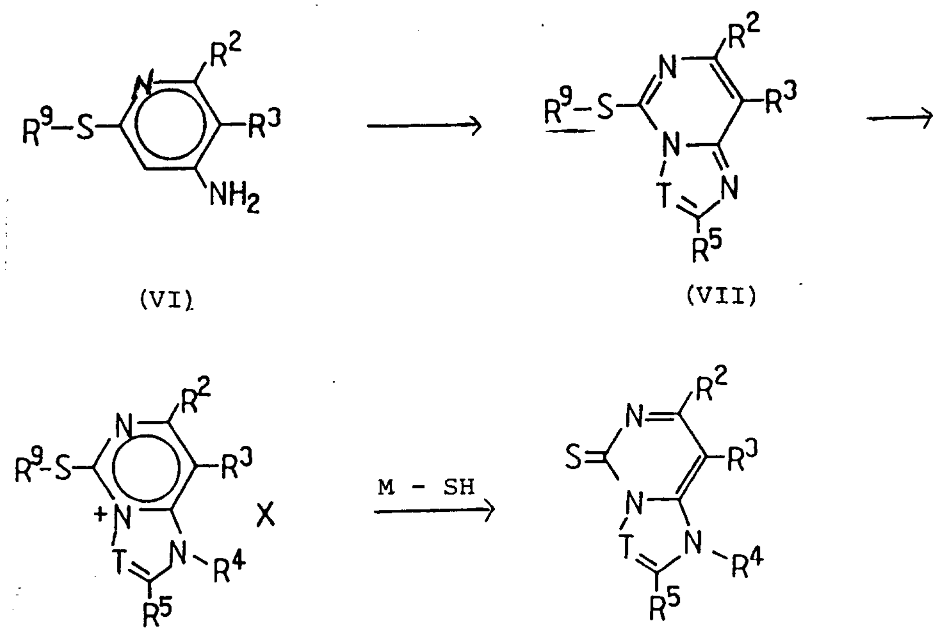


(VI) (VII)

(VIII) (III)

wherein:

$R^2$, $R^3$, $R^4$ and $R^5$ have the same meanings as defined above;
$R^9$ is a $C_{1-4}$ alkyl group;
X is an acid moiety such as a halogen atom or sulfate; and
M is an alkali metal atom such as lithium, sodium or potassium.

A compound of formula(VI) is subjected to a cyclization with a reagent which is suitably selected from the group consisting of acetaldehyde, methyl bromopyruvate, methyl-4-bromoacetoacetate, methyl bromoacetate and the like depending on the properties of a desired compound of formula(III). The reaction is carried out at a temperature of 0 to 150 °C, preferably 60 to 120 °C for 30 minutes to 48 hours, preferably 1 to 20 hours.

The compound(VII) is then reacted with a reagent which is suitably selected from the group consisting of alkyl halides such as methyliodide, ethylbromide or butyliodide, dialkylsulfates such as dimethylsulfate or

diethylsulfate, 2-iodoethanol, t-butylbromoacetate, O-alkyl(or aryl) sulfonylhydroxylamine and the like depending on the type of $R^4$ to be introduced. The reaction is carried out at a temperature of 0 to 150 ° C, preferably at a reflux temperature, for 1 to 48 hours, preferably 15 to 30 hours, in the presence of an inert solvent, preferably a mixture of water with a water-miscible polar solvent such as tetrahydrofuran, acetonitrile, alcohol and the like.

The compound(VIII) is reduced with an alkali metal hydrosulfide, preferably sodium hydrosulfide or potassium hydrosulfide. The reaction is carried out in the presence of an inert solvent, at a reflux temperature of the solvent until no bubble is generated. Preferred solvents include alcohols such as methanol or ethanol.

Amino or carboxy protecting groups can be readily removed by any of the conventional deprotection methods which are well known in the field of cephalosporin antibiotics. For example, acid- or base-hydrolysis or reduction is generally applicable.

Reduction of S-oxide can be conventionally carried out, for example, by adding potassium iodide and acetyl chloride to the reactants, followed by quenching the reaction mixture with sodium m-bisulfite.

The removal of amino or carboxy protecting group and reduction of S-oxide can be carried out before or after the reaction of the compound(II) with the compound(III).

The reaction for introducing the compound(III) into the 3-position of the compound(II) to prepare the compound(I) is carried out in the presence of a polar solvent such as N,N-dimethyl formamide, N,N-dimethylacetamide, dimethyl sulfoxide or methanol, wherein the temperature may range from 0 to 80 ° C, more preferably from 20 to 40 ° C; and the compound of the formula(III) is used in an amount of 0.5 to 2 molar equivalents, preferably 0.9 to 1.1 molar equivalents based on the compound of formula(II).

The separation and purification of the compound(I) can be carried out by using a conventional method such as recrystallization, silica gel column chromatography or ion-exchange chromatography, and the like.

The following Preparation Examples and Examples illustrate how some of the starting materials of formulas(II) and (III) and of the compounds of formula(I) can be prepared.

Preparation Example 1: Preparation of 7-amino[1,2,4] triazolo[1,5-c]pyrimidine-5-thione

A solution of 15.7g of 1,4,6-triamino-2(1H)-pyrimidinethione dissolved in 150ml of formic acid was heated under reflux and cooled to a room temperature. After the solvent was removed under a reduced pressure, 200ml of acetone was added to the residue to produce precipitates. The mixture was filtered; and the resulting solid was washed with 300ml of acetone and dried to yield 15.1g of the title compound as a white solid.

NMR(δ, DMSO-$d_6$): 5.90(s, 1H), 6.52(s, 2H), 8.19(s, 1H), 12.95(bs, 1H)
m.p.(melting point): >187 ° C(decomp.)

Preparation Example 2: Preparation of 7-amino-8-methyl [1,2,4]triazolo[1,5-c]pyrimidine-5-thione

The same procedures as described in Preparation Example 1 were repeated using a solution of 17.1g of 5-methyl-1,4,6-triamino-2(1H)-pyrimidinethione dissolved in 150ml of formic acid to yield 15.3g of the title compound as a white solid.

NMR(δ, DMSO-$d_6$): 2.10(s, 3H), 6.21(s, 2H), 8.12(s, 1H), 12.81(bs, 1H)
m.p.: >197 ° C(decomp.)

Preparation Example 3: Preparation of 2,7-diamino[1,2,4] triazolo[1,5-c]pyrimidine-5-thione

To a solution of 10g of 1,4,6-triamino-2(1H)-pyrimidinethione suspended in 500ml of distilled water was added 15g of bromonitrile; and the resulting solution was stirred for 24 hours at 70 ° C, cooled to a room temperature, adjusted to pH 7 to 8 with 1N sodium hydroxide solution and left at 0 ° C to 4 ° C for 6 hours. After the solution was filtered, the resulting solid was washed with 300ml of distilled water and 300ml of acetone in turn and dried to yield 12g of the title compound.

NMR(δ, DMSO-$d_6$): 5.83(s, 1H), 7.53(s, 2H), 7.85(s, 2H), 12.92(bs, 1H)
m.p.: >195 ° C(decomp.)

Preparation Example 4: Preparation of 7-amino-1-methyl [1,2,4]triazolo[1,5-c]pyrimidine-5-thione

To a solution of 16.9g of 7-amino[1,2,4]triazolo [1,5-c]pyrimidine-5-thione suspended in 100ml of distilled water and 30ml of tetrahydrofuran was added 4.4g of sodium hydroxide; and the resulting solution

was stirred until it became clean. After 17g of methyl iodide was added thereto, the mixture was stirred at a room temperature for 1 hour and filtered. The resulting solid was washed with 100ml of distilled water and dried in a vacuum oven to obtain 14.3g of a white solid. The solid so obtained was suspended in 200ml of tetrahydrofuran; and 30g of methyl iodide was added thereto. The reaction solution was heated to reflux for 40 hours, cooled to a room temperature and filtered. The resulting solid was washed with 400ml of acetone and dried to obtain 24.9g of a white solid. The solid so obtained was dissolved in 100ml of methanol; and to the solution was added 4.4g of 70% sodium hydrosulfide. The resulting solution was heated to reflux until no bubble was generated, cooled to a room temperature and filtered. The resulting solid was washed with 50ml of methanol and 30ml of distilled water in turn and dried to yield 5.7g of the title compound as a white solid.

NMR($\delta$, DMSO-$d_6$): 3.60(s, 3H), 5.75(s, 1H), 7.03(s, 2H), 8.62(bs, 1H)
m.p.: >195 °C(decomp.)

Preparation Example 5: Preparation of 7-amino-1,8-dimethyl[1,2,4]triazolo[1,5-c] pyrimidine-5-thione

The same procedures as descirbed in Preparation Example 4 were repeated using 18.1g of 7-amino-8-methyl[1,2,4] triazolo[1,5-c]pyrimidine-5-thione prepared in Preparation Example 2 as a starting material to yield 5.8g of the title compound as a white solid.

NMR($\delta$, DMSO-$d_6$): 2.19(s, 3H), 3.87(s, 3H), 6.79(s, 2H), 8.54(s, 1H)
m.p.: >200 °C(decomp.)

Preparation Example 6: Preparation of 2,7-diamino-1-methyl[1,2,4]triazolo[1,5-c] pyrimidine-5-thione

To a solution of 18.3g of 2,7-diamino[1,2,4] triazolo[1,5-c]pyrimidine-5-thione suspended in 150ml of distilled water and 40ml of tetrahydrofuran was added 4.5g of sodium hydroxide; and the resulting solution was stirred until it became clear. After 18g of methyl iodide was added thereto, the mixture was stirred at a room temperature for 1 hour and filtered. The resulting solid was washed with 200ml of distilled water and dried in a vacuum oven to obtain a white solid. The solid so obtained was suspended in 200ml of tetrahydrofuran; and 30g of methyl iodide was added thereto. The reaction solution was heated to reflux for 24 hours, cooled to a room temperature and filtered. The resulting solid was washed with 400ml of acetone and dried to obtain 26g of a white solid. The solid so obtained was dissolved in 100ml of methanol; and to the solution was added 4.6g of 70% sodium hydrosulfide. The resulting solution was heated to reflux until no bubble was generated, cooled to a room temperature and filtered. The solid was washed with 50ml of methanol and 30ml of distilled water in turn and dried to yield 6.2g of the title compound as a white solid.

NMR($\delta$, DMSO-$d_6$): 3.45(s, 3H), 6.15(s, 1H), 7.75(s, 2H), 7.83(s, 2H)
m.p.: >230 °C(decomp.)

Preparation Example 7: Preparation of 2,7-diamino-1,8-dimethyl[1,2,4]triazolo[1,5-c] pyrimidine-5-thione

To a solution of 12g of 5-methyl-1,4,6-triamino-2(1H)-pyrimidinethione suspended in 500ml of distilled water was added 16g of bromonitrile; and the resulting solution was stirred at 70 °C for 24 hours, cooled to a room temperature, adjusted to pH 7 to 8 with 1N sodium hydroxide solution and left at 0 °C to 4 °C for 6 hours. After the solution was filtered, the resulting solid was washed with 300ml of distilled water and 400ml of acetone in turn and dried to obtain 13.2g of a white solid. The solid so obtained was subjected to the same procedures as described in Preparation Example 4 to yield 6.4g of the title compound.

NMR($\delta$, DMSO-$d_6$): 2.15(s, 3H), 3.87(s, 3H), 7.82(s, 2H), 7.95(s, 2H)
m.p.: >281 °C(decomp.)

Preparation Example 8: Preparation of 7-amino-1-carboxymethyl[1,2,4]triazolo[1,5-c] pyrimidine-5-thione

To a solution of 14.3g of 7-amino[1,2,4]triazolo [1,5-c]pyrimidine-5-thione suspended in 100ml of distilled water and 30ml of tetrahydrofuran was added 3.5g of sodium hydroxide. To the resulting solution was added 18g of methyl iodide; and the mixture was stirred at a room temperature for 1 hour and filtered. The resulting solid was washed with 300ml of distilled water and dried in a vacuum oven to obtain a white solid. The solid so obtained was suspended in 200ml of tetrahydrofuran; and 30g of t-butylbromoacetate was added thereto. The reaction solution was heated to reflux for 24 hours, cooled to a room temperature and filtered. The resulting solid was washed with 300ml of acetone and dried to obtain 22g of a white solid. The solid so obtained was dissolved in 100ml of methanol; and to the resulting solution was added 4.5g of

70% sodium hydrosulfide. The mixture was heated to reflux until no bubble was generated, cooled to a room temperature and filtered. The solid was washed with 50ml of methanol and 50ml of distilled water in turn and dried to yield 6.4g of the title compound as a white solid.

NMR($\delta$, DMSO-$d_6$): 4.92(s, 2H), 5.87(s, 1H), 7.43(s, 2H), 8.52(s, 1H)
m.p.: >215 ° C(decomp.)

Preparation Example 9: Preparation of 7-amino-8-methyl-1-carboxymethyl[1,2,4]triazolo[1,5-c]pyrimidine-5-thione

To a solution of 15.4g of 7-amino-8-methyl[1,2,4] triazolo[1,5-c]pyrimidine-5-thione suspended in 100ml of distilled water and 30ml of tetrahydrofuran was added 3.5g of sodium hydroxide; and the resulting solution was stirred until it became clear. Thereafter, the solution was subjected to the same procedures as described in Preparation Example 8 to yield 7.2g of the title compound as a white solid.

NMR($\delta$, DMSO-$d_6$): 2.15(s, 3H), 4.84(s, 2H), 7.42(s, 2H), 8.49(s, 1H)
m.p.: >240 ° C(decomp.)

Preparation Example 10: Preparation of 7-amino-1,2-dimethyl[1,2,4]triazolo[1,5-c] pyrimidine-5-thione

To a solution of 8.6g of 1,4,6-triamino-2(1H)-pyrimidinethione dissolved in 50ml of acetic acid was added 10.2g of acetic anhydride; and the resulting solution was refluxed for 24 hours and filtered. The resulting solid was washed with 100ml of acetic acid and 200ml of acetone in turn and suspended in 100ml of distilled water. After 4g of sodium hydroxide was added dropwise slowly to the suspension, 20ml of tetrahydrofuran and then 7.1g of methyl iodide were added to the mixture; and the resulting solution was stirred for 1 hour, and filtered. The resulting solid was washed with 200ml of distilled water and 300ml of acetone, dried and dissolved in 50ml of tetrahydrofuran. To the solution was added 10g of methyl iodide; and the mixture was refluxed for 12 hours and filtered. The resulting solid was washed with 300ml of acetone and dried to obtain 13.2g of a solid. The solid was dissolved in 50ml of methanol; and to the solution was added 6g of 70% sodium hydrosulfide. The reaction solution was heated under reflux for 2 hours and cooled to a room temperature and filtered. The resulting solid was washed with 200ml of acetone and 200ml of distilled water to yield 5.3g of the title compound as a white solid.

NMR($\delta$, DMSO-$d_6$): 2.47(s, 3H), 3.63(s, 3H), 5.95(s, 1H), 6.68(s, 2H)
m.p.: >250 ° C(decomp.)

Preparation Example 11: Preparation of 7-amino-1,2,8-trimethyl[1,2,4]triazolo[1,5-c] pyrimidine-5-thione

To a solution of 7.4g of 5-methyl-1,4,6-triamino-2(1H)-pyrimidinethione dissolved in 50ml of acetic acid was added 9.5g of acetic anhydride. Thereafter, the reaction solution was subjected to the same procedures as described in Preparation Example 10 to yield 4.8g of the title compound as a white solid.

NMR($\delta$, DMSO-$d_6$): 2.02(s, 3H), 2.42(s, 3H), 3.67(s, 3H), 6.68(s, 2H)
m.p.: >245 ° C(decomp.)

Preparation Example 12: Preparation of 7-amino-1-hydroxyethyl[1,2,4]triazolo[1,5-c] pyrimidine-5-thione

To a solution of 16.9g of 7-amino[1,2,4]triazolo [1,5-c]pyrimidine-5-thione suspended in 100ml of distilled water and 30ml of tetrahydrofuran was added 4.4g of sodium hydroxide; and the resulting solution was stirred until it became clear. To the solution was added 20g of 2-iodoethanol; and the mixture was stirred at a room temperature for 1 hour and subjected to the same procedures as described in Preparation Example 4 to yield 6.1g of the title compound as a white solid.

NMR($\delta$, DMSO-$d_6$): 3.32(t, 2H), 3.94(t, 2H), 4.82(bs, 1H), 5.84(s, 1H), 7.27(s, 2H), 8.51(s, 1H)
m.p.: >270 ° C(decomp.)

Preparation Example 13: Preparation of 7-amino-2-methyl-1-carboxymethyl[1,2,4]triazolo [1,5-c]pyrimidine-5-thione

To a solution of 8.6g of 1,4,6-triamino-2(1H)-pyrimidinethione dissolved in 50ml of acetic acid was added 10.2g of acetic anhydride; and the resulting solution was heated under reflux for 24 hours and filtered. The resulting solid was washed with 150ml of acetic acid and 300ml of acetone in turn and suspended in 100ml of distilled water. After 4g of sodium hydroxide was added dropwise slowly to the

suspension, 20ml of tetrahydrofuran and then 7.1g of methyl iodide were added to the mixture; and the resulting solution was stirred for 1 hour and filtered. The resulting solid was washed with 300ml of distilled water and 400ml of acetone, dried and dissolved in 50ml of tetrahydrofuran. To the solution was added 15g of t-butylbromoacetate; and the mixture was heated to reflux for 12 hours and filtered. The resulting solid was washed with 300ml of acetone and dried to yield 14.3g of a solid. The solid was dissolved in 50ml of methanol; and to the solution was added 6g of 70% sodium hydrosulfide. The reaction solution was heated to reflux for 2 hours and cooled to a room temperature and filtered. The resulting solid was washed with 300ml of acetone and 300ml of distilled water to yield 5.3g of the title compound as a white solid.

NMR($\delta$, DMSO-$d_6$): 2.43(s, 3H), 4.84(s, 2H), 5.25(s, 1H), 7.21(s, 2H)
m.p.: >242 °C(decomp.)

Preparation Example 14: Preparation of 2,7-diamino-1-carboxymethyl[1,2,4]triazolo [1,5-c]pyrimidine-5-thione

A solution of 18.3g of 2,7-diamino[1,2,4]triazolo [1,5-c]pyrimidine-5-thione obtained in Preparation Example 3 suspended in 150ml of distilled water and 40ml of tetrahydrofuran was added 4.5g of sodium hydroxide; and the resulting solution was stirred until it became clear. After 18g of methyliodide was added, the reaction solution was stirred at a room temperature for 1 hour and filtered. The resulting solid was washed with 200ml of distilled water and dried to obtain a white solid. After the solid was suspended in 200ml of tetrahydrofuran, 32g of t-butylbromoacetate was added to the suspension; and the resulting mixture was heated to reflux for 24 hours and subjected to the same procedures as described in Preparation Example 6 to yield 6.7g of the title compound as a white solid.

NMR($\delta$, DMSO-$d_6$): 4.94(s, 2H), 5.42(s, 1H), 7.75(s, 2H), 7.97(s, 2H)
m.p.: >260 °C(decomp.)

Preparation Example 15: Preparation of 2,7-diamino-1-hydroxyethyl[1,2,4]triazolo [1,5-c]pyrimidine-5-thione

The same procedures as described in Preparation Example 14 were repeated using 27g of 2-iodoethanol instead of t-butylbromoacetate to yield 6.4g of the title compound as a white solid.

NMR($\delta$, DMSO-$d_6$): 3.51(t, 2H), 4.01(t, 2H), 4.91(bs, 1H), 5.36(s, 1H), 7.82(s, 2H), 8.02(s, 2H)
m.p.: >246 °C(decomp.)

Preparation Example 16: Preparation of 7-amino-1-methyl [1,2,4]triazol-3-one[1,5-c] pyrimidine-5-thione

To a solution of 10g of 1,4,6-triamino-2(1H)-pyrimidinethione dissolved in 20ml of dimethylformamide was added 11g of methyliodide; and the resulting solution was stirred at a room temperature for 2 hours. After 50ml of diethyl ether was added thereto, the mixture was filtered; and the resulting solid was washed with 400ml of acetone and dried to obtain 10.4g of a white powdery solid. The solid was dissolved in 20ml of dimethylformamide; and to the resulting solution was added 5g of carbonyldiimidazole. The mixture was stirred at a room temperature for 1 hour; and 60ml of diethylether was added thereto. The reaction solution was filtered; and the resulting solid was washed well with 100ml of methylenedichloride and dried to obtain 9.7g of 7-amino-5-methylthio[1,2,4]triazolo-3-one[1,5-c]pyrimidine as a white powdery solid. The solid so obtained was dissolved in 200ml of tetrahydrofuran; 25g of methyliodide was added to the resulting solution; and thereafter the solution was heated to reflux for 12 hours, cooled to a room temperature and filtered. The resulting solid was washed with 400ml of acetone and dried to obtain 23g of a white solid. The solid so obtained was dissolved in 100ml of methanol; and 4.4g of 70% sodium hydrosulfide was added to the resulting solution. The mixture was heated to reflux until no bubble was generated, cooled to a room temperature and filtered. The resulting solid was washed with 50ml of methanol and 30ml of distilled water in turn and dried to yield 5.7g of the title compound as a white solid.

NMR($\delta$, DMSO-$d_6$): 3.82(s, 3H), 5.31(s, 1H), 7.24(s, 2H)
m.p.: >260 °C(decomp.)

Preparation Example 17: Preparation of 7-amino-1-carboxymethyl[1,2,4]triazol-3-one[1,5-c] pyrimidine-5-thione

9.5g of the 7-amino-5-methylthio[1,2,4]triazol-3-one[1,5-c]pyrimidine obtained in Preparation Example 16 as an intermediate was dissolved in 200ml of tetrahydrofuran; 30g of t-butylbromoacetate was added thereto; and thereafter, the solution was heated to reflux for 30 hours, cooled to a room temperature and

filtered. The resulting solid was washed with 200ml of acetone and dried to obtain 8.4g of a white solid. The solid so obtained was dissolved in 100ml of methanol; and 4.4g of 70% sodium hydrosulfide was added to the resulting solution. The mixture was heated to reflux for 2 hours, cooled to a room temperature and filtered. The resulting solid was washed with 200ml of methanol and 200ml of distilled water in turn and dried to yield 5.6g of the title compound as a white solid.

NMR(δ, DMSO-$d_6$): 4.97(s, 2H), 5.29(s, 1H), 7.32(s, 2H)
m.p.: >255 °C(decomp.)

Preparation Example 18: Preparation of 7-amino-1-hydroxyethyl[1,2,4]triazol-3-one[1,5-c] pyrimidine-5-thione

The same procedures as described in Preparation Example 17 were repeated using 27g of 2-iodoethanol instead of t-butylbromoacetate to yield 5.3g of the title compound as a white solid.

NMR(δ, DMSO-$d_6$): 3.6(t, 3H), 4.1(t, 3H), 5.27(s, 1H), 7.36(s, 2H)
m.p.: >235 °C(decomp.)

Preparation Example 19: Preparation of 7-amino-1,8-dimethyl[1,2,4]triazol-3-one[1,5-c]pyrimidine-5-thione

The same procedures as described in Preparation Example 16 were repeated using 5-methyl-1,4,6-triamino-2(1H)-pyrimidinethione instead of 1,4,6-triamino-2(1H)-pyrimidinethione to yield 5.9g of the title compound as a white solid.

NMR(δ, DMSO-$d_6$): 1.83(s, 3H), 3.76(s, 3H), 6.68(s, 2H)
m.p.: >205 °C(decomp.)

Preparation Example 20: Preparation of 7,8-diamino-1-methyl[1,2,4]triazolo[1,5-c] pyrimidine-5-thione

A solution of 17.1g of 1,4,5,6-tetraamino-2(1H)-pyrimidinethione dissolved in 150ml of formic acid was heated under reflux for 12 hours and cooled to a room temperature. After the solvent was removed under a reduced pressure, to the residue was added 200ml of acetone to produce precipitates. The solution was filtered; and the resulting solid was washed with 500ml of acetone and dried to obtain 16.9g of a white solid. The solid so obtained was subjected to the same procedures as described in Preparation Example 4 to yield 5.4g of the title compound as a white solid.

NMR(δ, DMSO-$d_6$): 3.81(s, 3H), 7.12(s, 2H), 8.20(s, 2H), 8.57(s, 1H)
m.p.: >215 °C(decomp.)

Preparation Example 21: Preparation of 7,8-diamino-1,2-dimethyl[1,2,4]triazolo[1,5-c] pyrimidine-5-thione

The same procedures as described in Preparation Example 10 were repeated using 9.3g of 1,4,5,6-tetraamino-2(1H)-pyrimidinethione instead of 1,4,6-triamino-2(1H)-pyrimidinethione to yield 5.7g of the title compound as a white solid.

NMR(δ, DMSO-$d_6$): 2.51(s, 3H), 3.71(s, 3H), 7.04(s, 2H), 7.56(s, 2H)
m.p.: >205 °C(decomp.)

Preparation Example 22: Preparation of 1-methyl-2,7,8-triamino[1,2,4]triazolo[1,5-c] pyrimidine-5-thione

To a solution of 12g of 1,4,5,6-tetraamino-2(1H)-pyrimidinethione suspended in 50ml of distilled water was added 15g of bromonitrile; and the resulting solution was stirred at 70 °C for 24 hours, cooled to a room temperature, adjusted to pH 7 to 8 and left at 0 to 4 °C for 6 hours to produce precipitates. The solution was filtered; and the resulting solid was washed with 300ml of distilled water and 500ml of acetone and dried to obtain 12.7g of a white solid. The solid so obtained was subjected to the same procedures as described in Preparation Example 6 to yield 6.1g of the title compound as a white solid.

NMR(δ, DMSO-$d_6$): 3.52(s, 3H), 7.02(s, 2H), 7.43(s, 2H), 7.82(s, 2H)
m.p.: >220 °C(decomp.)

Preparation Example 23: Preparation of 7,8-diamino-1-methyl[1,2,4]triazol-3-one[1,5-c]pyrimidine-5-thione

The same procedures as described in Preparation Example 16 were repeated using 11g of 1,4,5,6-tetraamino-2(1H)-pyrimidinethione instead of 1,4,6-triamino-2(1H)-pyrimidinethione to yield 5.3g of the title

compound as a white solid.

NMR(δ, DMSO-$d_6$): 3.62(s, 3H), 7.21(s, 2H), 7.48(s, 2H)
m.p.: >215 °C(decomp.)

Preparation Example 24: Preparation of 7-amino-1-methyl-8-hydroxy[1,2,4]triazolo[1,5-c] pyrimidine-5-thione

A solution of 16.2g of 1,4,6-triamino-5-hydroxy-2(1H)-pyrimidinethione dissolved in 150ml of formic acid was heated to reflux for 12 hours and cooled to a room temperature. After the solvent was removed under a reduced pressure, 200ml of acetone was added to the residue; and the resulting solution was filtered. The solid was washed with 300ml of acetone and dried to obtain 16.8g of a solid. The solid so obtained was subjected to the same procedures as described in Preparation Example 4 to obtain 5.1g of the title compound as a white solid.

NMR(δ, DMSO-$d_6$): 3.65(s, 3H), 7.22(s, 2H), 8.47(s, 1H), 11.24(bs, 1H)
m.p.: >230 °C(decomp.)

Preparation Example 25: Preparation of 2,7-diamino-1-methyl-8-hydroxy[1,2,4]triazolo [1,5-c]pyrimidine-5-thione

To a solution of 10.5g of 1,4,6-triamino-5-hydroxy-2(1H)-pyrimidinethionesuspended in 500ml of distilled water was added 15g of bromonitrile; and the resulting solution was stirred at 70 °C for 20 hours, cooled to a room temperature, adjusted to pH 7 to 8 with 1N sodium hydroxide solution and left at 0 to 4 °C for 6 hours to produce precipitates. The solution was filtered; and the resulting solid was washed with 300ml of distilled water and 300ml of acetone in turn and dried to obtain 11.4g of a solid. The solid so obtained was subjected to the same precedures as described in Preparation Example 6 to yield 5.2g of the title compound as a white solid.

NMR(δ, DMSO-$d_6$): 3.58(s, 3H), 7.41(s, 2H), 7.96(s, 2H), 10.89(bs, 1H)
m.p.: >240 °C(decomp.)

Preparation Example 26: Preparation of 7-amino-1-methyl-8-hydroxy[1,2,4]triazol-3-one [1,5-c]pyrimidine-5-thione

The same procedures as described in Preparation Example 16 were repeated using 11g of 1,4,6-triamino-5-hydroxy-2(1H)-pyrimidinethione instead of 1,4,6-triamino-2(1H)-pyrimidinethione to yield 5.4g of the title compound as a white solid.

NMR(δ, DMSO-$d_6$): 3.64(s, 3H), 8.04(s, 2H), 11.2(bs, 1H), 11.7(bs, 1H)
m.p.: >235 °C(decomp.)

Preparation Example 27: Preparation of 7-amino-2-methoxy-1-methyl[1,2,4]triazolo[1,5-c] pyrimidine-5-thione

To a solution of 18.6g of 2-methylthio-1,4,6-triamino pyrimidine dissolved in 50ml of dimethylformamide was added 10.2g of carbonyldiimidazole; and the resulting solution was stirred at a room temperature for 1 hour. After adding 200ml of diethyl ether thereto, the solution was filtered; and the resulting solid was washed with 100ml of dichloromethane and 200ml of distilled water and dried. The solid so obtained was suspended in 100ml of distilled water and stirred until it became clear. To the solution was added dropwise slowly 4g of sodium hydroxide and added 17g of methyliodide. The resulting solution was stirred for 30 minutes and filtered; and the resulting solid was washed with 400ml of distilled water and dried to obtain 18.4g of a solid. The solid was dissolved in 150ml of tetrahydrofuran; 30g of methyliodide was added thereto; and the mixture was heated to reflux for 10 hours, cooled to a room temperature and filtered. The resulting solid was washed with 500ml of acetone and dried to obtain 25.4g of a white solid. The solid so obtained was dissolved in 100ml of methanol; 9g of 70% sodium hydrosulfide was added thereto; and the mixture was heated to reflux for 2 hours, cooled to a room temperature and filtered. The resulting solid was washed with 100ml of methanol and 100ml of distilled water in turn and dried to yield 9.7g of the title compound as a white powder.

NMR(δ, DMSO-$d_6$): 3.67(s, 3H), 4.01(s, 3H), 5.86(s, 1H), 7.03(s, 2H)
m.p.: >227 °C(decomp.)

Preparation Example 28: Preparation of 7-amino-1,8-dimethyl-2-methoxy[1,2,4] triazolo[1,5-c]pyrimidine-5-thione

The same procedures as described in Preparation Example 27 were repeated using 20.1g of 5-methyl-2-methylthio-1,4,6-triaminopyrimidine instead of 2-methylthio-1,4,6-triamino-pyrimidine to yield 8.7g of the title compound as a white powder.

NMR(δ, DMSO-$d_6$): 2.04(s, 3H), 3.54(s, 3H), 3.99(s, 3H), 7.12(s, 2H)
m.p.: >218 °C(decomp.)

Preparation Example 29: Preparation of 1-methyl[1,3] imidazo[1,2-c]pyrimidine-5-thione

To a solution of 14.1g of 4-amino-2-methylthio pyrimidine suspended in 200ml of distilled water was added 8.2g of sodium acetate and 32g of 50% chloroacetaldehyde while heating at 100 °C. The reaction solution was stirred for 30 minutes and cooled to a room temperature. After water was removed under a reduced pressure, to the residue was added 200ml of tetrahydrofuran; and the resulting solution was filtered to remove undissolved materials. To the filtrate was added 20g of methyl iodide; and the resulting mixture was heated with stirring for 10 hours, cooled to a room temperature and then filtered. The resulting solid was washed with 50ml of tetrahydrofuran and dried to obtain 17.8g of a solid; and the solid was suspended in 100ml of distilled water. To the suspension was added 8g of 70% sodium hydrosulfide; and the resulting mixture was heated at 70-80 °C with stirring until bubbles were not generated. The reaction solution was cooled to a room temperature, neutralized with concentrated hydrochloric acid and then filtered. The resulting solid was washed with 20ml of distilled water and 50ml of acetone and dried to yield 5.8g of the title compound as a white solid.

NMR(δ, DMSO-$d_6$): 3.83(s, 3H), 7.19(d, 1H), 7.89(d, 1H), 8.03(d, 1H), 8.22(d, 1H)
m.p.: >232 °C(decomp.)

Preparation Example 30: Preparation of 7-amino-1-methyl [1,3]imidazo[1,2-c]pyrimidine-5-thione

The same procedures as described in Preparation Example 29 were repeated using 15.6g of 4,6-diamino-2-methylthio pyrimidine as a starting material to yield 4.7g of the title compound.

NMR(δ, DMSO-$d_6$): 3.55(s, 3H), 5.72(s, 1H), 6.73(s, 2H), 7.39(d, 1H), 7.82(d, 1H)
m.p.: 241 °C(decomp.)

Preparation Example 31: Preparation of 1-methyl-7-methyl-amino[1,3]imidazo[1,2-c] pyrimidine-5-thione

The same procedures as described in Preparation Example 29 were repeated using 17g of 4-amino-6-methylamino-2-methylthio pyrimidine as a starting material to yield 5.7g of the title compound.

NMR(δ, DMSO-$d_6$): 2.76(d, 3H), 3.60(s, 3H), 5.77(s, 1H), 7.17(q, 1H), 7.38(d, 1H), 7.82(d, 1H)
m.p.: >267 °C(decomp.)

Preparation Example 32: Preparation of 7-dimethylamino-1-methyl[1,3]imidazo[1,2-c] pyrimidine-5-thione

The same procedures as described in Preparation Example 29 were repeated using 18.4g of 4-amino-6-dimethylamino-2-methylthio pyrimidine as a starting material to yield 6.9g of the title compound.

NMR(δ, DMSO-$d_6$): 3.08(s, 6H), 3.58(s, 3H), 5.97(s, 1H), 7.41(d, 1H), 7.80(d, 1H)
m.p.: >257 °C(decomp.)

Preparation Example 33: Preparation of 7-morpholine-1-methyl[1,3]imidazo[1,2-c] pyrimidine-5-thione

The same procedures as described in Preparation Example 29 were repeated using 22.6g of 4-amino-6-morpholine-2-methylthio pyrimidine as a starting material to yield 8.3g of the title compound.

NMR(δ, DMSO-$d_6$): 3.55-3.71(m, 8H), 3.61(s, 3H), 6.25 (s, 1H), 7.45(d, 1H), 7.82(d, 1H)
m.p.: >247 °C(decomp.)

Preparation Example 34: Preparation of 7-methoxy-1-methyl [1,3]imidazo[1,2-c]pyrimidine-5-thione

The same procedures as described in Preparation Example 29 were repeated using 17.1g of 4-amino-6-methoxy-2-methylthio pyrimidine as a starting material to yield 4.1g of the title compound.

NMR(δ, $DMSO-d_6$): 3.59(s, 3H), 4.07(s, 3H), 5.99(s, 1H), 7.41(d, 1H), 7.83(d, 1H)
m.p.: >221 °C(decomp.)

Preparation Example 35: Preparation of 7,8-diamino-1-methyl[1,3]imidazo[1,2-c] pyrimidine-5-thione

The same procedures as described in Preparation Example 29 were repeated using 17.1g of 2-methylthio-4,5,6-triamino pyrimidine as a starting material to yield 6.9g of the title compound.
NMR(δ, $DMSO-d_6$): 3.57(s, 3H), 4.23(s, 2H), 6.87(s, 2H), 7.37(d, 1H), 7.81(d, 1H)
m.p.: >259 °C(decomp.)

Preparation Example 36: Preparation of 7-amino-1,8-dimethyl[1,3]imidazo[1,2-c] pyrimidine-5-thione

The same procedures as described in Preparation Example 29 were repeated using 17g of 4,6-diamino-5-methyl-2-methylthio pyrimidine as a starting material to yield 6.3g of the title compound.
NMR(δ, $DMSO-d_6$): 1.97(s, 3H), 3.63(s, 3H), 6.97(s, 2H), 7.18(d, 1H), 7.40(d, 1H)
m.p.: >255 °C(decomp.)

Preparation Example 37: Preparation of 2-ethyl[1,3] imidazo[1,2-c]pyrimidine-5-thione

To a solution of 14.1g of 4-amino-2-methylthio pyrimidine suspended in 200ml of distilled water was added 8.2g of sodium acetate and 32g of 50% chloroacetaldehyde while heating at 100 °C. The reaction solution was stirred for 30 minutes, and cooled to a room temperature. After water was removed under a reduced pressure, to the residue was added 200ml of tetrahydrofuran; and the resulting solution was filtered to remove undissolved materials. To the filtrate was added 30g of ethyl iodide; and the resulting mixture was heated with stirring for 20 hours, cooled to a room temperature and then filtered. The resulting solid was washed with 50ml of tetrahydrofuran and dried to obtain 18.3g of a solid; and the solid was suspended in 100ml of distilled water. To the suspension was added 8g of 70% sodium hydrosulfide; and the resulting mixture was heated at 70-80 °C with stirring until bubbles were not generated. The reaction solution was cooled to a room temperature, neutralized with concentrated hydrochloric acid and then filtered. The resulting solid was washed with 20ml of distilled water and 50ml of acetone and dried to yield 6.3g of the title compound as a white solid.
NMR(δ, $DMSO-d_6$): 1.27(t, 3H), 4.13(q, 2H), 7.17(d, 1H), 7.87(d, 1H), 8.07(d, 1H), 8.20(d, 1H)
m.p.: >239 °C(decomp.)

Preparation Example 38: Preparation of 7-amino-2-ethyl [1,3]imidazo[1,2-c]pyrimidine-5-thione

The same procedures as described in Preparation Example 37 were repeated using 15.6g of 4,6-diamino-2-methylthio pyrimidine as a starting material to yield 4.8g of the title compound.
NMR(δ, $DMSO-d_6$): 1.31(t, 3H), 4.20(q, 2H), 5.81(s, 1H), 6.84(s, 2H), 7.37(d, 1H), 7.83(d, 1H)
m.p.: >243 °C(decomp.)

Preparation Example 39: Preparation of 1-ethyl-7-methylamino[1,3]imidazo[1,2-c] pyrimidine-5-thione

The same procedures as described in Preparation Example 37 were repeated using 17g of 4-amino-6-methylamino-2-methylthio pyrimidine as a starting material to yield 5.4g of the title compound.
NMR(δ, $DMSO-d_6$): 1.29(t, 3H), 2.83(d, 3H), 4.13(q, 2H), 5.78(s, 1H), 7.27(q, 1H), 7.31(d, 1H), 7.79(d, 1H)
m.p.: >257 °C(decomp.)

Preparation Example 40: Preparation of 7,8-diamino-1-ethyl[1,3]imidazo[1,2-c] pyrimidine-5-thione

The same procedures as described in Preparation Example 37 were repeated using 17.1g of 2-methylthio-4,5,6-triamino pyrimidine as a starting material to yield 6.7g of the title compound.
NMR(δ, $DMSO-d_6$): 1.33(t, 3H), 4.21(q, 2H), 4.39(bs, 2H), 6.93(s, 2H), 7.29(d, 1H), 7.71(d, 1H)
m.p.: >260 °C(decomp.)

Preparation Example 41: Preparation of 7-amino-1-ethyl-8-methyl[1,3]imidazo[1,2-c] pyrimidine-5-thione

The same procedures as described in Preparation Example 37 were repeated using 17g of 4,6-diamino-5-methyl-2-methylthio pyrimidine as a starting material to yield 6.4g of the title compound.

NMR(δ, DMSO-$d_6$): 1.31(t, 3H), 1.98(s, 3H), 4.19(q, 2H), 6.83(s, 2H), 7.42(d, 1H), 7.85(d, 1H)
m.p.: >257 °C(decomp.)

Preparation Example 42: Preparation of 7-amino-1-hydroxyethyl[1,3]imidazo[1,2-c] pyrimidine-5-thione

To a solution of 15.6g of 4,6-diamino-2-methylthio pyrimidine suspended in 200ml of distilled water was added 8.2g of sodium acetate and 32g of 50% chloroacetaldehyde while heating at 100 °C. The reaction solution was stirred for 30 minutes and cooled to a room temperature. After water was removed under a reduced pressure, to the residue was added 200ml of tetrahydrofuran; and the resulting solution was filtered to remove undissolved materials. To the filtrate was added 30g of 2-iodoethanol; and the resulting mixture was heated with stirring for 20 hours, cooled to a room temperature and then filtered. The resulting solid was washed with 50ml of tetrahydrofuran and dried to obtain 19.2g of a solid; and the solid was suspended in 100ml of distilled water. To the suspension was added 8g of 70% sodium hydrosulfide; and the resulting mixture was heated at 70-80 °C with stirring until bubbles were not generated. The reaction solution was cooled to a room temperature, neutralized with concentrated hydrochloric acid and then filtered. The resulting solid was washed with 20ml of distilled water and 20ml of acetone and dried to yield 7.6g of the title compound as a white solid.

NMR(δ, DMSO-$d_6$): 3.37(m; 2H), 3.92(t, 2H), 4.79(bs, 1H), 5.89(s, 1H), 7.01(s, 2H), 7.39(d, 1H), 7.79(d, 1H)
m.p.: >207 °C(decomp.)

Preparation Example 43: Preparation of 7-amino-1-carboxymethyl[1,3]imidazo[1,2-c] pyrimidine-5-thione

The same procedures as described in Preparation Example 42 were repeated using 30g of methylbromo acetate in place of 2-iodoethanol to yield 7.2g of the title compound.

NMR(δ, DMSO-$d_6$): 4.87(s, 2H), 5.75(s, 1H), 7.23(s, 2H), 8.21(d, 1H), 8.58(d, 1H)
m.p.: >201 °C(decomp.)

Preparation Example 44: Preparation of 7-amino-2-carboxy-1-methyl[1,3]imidazo[1,2-c] pyrimidine-5-thione

To a solution of 15.6g of 4,6-diamino-2-methylthio pyrimidine suspended in 100ml of distilled water and 100ml of N,N-dimethyl formamide was added 8.2g of sodium acetate and 45g of methylbromo pyruvate while heating at 100 °C. The reaction solution was stirred for 1 to 2 hours and cooled to a room temperature. After water was removed under a reduced pressure, to the residue was added 200ml of tetrahydrofuran; and the resulting solution was filtered to remove undissolved materials. To the filtrate was added 30g of methyl iodide; and the resulting mixture was heated with stirring for 10 hours, cooled to a room temperature and then filtered. The resulting solid was washed with 50ml of acetone and dried to obtain 18.6g of a solid; and the solid was suspended in 100ml of distilled water. To the suspension was added 12g of 70% sodium hydrosulfide; and the resulting mixture was heated to refulx until bubbles were not generated. The reaction solution was cooled to a room temperature, neutralized with concentrated hydrochloric acid, left for 1 hour on ice-bath and then filtered. The resulting solid was washed with 20ml of distilled water and 50ml of acetone and dried to yield 5.7g of the title compound as a yellow solid.

NMR(δ, DMSO-$d_6$): 3.69(s, 3H), 5.72(s, 1H), 7.01(s, 2H), 8.28(s, 1H)
m.p.: >197 °C(decomp.)

Preparation Example 45: Preparation of 7-amino-2-carboxy-1,8-dimethyl[1,3]imidazo[1,2-c] pyrimidine-5-thione

The same procedures as described in Preparation Example 44 were repeated using 17g of 4,6-diamino-5-methyl-2-methylthio pyrimidine as a starting material to yield 5.5g of the title compound.

NMR(δ, DMSO-$d_6$): 1.95(s, 3H), 3.57(s, 3H), 5.67(s, 1H), 6.99(s, 2H), 8.11(s, 1H)
m.p.: >198 °C(decomp.)

Preparation Example 46: Preparation of 7-amino-2-carboxymethyl-1-methyl[1,3]imidazo [1,2-c]pyrimidine-5-thione

To a solution of 15.6g of 4,6-diamino-2-methylthio pyrimidine suspended in 100ml of distilled water and 100ml of N,N-dimethylformamide was added 8.2g of sodium acetate and 40g of methylbromoacetate while heating at 100°C. The reaction solution was stirred for 1 to 2 hours and cooled to a room temperature. After the solvent was removed under a reduced pressure, to the residue was added 200ml of tetrahydrofuran; and the resulting solution was filtered to remove an undissolved material. To the filtrate was added 30g of methyl iodide; and the resulting mixture was heated with stirring for 10 hours, cooled to a room temperature and then filtered. The resulting solid was washed with 50ml of acetone and dried to obtain 17.9g of a solid; and the solid was suspended in 100ml of distilled water. To the suspension was added 12g of 70% sodium hydrosulfide; and the resulting mixture was heated to reflux with stirring until bubbles were not generated. The reaction solution was cooled to a room temperature, neutralized with concentrated hydrochloric acid, left for 1 hour on ice-bath and then filtered. The resulting solid was washed with 20ml of distilled water and 50ml of acetone and dried to yield 6.1g of the title compound as a yellow solid.

NMR($\delta$, DMSO-$d_6$): 3.48(s, 3H), 3.91(s, 2H), 5.75 (s, 1H), 6.73(s, 2H), 7.84(s, 1H)
m.p.; >203°C(decomp.)

Preparation Example 47: Preparation of 7-amino-2-carboxymethyl-1,8-dimethyl[1,3]imidazo [1,2-c]-pyrimidine-5-thione

The same procedures as described in Preparation Example 46 were repeated using 17g of 4,6-diamino-5-methyl-2-methylthio pyrimidine as a starting material to yield 5.5g of the title compound.

NMR($\delta$, DMSO-$d_6$): 1.97(s, 3H), 3.51(s, 3H), 3.95(s, 2H), 6.89(s, 2H), 7.90(s, 1H)
m.p.: >205°C(decomp.)

Preparation Example 48: Preparation of 7-amino-1-methyl [1,3]imidazol-4-one[1,2-c] pyrimidine-5-thione

To a solution of 15.6g of 4,6-diamino-2-methylthio pyrimidine suspended in 200ml of tetrahydrofuran was added 40g of methylbromo acetate; and the resulting solution was heated to reflux for 20 hours, cooled to a room temperature and then filtered. The resulting solid was washed with 100ml of acetone, dried and then suspended in 100ml of distilled water. To the suspension was added 4g of sodium hydroxide; and the resulting mixture was stirred at 50°C for 1 hour, cooled to a room temperature and filtered. The resulting solid was washed with 20ml of distilled water and 50ml of acetone and dried. The dried solid was dissolved in 100ml of N,N-dimethylformamide; and 15g of methyl iodide was added thereto. The resulting solution was stirred at 80°C for 2 hours and evaporated under a reduced pressure to remove the solvent. To the residue was added 100ml of acetone; and the resulting solid was filtered therefrom, washed with 50ml of acetone and dried to obtain 11.7g of a solid. The solid thus obtained was suspended in 50ml of distilled water; to the suspension was added 4g of 70% sodium hydrosulfide; and the resulting mixture was heated to reflux until bubbles were not generated. The reaction solution was cooled to a room temperature, neutralized with concentrated hydrochloric acid and then filtered. The resulting solid was washed with 20ml of distilled water and 50ml of acetone and dried to yield 3.1g of the title compound as an ivory solid.

NMR($\delta$, DMSO-$d_6$): 3.03(s, 3H), 4.47(s, 2H), 5.63(s, 1H), 7.52(bd, 2H)
m.p.: >237°C(decomp.)

Preparation Example 49: Preparation of 7-amino-1,8-dimethyl[1,3]imidazol-4-one [1,2-c]pyrimidine-5-thione

The same procedures as described in Preparation Example 48 were repeated using 17g of 4,6-diamino-5-methyl-2-methylthio pyrimidine as a starting material to yield 3.5g of the title compound.

NMR($\delta$, DMSO-$d_6$): 1.97(s, 3H), 3.07(s, 3H), 4.41(s, 2H), 7.47(bd, 2H)
m.p.: >242°C(decomp.)

Preparation Example 50: Preparation of paramethoxybenzyl 3-chloromethyl-7-{(Z)-2-[1-(diphenylmethoxycarbonyl)cyclopentoxyimino]-2-[2-(triphenylmethyl)aminothiazol-4-yl] acetamido}-3-cephem-4-carboxylate

Step 1) Preparation of diphenylmethyl 1-bromocyclopentane carboxylate

To 57g of cyclopentane carboxylic acid was added 1g of red phosphorus; and the reaction solution was heated at 130-140°C with stirring while 96g of bromine was added dropwise over 3 hours. The resulting solution was further stirred for 1 hour and cooled to a room temperature; and 300ml of ethyl ether and 300ml of water were added thereto. The mixture was stirred for 5 minutes; and the separated organic phase was dried over magnesium sulfate and filtered. To the dried organic phase was added dropwise with stirring 1M solution of diphenyl diazomethane in ethyl ether until bubbles were not generated; and the resulting solution was washed with 500ml of 5% aqueous sodium bicarbonate solution and 500ml of saturated sodium chloride solution. The separated organic phase was dried over magnesium sulfate, filtered and concentrated under a reduced pressure to yield 95g of the title compound in the form of oil.

NMR(δ, $CDCl_3$): 1.63-1.88(m, 2H), 1.88-2.08(m, 2H), 2.20-2.42(m, 4H), 6.88(s, 1H), 7.20-7.43(m, 10H)

Step 2) Preparation of (Z)-2-{[2-(triphenylmethyl)aminothiazol-4-yl]-2-[1-(diphenylmethoxycarbonyl) cyclopentoxyimino]}acetic acid

To a solution of 46.9g of allyl 2-[2-(triphenylmethyl)aminothiazol-4-yl]-2-hydroxyiminoacetate dissolved in 200ml of dimethyl sulfoxide was added 27.2g of potassium carbonate and 39g of the compound prepared in Step 1 above. The resulting solution was heated at 30-40°C, stirred for 5 hours and cooled to a room temperature; and 1L of ethyl acetate and 1L of distilled water were added thereto. The mixture was stirred for 5 minutes; and the organic phase was separated therefrom, washed twice with 1L of saturated aqueous sodium chloride solution, dried over magnesium sulfate, filtered and concentrated under a reduced pressure. The concentrated residue was dissolved in 400ml of dichloromethane; and to he resulting solution were added 2.6g of triphenylphosphine, 0.1g of tetrakis(triphenylphosphine)palladium and a solution of 19g of potassium 2-ethylhexanoate in 200ml of ethyl acetate in turn at a room temperature with stirring for 2 hours. To the reaction solution were added 500ml of ethyl acetate and 1L of saturated aqueous sodium chloride solution. The mixture was adjusted to about pH 3 with 2N HCl solution with stirring; and the organic phase was separated, dried over magnesium sulfate, filtered and concentrated under a reduced pressure to yield 58.1g of the title compound in the form of a foam.

NMR(δ, $CDCl_3$): 1.48-1.75(m, 4H), 1.95-2.25(m, 4H), 6.39(s, 1H), 6.88(s, 1H), 7.05-7.40 (m, 26H), 8.95-(bs, 1H)

Step 3) Preparation of paramethoxybenzyl 3-chloromethyl7-{(Z)-2-[1(diphenylmethoxycarbonyl)cyclopentoxyimino]-2-[2-(triphenylmethyl)aminothiazol-4-yl) acetamido)-3-cephem-4-carboxylate

8.5g of paramethoxybenzyl 7-amino-3-chloromethyl-3-cephem-4-carboxylatehydrochloride and 14g of the compound prepared in Step 2 above were suspended 100ml of dichloromethane. The suspension was cooled to -20°C; and 5.4g of pyridine and 3.1g of phosphorous oxychloride were added thereto while maintaining at the same temperature. The mixture was stirred and warmed to a room temperature over 1 hour. After adding 200ml of ethyl acetate and 200ml of 1% HCl solution, the resulting solution was stirred for 5 minutes. The organic phase was separated therefrom, washed with 200ml of saturated sodium chloride solution, dried over magnesium sulfate, filtered and concentrated under a reduced pressure to yield 18.1g of the title compound in the form of a foam.

NMR(δ, $CDCl_3$): 1.65-1.86(m, 4H), 2.10-2.25(m, 2H), 2.32-2.55(m, 2H), 3.22-3.47(ABq, 2H), 3.80(s, 3H), 4.40-4.55(ABq, 2H), 5.02 (d, 1H), 5.23(q, 2H), 5.95(q, 1H), 6.67 (s, 1H), 6.88(s, 1H), 6.90(d, 2H), 7.05-7.42(m, 28H), 7.46(d, 1H)

Preparation Example 51: Preparation of paramethoxybenzyl-3-chloromethyl-7-{(Z)-1-((R)diphenylmethoxycarbonylprop-1-oxyimino)-2-[2-(triphenylmethyl) aminothiazol-4-yl]acetamido}-3-cephem-4-carboxylate

Step 1) Preparation of S-(-)-2-chlorobutanoic acid

A solution of 25.78g of S-(+)-2-aminobutanoic acid dissolved in 400ml of 6M HCl solution was cooled to 0°C; and a solution of 27.6g of sodium nitrite dissolved in 100ml of distilled water was added dropwise slowly thereto while maintaining the temperature of the solution below 5°C. The reaction solution was

warmed to a room temperature, left for 18 hours and stirred for 3 hours with degassing. 25g of sodium bicarbonate was added slowly thereto; and the mixture was extracted four times with 100ml of ethyl ether to separate the organic phase. The separated organic phase was concentrated under a reduced pressure to remove the solvent and the volatile materials. The residue was dissolved in 15ml of aqueous sodium chloride solution; and the resulting solution was extracted with 30ml of ethyl ether. The extracted organic phase was dried over calcium chloride and filtered; and the filtrate was evaporated under a reduced pressure to remove the solvent. The residue was distilled at 40-50°C under a reduced pressure to yield 17.43g of the title compound.

NMR(δ, $CDCl_3$): 1.05(t, 3H), 1.88-2.09(m, 2H), 4.27 (dd, 1H), 11.35(bs, 1H)
$[\alpha]_D^{20}$ = -13.07(C=6.71, methanol)

Step 2) Preparation of S-(-)-2-chlorobutanoic benzhydryl ester

To a solution of 16.7g of S-(-)-2-chlorobutanoic acid prepared in Step 1 above dissolved in 100ml of diethyl ether was added dropwise diphenyldiazomethane dissolved in diethyl ether until nitrogen gas was not generated. After the completion of the reaction, 200ml of 5% aqueous sodium bicarbonate solution was added to the reaction solution. The solution was mixed well; and the organic phase was separated therefrom, dried over anydrous magnesium sulfate and filtered. The filtrate was distilled under a reduced pressure to remove the solvent and purified by a silica gel column chromatography to yield 34g of the title compound.

NMR(δ, $CDCl_3$): 0.95(t, 3H), 1.87-2.15(m, 2H), 4.30 (dd, 1H), 6.90(s, 1H), 7.20-7.40(m, 10H)
$[\alpha]_D^{20}$ = -6.03(C=3.17, methanol)

Step 3) Preparation of allyl 2-(tritylaminothiazol-4-yl)-2-[(R)-(+)-1-(diphenylmethoxycarbonyl)prop-1-oxyimino]acetate

To a solution of 55.91g of allyl [2-(tritylamino) thiazol-4-yl]hydroxyimino acetate and 34g of the compound, as prepared in Step 2 above, dissolved in 200ml of dimethyl sulfoxide was added 32.1g of potassium carbonate. The resulting solution was heated at 55°C with stirring for 1.5 hours. After the completion of the reaction, to the reaction solution was added 500ml of distilled water; and the resulting solution was extracted with 500ml of diethyl ether. The organic phase was separated therefrom, washed with 500ml of 10% HCl solution and 500ml of saturated sodium chloride solution in turn, dried over anhydrous magnesium sulfate, filtered and distilled under a reduced pressure to yield 75.58g of the title compound.

NMR(δ, $CDCl_3$): 0.92(t, 3H), 1.80-2.00(m, 2H), 4.79 (d, 2H), 4.88(dd, 1H), 5.20(d, 1H), 5.38(d, 1H), 5.80-6.00(m, 1H), 6.50 (s, 1H), 6.90(s, 1H), 7.17-7.38(m, 26H)
$[\alpha]_D^{20}$ = +9.87°(C=14.35, ethyl acetate)

Step 4) Preparation of 2-(tritylaminothiazol-4-yl)-2-[(R)-(+)-1-(diphenylmethoxycarbonyl)prop-1-oxyimino]-acetic acid

To a solution of 75.58g of the compound prepared in Step 3 above dissolved in 500ml of dichloromethane were added 10.61g of triphenylphosphine, 2.75g of tetrakis (triphenylphosphine) palladium and a solution of 20.1g of potassium 2-ethylhexanoate dissolved in 120ml of ethyl acetate in turn; and the resulting solution was stirred at a room temperature for 1 hour. After the completion of the reaction, the solution was distilled under a reduced pressure to remove the solvent; and 500ml of ethyl acetate and 400ml of distilled water were added thereto with stirring. The mixture was adjusted to pH 2.4 with 10% HCl solution and filtered. The resulting solid was washed with 500ml of distilled water and dried to yield 64.02g of the title compound.

NMR(δ, DMSO-$d_6$): 0.83(t, 3H), 1.65-1.90(m, 2H), 4.68 (dd, 1H), 6.80(s, 1H), 6.83(s, 1H), 7.10-7.50(m, 25H), 8.85(s, 1H), 13.80 (bs, 1H)
$[\alpha]_D^{20}$ = +13.6°(C=8.9, dimethyl sulfoxide)

Step 5) Preparation of paramethoxybenzyl 3-chloromethyl-7-{(Z)-1-((R)-diphenylmethoxycarbonylprop-1-oxyimino)-2-[2-(triphenylmethyl)aminothiazol-4-yl]acetamido)-3-cephem-4-carboxylate

86.68g of paramethoxybenzyl 7-amino-3-chloromethyl-3-cephem-4-carboxylate and 50.64g of pyridine were dissolved completely in 1L of dichloromethane with stirring; and the resulting solution was cooled to

-20°C - -25°C. 135.5g of the compound prepared in Step 4 and 32.8g of phosphorous oxychloride were added thereto; and the reaction mixture was stirred for 30 minutes. After the completion of the reaction, the mixture was washed with 500ml of 1% HCl solution. The organic phase was separated therefrom, washed with 500ml of distilled water and then 500ml of saturated sodium chloride solution, dried over anhydrous magnesium sulfate, filtered and concentrated under a reduced pressure to yield 185g of the title compound as an ivory solid.

NMR(δ, $CDCl_3$): 1.06(t, 3H), 1.95(q, 2H), 3.29(ABq, 2H), 3.81(s, 3H), 3.97(ABq, 2H), 4.95(d, 1H), 5.02-(dd, 1H), 5.21(ABq, 2H), 5.92(dd, 1H), 7.1(ABq, 4H), 6.72(s, 1H), 6.95(s, 1H), 7.22-7.39(m, 25H), 8.08(d, 1H)

Preparation Example 52: Preparation of paramethoxybenzyl 3-chloromethyl-7-{(Z)-1-((R,S)diphenylmethoxycarbonylprop-1-oxyimino)-2-[2-(triphenylmethyl) aminothiazol-4-yl]acetamido}-3-cephem-4-carboxylate

Step 1) Preparation of (±)-2-bromobutanoic acid benzhydryl ester

The same procedures as described in Step 2 of Preparation Example 51 were repeated using 17.4g of (±)-2-bromobutanoic acid in place of S-(-)-2-chlorobutanoic acid to yield 35.2g of the title compound.

NMR(δ, $CDCl_3$): 1.02(m, 3H), 2.08(m, 2H), 4.18(dd, 1H), 6.90(s, 1H), 7.21-7.41(m, 10H)

Step 2) Preparation of allyl 2-(tritylaminothiazol-4-yl)-2-[(±)-1-(diphenylmethoxycarbonyl)-prop-1-oxyimino]-acetate

The same procedures as described in Step 3 of Preparation Example 51 were repeated using 57.24g of (±)-2-bromobutanoic acid benzhydryl ester prepared in Step 1 above in place of S-(-)-2-chlorobutanoic acid benzhydryl ester to yield 78.4g of the title compound.

NMR(δ, $CDCl_3$): 0.99(m, 3H), 1.86(m, 2H), 4.67(dd, 1H), 4.72(d, 2H), 5.35(ABq, 2H), 5.98(m, 1H), 6.53-(s, 1H), 6.91(s, 1H), 6.93(s, 1H), 7.21-7.42(m, 25H)

Step 3) Preparation of 2-(tritylaminothiazol-4-yl)-2-[(±)-1-(diphenylmethoxycarbonyl)-prop-1-oxyimino]acetic acid

The same procedures as described in Step 4 of Preparation Example 51 were repeated using 75.6g of allyl 2-(tritylaminothiazol-4-yl)-2- [(±)-1-(diphenylmethoxycarbonyl)prop-1-oxyimino]acetate prepared in Step 2 above in place of allyl 2-(tritylaminothiazol-4-yl)-2-[(R)-(+)-1-(diphenylmethoxycarbonyl)prop-1-oxyimino] acetate to yield 64.3g of the title compound as a white solid.

NMR(δ, $CDCl_3$): 0.98(m, 3H), 1.87(m, 2H), 4.65(dd, 1H), 6.53(s, 1H), 6.92(s, 1H), 6.94(s, 1H), 7.22-7.41(m, 25H), 11.12(bs, 1H)

Step 4) Preparation of paramethoxybenzyl 3-chloromethyl7-{(Z)-1-((R,S)-diphenylmethoxycarbonylprop-1-oxyimino)-2-[2-(triphenylmethyl)aminothiazol-4-yl]acetamido)-3-cephem-4-carboxylate

The same procedures as descrbied in Step 5 of Preparation Example 51 were repeated using 135.5g of 2-(tritylaminothiazol-4-yl)-2-[(±)-1-(diphenylmethoxycarbonyl)prop-1-oxyimino]acetic acid prepared in Step 3 above in place of 2-(tritylaminothiazol-4-yl)-2-[(R)-(+)-1-(diphenylmethoxycarbonyl)prop-1-oxyimino]acetic acid to yield 185g of the title compound as a white powder.

NMR(δ, $CDCl_3$): 1.02(m, 3H), 1.91(m, 2H), 3.51(ABq, 2H), 4.47(ABq, 2H), 4.74-4.85(m, 1H), 5.02 (dd, 1H), 5.20(ABq, 2H), 5.81-6.01(m, 1H), 6.78(s, 1H), 6.92(s, 1H), 6.98(s, 1H), 7.12(ABq, 2H), 7.21-7.38(m, 25H), 8.84 (dd, 1H)

Preparation Example 53: Preparation of paramethoxybenzyl 3-chloromethyl-7-{(Z)-2-((R,S)-diphenylmethoxycarbonylbut-2-oxyimino)-2-[2-(triphenylmethyl) aminothiazol-4-yl]acetamido)-3-cephem-4-carboxylate

Step 1) Preparation of allyl 2-(tritylaminothiazol-4-yl)-2-[2-(diphenylmethoxycarbonyl)but-2-oxyimino] acetate

To a solution of 250g of allyl [2-(tritylamino)thiazol-4-yl]hydroxyimino acetate and 200g of 2-bromo-2-methylbutanoic acid benzhydryl ester dissolved in 600ml of dimethyl sulfoxide was added 150g of potassium carbonate. The resulting solution was heated at 60°C with stirring for 18 hours and then poured into a mixture of 3L of ethyl acetate and 1.5L of an ice water. The mixture was stirred well; and the organic

phase was separated therefrom, washed with 1.5L of 1% HCl solution and 1.5L of saturated sodium chloride solution in turn, dried over anhydrous magnesium sulfate, filtered and concentrated under a reduced pressure to yield 350g of the title compound as a white solid.

NMR(δ, $CDCl_3$): 0.81(t, 3H), 1.54(s, 3H), 1.91(q, 2H), 4.82(d, 2H), 5.31(ABq, 2H), 5.86-6.02 (m, 1H), 6.47(s, 1H), 6.74(s, 1H), 6.86 (s, 1H), 7.12-7.41(m, 25H)

Step 2) Preparation of 2-(tritylaminothiazol-4-yl)-2-[2-(diphenylmethoxycarbonyl)but-2-oxyimino]acetic acid

350g of the compound prepared in Step 1 above was dissolved in 1.4L of dichloromethane; and to the resulting solution were added 17.2g of triphenylphosphine, 3.05g of tetrakis(triphenyl phosphine) palladium and a solution of 95.25g of potassium 2-ethylhexanoate dissolved in 600ml of ethyl acetate in turn. The reaction solution was stirred at a room temperature for 4 hours and distilled under a reduced pressure; and to the residue was added a mixture of 600ml of ethyl acetate and 600ml of distilled water with stirring. The mixture was adjusted to pH 2.8 with 5% HCl solution; and the organic phase was separated, dried over anhydrous magnesium sulfate and filtered. The filtrate was distilled under a reduced pressure; and the residue was purified by a silica gel column chromatography to yield 280g of the title compound as a white solid.

NMR(δ, $CDCl_3$): 0.72 (t, 3H), 1.57(s, 3H), 1.87(q, 2H), 6.33(s, 1H), 6.90(s, 1H), 7.14-7.42 (m, 26H), 9.02(bs, 1H)

Step 3) Preparation of paramethoxybenzyl 3-chloromethyl-7-{(Z)-2-((R,S)-diphenylmethoxycarbonylbut-2-oxyimino)-2-[2-(triphenylmethyl)aminothiazol-4-yl]acetamido}-3-cephem-4-carboxylate

86.7g of paramethoxybenzyl 7-amino-3-chloromethyl-3-cephem-4-carboxylate and 50.64g of pyridine were dissolved completely in 1L of dichloromethan with stirring; and the resulting solution was cooled to -20°C - -25°C. 135.5g of the compound prepared in Step 2 above and 32.8g of phosphorous oxychloride were added thereto; and the reaction mixture was stirred for 30 minutes. After the completion of the reaction, the mixture was washed with 500ml of 1% HCl solution; and the organic phase was separated, washed with 500ml of distilled water and 500ml of saturated sodium chloride solution in turn, dried over anhydrous magnesium sulfate, filtered and concentrated under a reduced pressure to yield 185g of the title compound as an ivory solid.

NMR(δ, $CDCl_3$): 0.85-0.89(m, 3H), 1.69(d, 3H), 1.87-2,24 (m, 2H), 3.08-3.17(m, 2H), 3.85(s, 3H), 4.44-4.61(m, 2H), 5.02(dd, 1H), 5.20-5.36 (m, 2H), 5.89-6.01(m, 1H), 6.67(d, 1H), 6.91(d, 1H), 6.97(d, 1H), 7.15-7.45 (m, 29H)

## Example 1

Synthesis of 7-[(Z)-2-(2-aminothiazol-4-yl)-2-(2-carboxyprop-2-oxyimino)acetamido]-3-(7-amino-1-methyl [1,2,4]triazolo[1,5-c]pyrimidinium-5-yl)thiomethyl-3-cephem-4-carboxylate

To a solution of 2g of paramethoxybenzyl 3-chloromethyl-7-{(Z)-2-(2-t-butoxycarbonylprop-2-oxyimino)-2-[2-(triphenylmethyl)aminothiazol-4-yl]acetamido}-3-cephem-4-carboxylate dissolved in 10ml of dimethyl sulfoxide was added 400mg of 7-amino-1-methyl[1,2,4]triazolo[1,5-c] pyrimidine-5-thione prepared in Preparation Example 4; and the resulting mixture was stirred for 2 hours at a room temperature. To the reaction solution was added 50ml of diethyl ether; and the mixture was stirred vigorously. After diethyl ether was removed from the mixture, 15ml of dichloromethane was added to the residue. To the resulting solution was added dropwise slowly 100ml of ethyl ether; and the mixture was filtered. The resulting solid was washed with 50ml of ethyl ether, dried and dissolved in 10ml of phenol. To the resulting solution was added 1.0ml of concentrated hydrochloric acid; and the mixture was stirred at 50°C for 2 hours. The reaction solution was cooled to a room temperature; and 100ml of acetone was added thereto. The resulting mixture was filtered; and the resulting solid was washed with 40ml of acetone and dried to yield 1.3g of an ivory solid. The solid thus obtained was fractionated onto the liquid chromatography(μ-Bondapak $C_{18}$ Steel Column, 19mm x 30cm) using 10% methanol solution as an eluent to yield 700mg of the title compound as a white solid.

MS(FAB, M + 1): 637

NMR(δ, $D_2O$ + $NaHCO_3$): 1.42(d, 6H), 3.41(ABq, 2H), 3.67 (s, 3H), 4.41(ABq, 2H), 4.97 (d, 1H), 5.70-(q, 1H), 6.43(s, 1H), 6.71(s, 1H), 7.21(s, 2H), 8.55 (bs, 2H), 9.07(s, 1H)

IR(KBr, $cm^{-1}$): 1765(β-lactam), 1660, 1630, 1550

Examples 2 to 21

The same procedures as described in Example 1 were repeated using paramethoxybenzyl 3-chloromethyl-7-{(Z)-2-(2-t-butoxycarbonylprop-2-oxyimino)-2-[2-(triphenylmethyl)amino-thiazol-4-yl]-acetamido}-3-cephem-4-carboxylate and thiones synthesized in Preparation Examples 4 to 7, 10 to 12, 14 to 16 and 18 to 28. The produced compounds are listed in Table 2.

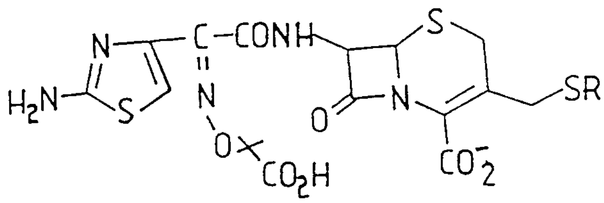

Table 2

| Ex.. | R | MS(FAB, M+1) | NMR(δ, $D_2O$ + $NaHCO_3$) | IR(KBr, $cm^{-1}$) |
|---|---|---|---|---|
| 2 | 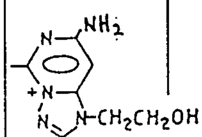 | 667 | 1.45(d,6H), 3.64(ABq,2H), 3.72 (t,3H), 4.31(t,3H), 4.37(ABq,2H) 5.16(d,1H), 5.76(d,1H), 6.36(s, 1H), 6.92(s, 1H), 8.47(s, 1H) | 1769(β-lactam), 1700, 1650, 1590 |
| 3 | 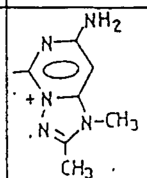 | 651 | 1.46(d,6H), 2.54(s,3H), 3.53 (ABq,2H), 3.82(s,3H), 4.33(ABq, 2H), 5.08(d,1H), 5.72(d,1H), 6.27(s,1H), 6.84(s,1H) | 1767(β-lactam), 1695, 1640, 1580 |
| 4 | 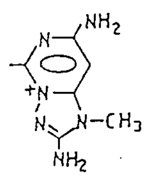 | 652 | * 1.36(d,6H), 3.35(ABq,2H), 3.41 (s,3H), 4.52(ABq,2H), 4.95(d,1H) 5.68(dd,1H), 6.30(s,1H), 6.71(s, 1H), 7.18(s,2H), 7.84(s,2H), 8.06(s,2H), 11.64(d,1H) | 1760(β-lactam), 1680, 1620, 1560 |
| 5 | 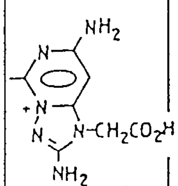 | 696 | * 1.34(d,6H), 3.37(ABq,2H), 4.51 (ABq,2H), 4.84(s,2H), 4.96(d,1H) 5.72(dd,1H), 6.27(s,1H), 6.73(s, 1H), 7.22(s,2H), 7.86(s,2H), 8.05(s,2H), 11.92(d,1H) | 1765(β-lactam), 1690, 1630, 1570 |
| 6 | 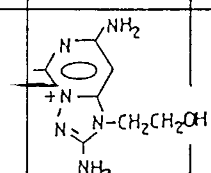 | 682 | 1.46(d,6H), 3.63(ABq,2H), 3.71 (t,3H), 4.27(t,3H), 4.37(ABq,2H) 5.16(d,1H), 5.74(d,1H), 6.36(s, 1H), 6.93(s,1H) | 1770(β-lactam), 1680, 1590, 1530 |
| 7 | 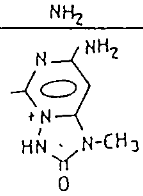 | 653 | * 1.43(d,6H), 3.32(ABq,2H), 3.52 (s,3H), 4.30(ABq,2H), 4.97(d,1H) 5.63(dd,1H), 6.52(s,1H), 6.81(s, 1H), 7.52(s, 2H), 11.82(d, 1H) | 1768(β-lactam), 1671, 1625, 1523 |
| 8 | 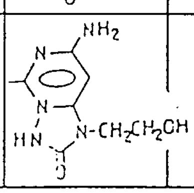 | 683 | 1.46(d,6H), 3.64(ABq,2H), 3.73 (t,3H), 4.12(t,3H), 4.35(ABq,2H) 5.14(d,1H), 5.73(d,1H), 6.42(s, 1H), 6.92(s,1H) | 1765(β-lactam), 1670, 1620, 1530 |

Table 2(continued)

| Ex. | R | MS(FAB, M+1) | NMR($\delta$, $D_2O$ + $NaHCO_3$) | IR(KBr, $cm^{-1}$) |
|---|---|---|---|---|
| 9 | 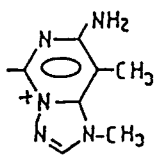 | 651 | * 2.28(s,3H), 3.43(ABq,2H), 3.81 (s,3H), 4.52(ABq,2H), 4.91(d,1H) 5.52(d,1H), 6.74(s,1H), 7.23(s, 2H), 8.97(s,1H), 9.53(d,1H) | 1766($\beta$-lactam), 1765, 1595, 1525 |
| 10 | 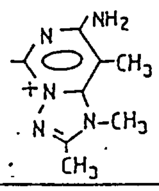 | 665 | 1.48(d,6H), 2.32(s,3H), 2.56(s, 3H), 3.58(ABq,2H), 3.85(s,3H), 4.31(ABq,2H), 5.08(d,1H), 5.70 (d,1H), 6.82(s,1H) | 1765($\beta$-lactam), 1590, 1530 |
| 11 | 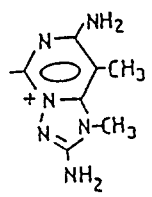 | 666 | * 1.36(d,6H), 2.23(s,3H), 3.37 (ABq,2H), 3.42(s,3H), 4.52(ABq, 2H), 4.95(d,1H), 5.68(dd,1H), 6.72(s,1H), 7.20(s,2H), 7.82(s, 2H), 8.05(s,2H), 11.60(d,1H) | 1760($\beta$-lactam), 1671, 1590, 1525 |
| 12 | 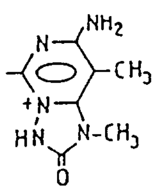 | 667 | * 1.41(d,6H), 1.88(s,3H), 3.35 (ABq,2H), 3.51(s,3H), 4.30(ABq, 2H), 4.95(d,1H), 5.69(dd,1H), 6.72(s,1H), 7.19(s,2H), 7.74(s, 2H), 11.72(d,1H) | 1765($\beta$-lactam), 1680, 1600, 1530 |
| 13 | 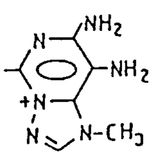 | 652 | 1.45(d,6H), 3.62(ABq,2H), 3.67 (s,3H), 4.35(ABq,2H), 5.14(d,1H) 5.75(d,1H), 6.91(s,1H), 8.32(s, 1H) | 1760($\beta$-lactam), 1670, 1600, 1550 |
| 14 | 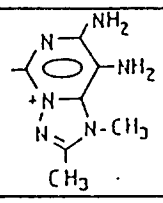 | 666 | 1.45(d,6H), 2.57(s,3H), 3.54 (ABq,2H), 3.75(s,3H), 4.35(ABq, 2H), 5.08(d,1H), 5.74(d,1H), 6.91(s,1H) | 1765($\beta$-lactam), 1670, 1600, 1520 |

Table 2(continued)

| Ex. | R | MS(FAB, M+1) | NMR(δ, $D_2O$ + $NaHCO_3$) | IR(KBr, $cm^{-1}$) |
|---|---|---|---|---|
| 15 | 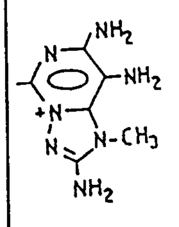 | 667 | * 1.36(d,6H), 3.32(ABq,2H), 3.42 (s,3H), 4.53(ABq,2H), 4.95(d,1H) 5.62(dd,1H), 6.72(s,1H), 7.18(s, 2H), 7.84(s,2H), 8.04(s,2H), 8.27(s,2H), 11.65(d,1H) | 1770(β-lactam), 1680, 1615, 1570 |
| 16 | 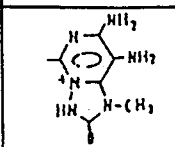 | 668 | 1.46(d,6H), 3.45(ABq,2H), 3.52 (s,3H), 4.41(ABq,2H), 5.1(d,1H), 5.72(d,1H), 6.81(s,1H) | 1765(β-lactam), 1665, 1585, 1530 |
| 17 | 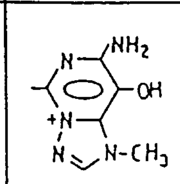 | 653 | 1.45(d,6H), 3.63(ABq,2H), 3.65 (s,3H), 4.35(ABq,2H), 5.14(d,1H) 5.76(d,1H), 6.92(s,1H), 8.47(s, 1H) | 1760(β-lactam), 1680, 1610, 1580 |
| 18 | 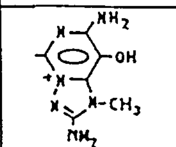 | 668 | 1.46(d,6H), 3.47(ABq,2H), 3.62 (s,3H), 4.32(ABq,2H), 5.12(d,1H) 5.74(d,1H), 6.92(s,1H) | 1769(β-lactam), 1700, 1640, 1580 |
| 19 | 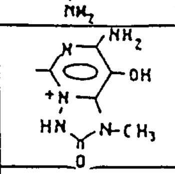 | 669 | 1.45(d,6H), 3.46(ABq,2H), 3.53 (s,3H), 4.35(ABq,2H), 5.12(d,1H) 5.76(d,1H), 6.91(s,1H) | 1767(β-lactam), 1695, 1630, 1580 |
| 20 | 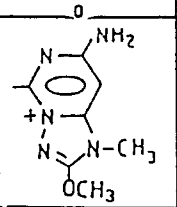 | 667 | 1.46(d,6H), 3.43(ABq,2H), 3.57 (s,3H), 3.83(s,3H), 4.35(ABq,2H) 5.14(d,1H), 5.77(d,1H), 6.28(s, 1H), 6.92(s,1H) | 1765(β-lactam), 1690, 1625, 1575 |
| 21 | 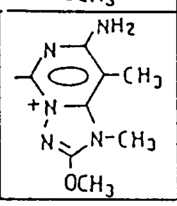 | 681 | 1.47(d,6H), 2.32(s,3H), 3.57 (ABq,2H), 3.64(s,3H), 3.92(s,3H) 4.31(ABq,2H), 5.08(d,1H), 5.72 (d,1H), 6.82(s,1H) | 1770(β-lactam), 1685, 1625, 1550 |

* NMR(δ, DMSO-$d_6$)

Example 22

Synthesis of 7-[(Z)-2-(2-aminothiazol-4-yl)-2-((S-1-carboxyeth-1-oxyimino)acetamido]-3-(7-amino-1-methyl [1,2,4]triazolo[1,5-c]pyrimidinium-5-yl)thiomethyl-3-cephem-4-carboxylate and 7-[(Z)-2-(2-aminothiazol-4-yl)-2-((R)-1-carboxyeth-1-oxyimino)acetamido]-3-(7-amino-1-methyl[1,2,4]triazolo[1,5-c]pyrimidinium-5-yl)-thiomethyl-3-cephem-4-carboxylate

To a solution of 2g of paramethoxybenzyl 3-chloro-7-{(Z)-2-((S,R)-1-t-butoxycarbonyleth-1-oxyimino)-2-[2-(triphenylmethyl)aminothiazol-4-yl]acetamido}-3-cephem-4-carboxylate dissolved in 10ml of dimethyl sulfoxide was added 380mg of 7-amino-1-methyl[1,2,4]triazolo[1,5-c] pyrimidine-5-thione prepared in Preparation Example 4; and the resulting mixture was stirred for 3 hours at a room temperature. To the reaction solution was added 50ml of diethyl ether; and the mixture was stirred vigorously. After diethyl ether was removed from the mixture, 20ml of dichloromethane was added to the residue; to the resulting solution was added dropwise slowly 100ml of diethyl ether; and the mixture was filtered. The resulting solid was washed with 50ml of ethyl ether, dried and dissolved in 10ml of phenol. To the resulting solution was added 1.0ml of concentrated hydrochloric acid; and the mixture was stirred at 50 °C for 2 hours. The reaction solution was cooled to a room temperature; and 100ml of acetone was added thereto. The resulting mixture was filtered; and the resulting solid was washed with 50ml of acetone and dried to yield 1.2g of an ivory solid. The solid thus obtained was fractionated onto the liquid chromatography($\mu$-Bondapak $C_{18}$ Steel Column, 19mm x 30cm) using 5% methanol solution as an eluent to yield 300mg and 270mg of the title compound as S and P forms, respectively.

| | |
|---|---|
| MS(FAB, M + 1): | 623 |
| NMR($\delta$, $D_2O$ + $NaHCO_3$): | |
| (S): | 1.53(d, 3H), 3.45(ABq, 2H), 3.64(s, 3H), 4.52 (ABq, 2H), 4.63(q, 1H), 5.10(d, 1H), 5.72(d, 1H), 6.47(s, 1H), 6.78(s, 1H), 9.09(s, 1H) |
| (R): | 1.53(d, 3H), 3.45(ABq, 2H), 3.64(s, 3H), 4.52 (ABq, 2H), 4.63(q, 1H), 5.10(d, 1H), 5.68(d, 1H), 6.47(s, 1H), 6.78(s, 1H), 9.09(s, 1H) |
| IR(KBr, $cm^{-1}$): | 1766($\beta$-lactam), 1765, 1590, 1525 |

Examples 23 to 27

The same procedures as described in Example 22 were repeated using paramethoxybenzyl 3-chloromethyl-7-{(Z)-2-((R,S)-1-t-butoxycarbonyleth-1-oxyimino)-2-[2-(triphenylmethyl)amino-thiazol-4-yl]-acetamido}-3-cephem-4-carboxylate and thiones synthesized in Preparation Examples 4 to 7, 16 and 19. The produced compounds are listed in Table 3.

Table 3

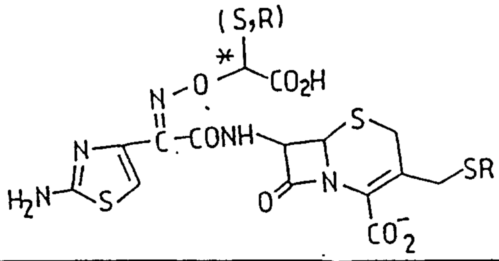

| Ex. | R | MS(FAB, M+1) | NMR(δ, $D_2O$ + $NaHCO_3$) | IR(KBr, $cm^{-1}$) |
|---|---|---|---|---|
| 23(S) | 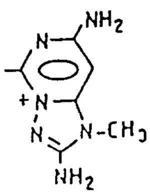 | 638 | 1.47(d,3H), 3.46(ABq,2H), 3.67 (s,3H), 4.45(ABq,2H), 4.64(q,1H) 5.02(d,1H), 5.74(d,1H), 6.52(s, 1H), 6.83(s,1H) | 1765(β-lactam), 1765, 1580, 1520 |
| 23(R) | | | 1.47(d,3H), 3.46(ABq,2H), 3.67 (s,3H), 4.45(ABq,2H), 4.64(q,1H) 5.02(d,1H), 5.71(d,1H), 6.52(s, 1H), 6.83(s,1H) | |
| 24(S) | 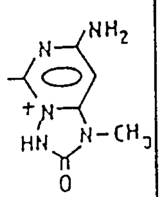 | 639 | 1.51(d,3H), 3.45(ABq,2H), 3.62 (s,3H), 4.43(ABq,2H), 4.62(q,1H) 5.02(d,1H), 5.74(d,1H), 6.52(s, 1H), 6.87(s,1H) | 1760(β-lactam), 1670, 1590, 1525 |
| 24(R) | | | 1.51(d,3H), 3.45(ABq,2H), 3.62 (s,3H), 4.43(ABq,2H), 4.62(q,1H) 5.02(d,1H), 5.71(d,1H), 6.52(s, 1H), 6.87(s,1H) | |
| 25(S) | 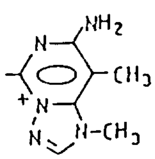 | 637 | 1.52(d,3H), 2.32(s,3H), 3.57 (ABq,2H), 3.79(s,3H), 4.32(ABq, 2H), 4.65(q,1H), 5.07(d,1H), 5.71(d,1H), 6.82(s,1H), 8.84 (s,1H) | 1765(β-lactam), 1670, 1610, 1520 |
| 25(R) | | | 1.52(d,3H), 2.32(s,3H), 3.57 (ABq,2H), 3.79(s,3H), 4.32(ABq, 2H), 4.65(q,1H), 5.07(d,1H), 5.68(d,1H), 6.82(s,1H), 8.84 (s,1H) | |

Table 3(continued)

| Ex. | R | MS(FAB, M+1) | NMR(δ, $D_2O$ + $NaHCO_3$) | IR(KBr, $cm^{-1}$) |
|---|---|---|---|---|
| 26(S) | 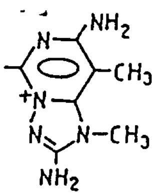 | 652 | 1.51(d,3H), 2.34(s,3H), 3.51 (ABq,2H), 3.62(s,3H), 4.36(ABq, 2H), 4.61(q,1H), 5.06(d,1H), 5.72(d,1H), 6.84~~(s,1H)~~ | 1765(β-lactam), 1660, 1590, 1530 |
| 26(R) | | | 1.51(d,3H), 2.34(s,3H), 3.51 (ABq,2H), 3.62(s,3H), 4.36(ABq, 2H), 4.61(q,1H), 5.06(d,1H), 5.70(d,1H), 6.84(s,1H) | |
| 27(S) | 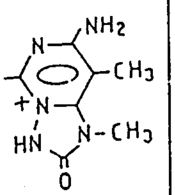 | 653 | 1.54(d,3H), 2.27(s,3H), 3.45 (ABq,2H), 3.51(s,3H), 4.43(ABq, 2H), 4.62(q,1H), 5.10(d,1H), 5.72(d,1H), 6.81(s,1H) | 1765(β-lactam), 1750, 1685, 1630 1580 |
| 27(R) | | | 1.54(d,3H), 2.27(s,3H), 3.45 (ABq,2H), 3.51(s,3H), 4.43(ABq, 2H), 4.62(q,1H), 5.10(d,1H), 5.69(d,1H), 6.81(s,1H) | |

Example 28

Synthesis of 7-[(Z)-2-(2-aminothiazol-4yl)-2-(1-carboxymethoxyimino)acetamido]-3-(7-amino-1-methyl [1,2,4]-triazolo[1,5-c]pyrimidinium-5-yl)thiomethyl-3-cephem-4-carboxylate

To a solution of 2g of paramethoxybenzyl 3-chloromethyl-7-{(Z)-2-(1-t-butoxycarbonylmethoxyimino)-2-[2-(triphenylmethyl)aminothiazol-4-yl]acetamido}-3-cephem-4-carboxylate dissolved in 10ml of dimethyl sulfoxide was added 420mg of 7-amino-1-methyl[1,2,4]triazolo[1,5-c] pyrimidine-5-thione prepared in Preparation Example 4; and the resulting mixture was stirred for 2 hours at a room temperature. To the reaction solution was added 50ml of diethyl ether; and the mixture was stirred vigorously. After the organic phase was removed from the mixture, 20ml of dichloromethane was added to the residue. To the resulting solution was added dropwise slowly 100ml of diethyl ether; and the mixture was filtered. The resulting solid was washed with 50ml of diethyl ether, dried and dissolved in 10ml of phenol. To the resulting solution was added 1.0ml of concentrated hydrochloric acid; and the mixture was stirred at 50°C for 2 hours. The reaction solution was cooled to a room temperature; and 100ml of acetone was added thereto. The resulting mixture was filtered; and the resulting solid was washed with 40ml of acetone and dried to yield 1.2g of an ivory solid. The solid thus obtained was fractionated onto the liquid chromatography (μ-Bondapak $C_{18}$ Steel Column, 19mm x 30cm) using 10%-methanol solution as an eluent to yield 680mg of the title compound as a white solid.

MS(FAB, M + 1): 609
NMR(δ, DMSO-$d_6$): 3.44(ABq, 2H), 3.66(s, 3H), 4.38 (ABq, 2H), 4.82(s, 2H), 4.99(d, 1H), 5.70(dd, 1H), 6.42(s, 1H), 6.73(s, 1H), 7.24(s, 2H), 8.62(s, 2H), 9.08(s, 1H)
IR(KBr, $cm^{-1}$): 1760(β-lactam), 1670, 1600, 1550

Example 29 to 35

The same procedures as described in Example 28 were repeated using paramethoxybenzyl 3-chloromethyl-7-{(Z)-2-(1-t-butoxycarbonylmethoxyimino)-2-[-2-(triphenylmethyl)amino-thiazol-4-yl]-acetamido}-3-cephem-4-carboxylate and thiones synthesized in Preparation Examples 4 to 7, 16, 19, 20 and 27. The produced compounds are listed in Table 4.

Table 4

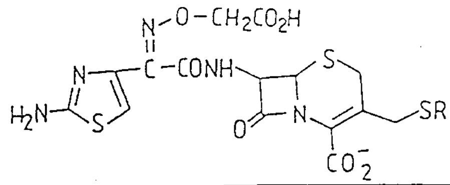

| Ex. | R | MS(FAB, M+1) | NMR(δ, DMSO-$d_6$) | IR(KBr, $cm^{-1}$) |
|---|---|---|---|---|
| 29 | 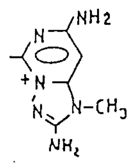 | 624 | 3.32(ABq,2H), 3.43(s,3H), 4.54 (ABq,2H), 4.80(s,2H), 4.95(d,1H) 5.70(dd,1H), 6.30(s,1H), 6.75(s, 1H), 7.20(s,2H), 7.86(s,2H), 8.07(s,2H), 11.62(d,1H) | 1765(β-lactam), 1670, 1600, 1520 |
| 30 | 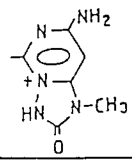 | 625 | 3.35(ABq,2H), 3.52(s,3H), 4.42 (ABq,2H), 4.82(s,2H), 4.99(d,1H) 5.62(dd,1H), 6.57(s,1H), 6.84(s, 1H), 7.52(s,2H), 11.84(d,1H) | 1763(β-lactam), 1680, 1620, 1530 |
| 31 | 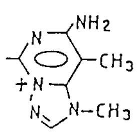 | 623 | 2.27(s,3H), 3.36(ABq,2H), 3.76 (s,3H), 4.52(ABq,2H), 4.80(s,2H) 4.93(d,1H), 5.54(dd,1H), 6.74(s, 1H), 7.26(s,2H), 8.82(s,1H), 9.56(d,1H) | 1765(β-lactam), 1660, 1620, 1530 |
| 32 | 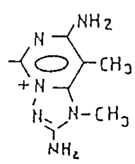 | 638 | 2.21(s,3H), 3.32(ABq,2H), 3.41 (s,3H), 4.52(ABq,2H), 4.97(d,1H) 5.66(dd,1H), 6.71(s,1H), 7.40(s, 2H), 7.79(s,2H), 8.04(s,2H), 11.54(d,1H) | 1770(β-lactam), 1660, 1600, 1535 |
| 33 | 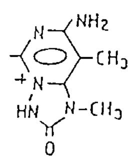 | 639 | 1.92(s,3H), 3.36(ABq,2H), 3.57 (s,3H), 4.36(ABq,2H), 4.84(s,2H) 4.98(d,1H), 5.62(dd,1H), 6.70(s, 1H), 7.18(s,2H), 7.72(s,2H), 11.64(d,1H) | 1766(β-lactam), 1655, 1600, 1533 |
| 34 | 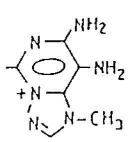 | 624 | 3.34(ABq,2H), 3.45(s,3H), 4.53 (ABq,2H), 4.81(s,2H), 4.96(d,1H) 5.66(dd,1H), 6.74(s,1H), 7.24(s, 2H), 7.84(s,2H), 8.04(s,2H), 8.71(s,1H), 11.52(d,1H) | 1765(β-lactam), 1670, 1610, 1525 |
| 35 | 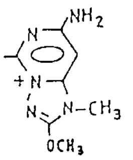 | 639 | 3.32(ABq,2H), 3.56(s,1H), 3.93 (s,3H), 4.32(ABq,2H), 4.84(s,2H) 4.96(d,1H), 5.64(dd,1H), 6.52(s, 1H), 6.86(s,1H), 7.52(s,2H), 11.80(d,1H) | 1770(β-lactam), 1690, 1610, 1520 |

Example 36

Synthesis of 7-[(Z)-2-(2-aminothiazol-4-yl)-2-(ethoxyimino)acetamido]-3-(7-amino-1-methyl[1,2,4]triazolo[1,5-c]pyrimidinium-5-yl)thiomethyl-3-cephem-4-carboxylate

To a solution of 2g of paramethoxybenzyl 3-chloromethyl-7-{(Z)-2-(2-ethoxyimino)-2-[2-(triphenylmethyl)aminothiazol-4-yl]acetamido}-3-cephem-4-carboxylate dissolved in 10ml of dimethyl sulfoxide was added 430mg of 7-amino-1-methyl[1,2,4] triazolo[1,5-c]pyrimidine-5-thione prepared in Preparation Example 4; and the resulting mixture was stirred for 2 hours at a room temperature and extracted with a mixture of 30ml of tetrahydrofuran and 30ml of ethyl acetate. The extract was washed with 100ml of saturated sodium chloride solution; and the organic phase was separated therefrom, dried over anhydrous magnesium sulphate, distilled under a reduced pressure to remove the solvent and dried. The resulting solid was dissolved in 4ml of anisole; and the reaction solution was cooled to 0 °C to 4 °C. After 8ml of trifluoroacetic acid was added dropwise thereto, the mixture was stirred at a room temperature for 50 minutes and cooled to -20 °C to -30 °C; and 50ml of diethyl ether was added dropwise thereto. The resulting mixture was filtered; and the resulting solid was washed with 100ml of acetone and dried to yield 1.2g of an ivory powdery solid. The solid thus obtained was fractionated onto the liquid chromatography($\mu$-Bondapak $C_{18}$ Steel Column, 19mm x 30cm) using 20%-methanol solution as an eluent to yield 700mg of the title compound as a white solid.

| | |
|---|---|
| MS(FAB, M + 1): | 579 |
| NMR($\delta$, DMSO-$d_6$): | 1.27(t, 3H), 3.42(ABq, 2H), 3.69(s, 3H), 4.11(q, 2H), 4.45(ABq, 2H), 5.13(d, 1H), 5.57(q, 1H), 6.30(s, 1H), 6.71(s, 1H), 7.23(s, 2H), 9.01(s, 1H), 9.47(d, 1H) |
| IR(KBr, $cm^{-1}$): | 1760($\beta$-lactam), 1660, 1590 |

Examples 37 to 45

The same procedures as described in Example 36 were repeated using paramethoxybenzyl 3-chloromethyl-7-{(Z)-2-(ethoxyimino)-2-[2-(triphenylmethyl)aminothiazol-4-yl] acetamido}-3-cephem-4-carboxylate and thiones synthesized in Preparation Examples 4 to 6, 8 to 10, 13, 14, 16 and 19. The produced compounds are listed in Table 5.

Table 5

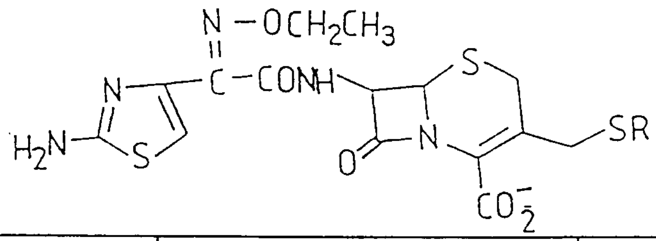

| Ex . | R | MS(FAB, M+1) | NMR(δ, DMSO-$d_6$) | IR(KBr, $cm^{-1}$) |
|---|---|---|---|---|
| 37 | 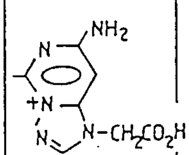 | 623 | * 1.31(t,3H), 3.61(ABq,2H), 4.21 (q,2H), 4.51(ABq,2H), 4.92(s,2H) 5.15(d,1H), 5.72(dd,1H), 6.27(s, 1H), 6.95(s,1H), 8.63(s,1H) | 1768(β-lactam), 1680, 1620, 1560 |
| 38 | 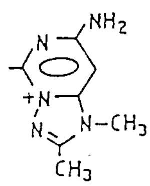 | 593 | 1.23(t,3H), 2.54(s,3H), 3.32 (ABq,2H), 3.81(s,3H), 4.06(q,2H) 4.42(ABq,2H), 5.01(d,1H), 5.67 (dd,1H), 6.17(s,1H), 6.72(s,1H), 7.23(s,1H), 7.92(s,2H), 9.54 (d,1H) | 1765(β-lactam), 1670, 1625, 1550 |
| 39 | 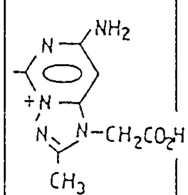 | 637 | 1.27(t,3H), 2.57(s,3H), 3.56 (ABq,2H), 4.07(q,2H), 4.46(ABq, 2H), 4.95(s,2H), 5.07(d,1H), 5.72(dd,1H), 6.30(s,1H), 6.77(s, 1H), 7.04(s,2H), 7.82(s,2H), 9.47(d,1H) | 1765(β-lactam), 1680, 1590 |
| 40 | 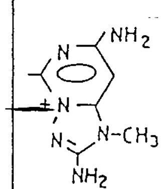 | 594 | 1.21(t,3H), 3.46(s,3H), 3.52 (ABq,2H), 4.08(q,2H), 4.42(ABq, 2H), 5.02(d,1H), 5.65(dd,1H), 6.17(s,1H), 6.73(s,1H), 7.21(s, 2H), 7.78(s,2H), 7.94(s,2H), 9.52(d,1H) | 1760(β-lactam), 1675, 1580 |
| 41 | 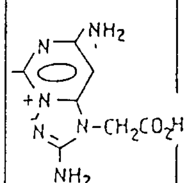 | 638 | 1.23(t,3H), 3.54(ABq,2H), 4.12 (q,2H), 4.45(ABq,2H), 4.92(s,2H) 5.01(d,1H), 5.72(dd,1H), 6.24(s, 1H), 6.81(s,1H), 7.07(s,2H), 7.56(s,2H), 8.21(s,2H), 9.57 (d,1H) | 1769(β-lactam), 1690, 1630 |

Table 5(continued)

| Ex. | R | MS(FAB, M+1) | NMR(δ, DMSO-$d_6$) | IR(KBr, $cm^{-1}$) |
|---|---|---|---|---|
| 42 | 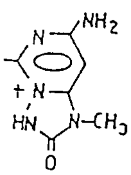 | 595 | 1.26(t,3H), 3.43(ABq,2H), 3.54 (s,3H), 4.08(q,2H), 4.58(ABq,2H) 4.96(d,1H), 5.62(dd,1H), 6.21(s, 1H), 6.70(s,1H), 7.31(s,2H), 7.45(s,2H), 9.49(d,1H) | 1765(β-lactam), 1685, 1640, 1620 |
| 43 | 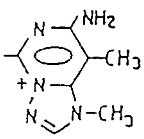 | 593 | 1.21(t,3H), 2.27(s,3H), 3.37 (ABq,2H), 3.97(s,3H), 4.07(q,2H) 4.49(ABq,2H), 4.92(d,1H), 5.57 (q,1H), 6.71(s,1H), 7.21(s,2H), 8.99(s,1H), 9.47(d,1H) | 1768(β-lactam), 1643, 1612, 1600 1515 |
| 44 | 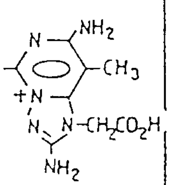 | 652 | 1.24(t,3H), 2.19(s,3H), 3.52 (ABq,2H), 4.05(q,2H), 4.48(ABq, 2H), 4.96(s,2H), 4.99(d,1H), 5.74(dd,1H), 6.75(s,1H), 7.21 (s,2H), 7.46(s,2H), 8.14(s,2H), 9.52(d,1H) | 1765(β-lactam), 1630, 1520 |
| 45 | 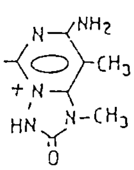 | 609 | 1.23(t,3H), 2.21(s,3H), 3.45 (ABq,2H), 3.56(s,3H), 4.12(q,2H) 4.55(ABq,2H), 4.96(d,1H), 5.65 (dd,1H), 6.72(s,1H), 7.32(s,2H), 7.47(s,2H), 9.48(d,1H) | 1765(β-lactam), 1643, 1610, 1530 |

* NMR(δ, $D_2O$ + $NaHCO_3$)

Example 46

Synthesis of 7-[(Z)-2-(2-aminothiazol-4-yl)-2-(methoxy-imino)acetamido]-3-(7-amino-1-methyl[1,2,4]triazolo-[1,5-c]pyrimidinium-5-yl)thiomethyl-3-cephem-4-carboxylate

To a solution of 2g of paramethoxybenzyl 3-chloromethyl-7-{(Z)-2-(methoxyimino)-2-[2-(triphenylmethyl)aminothiazol-4-yl]acetamido)-3-cephem-4-carboxylate dissolved in 10ml of dimethyl sulfoxide was added 430mg of 7-amino-1-methyl[1,2,4] triazolo[1,5-c]pyrimidine-5-thione prepared in Preparation Example 4; and the resulting mixture was stirred for 2 hours at a room temperature and extracted with a mixture of 30ml of tetrahydrofuran and 30ml of ethyl acetate. The extract was washed with 100ml of saturated sodium chloride solution; and the organic phase was separated therefrom, dried over anhydrous magnesium sulphate, distilled under a reduced pressure to remove the solvent and dried. The resulting solid was dissolved in 4ml of anisole; and the reaction solution was cooled to 0 °C to 4 °C. After 8ml of trifluoroacetic acid was added dropwise thereto, the mixture was stirred at a room temperature for 50 minutes, cooled to -20 °C to -30 °C; and 50ml of diethyl ether was added dropwise thereto. The resulting mixture was filtered; and the resulting solid was washed with 100ml of acetone and dried to yield 1.2g of an ivory powdery solid. The solid thus obtained was fractionated onto the liquid chromatography(μ-Bondapak $C_{18}$ Steel Column, 19mm x 30cm) using 20%-methanol solution as an eluent to yield 700mg of the title compound as a white

solid.

MS(FAB, M + 1): 565

NMR($\delta$, DMSO-$d_6$): 3.41(ABq, 2H), 3.68(s, 3H), 3.81(s, 3H), 4.41(ABq, 2H), 4.93(d, 1H), 5.57(q, 1H), 6.31(s, 1H), 6.74(s, 1H), 7.24(s, 2H), 9.03(s, 1H), 9.51(d, 1H)

IR(KBr, $cm^{-1}$): 1765($\beta$-lactam), 1610, 1640, 1540

Examples 47 to 67

The same procedures as described in Example 46 were repeated using paramethoxybenzyl 3-chloromethyl-7-{(Z)-2-(methoxyimino)-2-[2-(triphenylmethyl)aminothiazol-4-yl] acetamido)-3-cephem-4-carboxylate and thiones synthesized in Preparation Examples 4 to 8 and 12 to 28. The produced compounds are listed in Table 6.

Table 6

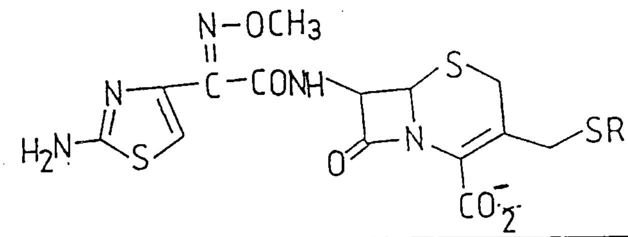

| Ex. | R | MS(FAB, M+1) | NMR(δ, DMSO-$d_6$) | IR(KBr, $cm^{-1}$) |
|---|---|---|---|---|
| 47 | 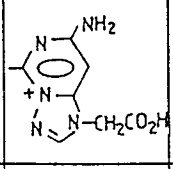 | 609 | * 3.61(ABq,2H), 3.82(s,3H), 4.52 (ABq,2H), 4.94(s,2H), 5.10(d,1H) 5.70(d,1H), 6.27(s,1H), 6.93(s, 1H), 8.63(s,1H) | 1765($\beta$-lactam), 1660, 1630, 1550 |
| 48 | 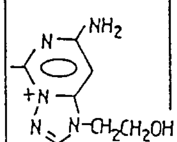 | 595 | * 3.59(ABq,2H), 3.79(s,3H), 3.82 (t,2H), 4.23(t,2H), 4.50(ABq,2H) 5.10(d,1H), 5.72(d,1H), 6.28(s, 1H), 6.91(s,1H), 8.62(s,1H) | 1770($\beta$-lactam), 1665, 1630, 1550 |
| 49 | 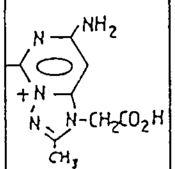 | 623 | 2.54(s,3H), 3.54(ABq,2H), 3.79 (s,3H), 4.45(ABq,2H), 4.92(s,2H) 5.06(d,1H), 5.71(dd,1H), 6.29(s, 1H), 6.82(s,1H), 7.02(s,2H), 7.79(s,2H), 9.49(d,1H) | 1765($\beta$-lactam), 1670, 1620, 1560 |
| 50 | 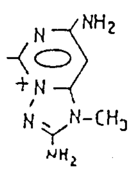 | 580 | 3.51(s,3H), 3.67(ABq,2H), 3.76 (s,3H), 4.32(ABq,2H), 5.11(d,1H) 5.73(dd,1H), 6.28(s,1H), 6.72(s, 1H), 7.44(s,2H), 7.73(s,2H), 7.90(s,2H), 9.62(d,1H) | 1765($\beta$-lactam), 1680, 1630, 1570 |
| 51 | 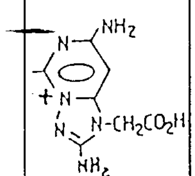 | 624 | 3.53(ABq,2H), 3.81(s,3H), 4.43 (ABq,2H), 4.91(s,2H), 5.02(d,1H) 5.72(dd,1H), 6.24(s,1H), 6.83(s, 1H), 7.06(s,2H), 7.62(s,2H), 8.23(s,2H), 9.56(d,1H) | 1770($\beta$-lactam), 1675, 1650, 1540 |
| 52 | 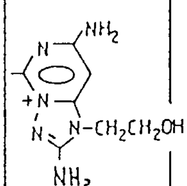 | 610 | 3.54(ABq,2H), 3.76(s,3H), 3.83 (t,2H), 4.09(t,2H), 4.42(ABq,2H) 5.01(d,1H), 5.68(dd,1H), 6.24(s, 1H), 6.82(s,1H), 7.02(s,2H), 7.56(s,2H), 8.31(s,2H), 9.49 (d,1H) | 1770($\beta$-lactam), 1690, 1630, 1580 |

Table 6(continued)

| Ex. | R | MS(FAB, M+1) | NMR(δ, DMSO-$d_6$) | IR(KBr, $cm^{-1}$) |
|---|---|---|---|---|
| 53 | 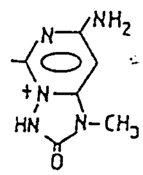 | 581 | * 3.43(ABq,2H), 3.54(s,3H), 3.82 (s,3H), 4.52(ABq,2H), 4.99(d,1H) 5.66(dd,1H), 6.22(s,1H), 6.71(s, 1H), 7.31(s,2H), 7.45(s,2H), 9.48(s,1H) | 1775(β-lactam), 1750, 1695, 1640 1550 |
| 54 | 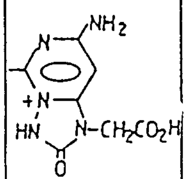 | 625 | * 3.50(ABq,2H), 3.76(s,3H), 4.35 (ABq,2H), 4.90(s,2H), 5.01(d,1H) 5.68(dd,1H), 6.21(s,1H), 6.73(s, 1H), 7.32(s,2H), 7.52(s,2H), 9.52(s,1H) | 1776(β-lactam), 1760, 1680, 1650 1530 |
| 55 | 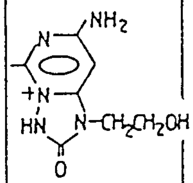 | 611 | * 3.47(ABq,2H), 3.82(s,3H), 3.86 (t,2H), 4.12(t,2H), 4.42(ABq,2H) 5.02(d,1H), 5.67(dd,1H), 6.22(s, 1H), 6.72(s,1H), 7.32(s,2H), 7.62(s,2H), 9.54(s,1H) | 1775(β-lactam), 1750, 1680, 1630 1580 |
| 56 | 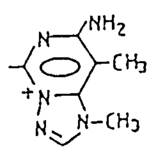 | 579 | 2.20(s,3H), 3.38(ABq,2H), 3.82 (s,3H), 3.85(s,3H), 4.49(ABq,2H) 4.96(d,1H), 5.62(dd,1H), 6.71(s, 1H), 7.32(s,2H), 8.99(s,1H), 9.51(d,1H) | 1765(β-lactam), 1695, 1640, 1580 |
| 57 | 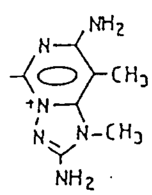 | 594 | 2.17(s,3H), 3.45(s,3H), 3.51 (ABq,2H), 3.82(s,3H), 4.41(ABq, 2H), 5.02(d,1H), 5.66(dd,1H), 6.72(s,1H), 7.24(s,2H), 7.69(s, 2H), 7.89(s,2H), 9.56(d,1H) | 1770(β-lactam), 1680, 1650, 1575 |
| 58 | 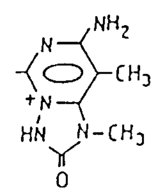 | 595 | 2.22(s,3H), 3.44(ABq,2H), 3.57 (s,3H), 3.78(s,3H), 4.56(ABq,2H) 4.99(d,1H), 5.64(dd,1H), 6.73(s, 1H), 7.32(s,2H), 7.48(s,2H), 9.51(d,1H) | 1760(β-lactam), 1750, 1700, 1650 1590 |

Table 6(continued)

| Ex . | R | MS(FAB, M+1) | NMR($\delta$, DMSO-$d_6$) | IR(KBr, $cm^{-1}$) |
|---|---|---|---|---|
| 59 | 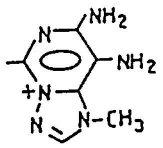 | 580 | 3.36(ABq,2H), 3.86(s,3H), 3.89 (s,3H), 4.43(ABq,2H), 4.96(d,1H) 5.56(dd,1H), 6.72(s,1H), 7.21(s, 2H), 7.56(s,2H), 8.92(s,1H), 9.53(d,1H) | 1765($\beta$-lactam), 1660, 1640, 1530 |
| 60 | 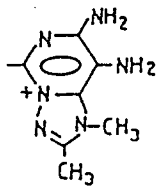 | 594 | 2.41(s,3H), 3.36(ABq,2H), 3.76 (s,3H), 3.83(s,3H), 4.42(ABq,2H) 5.01(d,1H), 5.62(dd,1H), 6.72(s, 1H), 7.21(s,2H), 7.67(s,2H), 7.98(s,2H), 9.56(d,1H) | 1770($\beta$-lactam), 1640, 1620, 1530 |
| 61 | 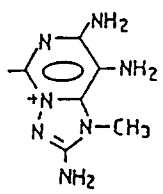 | 595 | 3.35(ABq,2H), 3.86(s,3H), 3.94 (s,3H), 4.43(ABq,2H), 4.96(d,1H) 5.56(dd,1H), 6.71(s,1H), 7.21(s, 2H), 7.56(s,2H), 7.82(s,2H), 8.21(s,2H), 9.46(d,1H) | 1770($\beta$-lactam), 1660, 1630, 1520 |
| 62 | 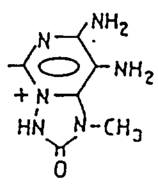 | 596 | 3.41(ABq,2H), 3.62(s,3H), 3.84 (s,3H), 4.53(ABq,2H), 5.01(d,1H) 5.71(dd,1H), 6.72(s,1H), 7.23(s, 2H), 7.46(s,2H), 7.86(s,2H), 9.52(d,1H) | 1770($\beta$-lactam), 1680, 1635, 1570 |
| 63 | 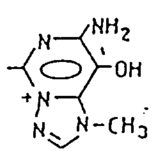 | 581 | 3.37(ABq,2H), 3.84(s,3H), 3.91 (s,3H), 4.43(ABq,2H), 4.96(d,1H) 5.57(dd,1H), 6.71(s,1H), 7.22 (s,2H), 7.42(s,2H), 8.93(s,1H), 9.48(d,1H), 11.21(bs,1H) | 1775($\beta$-lactam), 1750, 1695, 1530 |
| 64 | 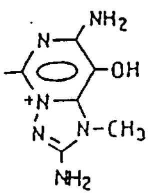 | 596 | 3.41(ABq,2H), 3.62(s,3H), 3.76 (s,3H), 4.45(ABq,2H), 4.96(d,1H) 5.62(dd,1H), 6.72(s,1H), 7.22(s, 2H), 7.86(s,2H), 8.23(s,2H), 9.52(d,1H), 10.92(bs,1H) | 1776($\beta$-lactam), 1680, 1640, 1530 |

Table 6(continued)

| Ex. | R | MS(FAB, M+1) | NMR(δ, DMSO-$d_6$) | IR(KBr, $cm^{-1}$) |
|---|---|---|---|---|
| 65 | 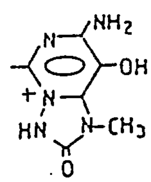 | 597 | 3.39(ABq,2H), 3.71(s,3H), 3.86 (s,3H), 4.52(ABq,2H), 4.96(d,1H) 5.68(dd,1H), 6.73(s,1H), 7.23 (s,2H), 7.62(s,2H), 9.47(d,1H), 10.89(bs,1H) | 1775(β-lactam), 1750, 1680, 1625 1570 |
| 66 | 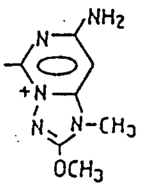 | 595 | 3.34(ABq,2H), 3.76(s,3H), 3.84 (s,3H), 3.86(s,3H), 4.32(ABq,2H) 5.01(d,1H), 5.62(dd,1H), 6.20 (s,1H), 6.70(s,1H), 7.23(s,2H), 7.85(s,2H), 9.48(d,1H) | 1765(β-lactam), 1660, 1640, 1520 |
| 67 | 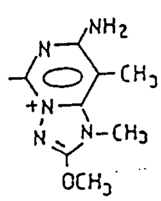 | 609 | 2.21(s,3H), 3.36(ABq,2H), 3.67 (s,3H), 3.74(s,3H), 3.85(s,3H), 3.87(s,3H), 4.41(ABq,2H), 4.99 (d,1H), 5.66(dd,1H), 6.71(s,1H), 7.23(s,2H), 7.67(s,2H), 9.52 (d,1H) | 1770(β-lactam), 1650, 1630, 1530 |

* NMR(δ, $D_2O$ + $NaHCO_3$)

Example 68

Synthesis of 7-[(Z)-2-(2-aminothiazol-4-yl)-2-(2-propyn-1-oxyimino)acetamido]-3-(7-amino-1-methyl[1,2,4]-triazolo [1,5-c]pyrimidinium-5-yl)thiomethyl-3-cephema-4-carboxylate

To a solution of 1.5g of paramethoxybenzyl 3-chloro-methyl-7-{(Z)-2-(2-propyn-1-oxyimino)-2-[2-(triphenylmethyl)aminothiazol-4-yl]acetamido)-3-cephem-4-carboxylate dissolved in 10ml of dimethyl sulfoxide was added 340mg of 7-amino-1-methyl[1,2,4] triazolo[1,5-c]pyrimidine-5-thione prepared in Preparation Example 4; and the resulting mixture was stirred for 3 hours at a room temperature and extracted with a mixture of 20ml of tetrahydrofuran and 20ml of ethyl acetate. The extract was washed with 100ml of aqueous sodium chloride solution; and the organic phase was separated therefrom, dried over anhydrous magnesium sulphate, distilled under a reduced pressure to remove the solvent and dried. The resulting solid was dissolved in 4ml of anisole; and the reaction solution was cooled to 0°C to 4°C. After 8ml of trifluoroacetic acid was added dropwise thereto, the mixture was stirred at a room temperature for 40 minutes and cooled to -20°C to -30°C; and 50ml of diethyl ether was added dropwise thereto. The resulting mixture was filtered; and the resulting solid was washed with 50ml of acetone and dried to yield 0.9g of an ivory powdery solid. The solid thus obtained was fractionated onto the liquid chromatography(μ-Bondapak $C_{18}$ Steel Column, 19mm x 30cm) using 25%-methanol solution as an eluent to yield 500mg of the title compound as a white solid.

| | |
|---|---|
| MS(FAB, M + 1): | 589 |
| NMR(δ, DMSO-$d_6$): | 3.03(s, 1H), 3.64(s, 3H), 4.43(ABq, 2H), 4.84(s, 2H), 5.07(d, 1H), 5.57(q, 1H), 6.30(s, 1H), 6.74(s, 1H), 7.24(s, 2H), 9.04(s, 1H), 9.46(d, 1H) |
| IR(KBr, $cm^{-1}$): | 1766(β-lactam), 1685, 1632, 1525 |

Examples 69 to 71

The same procedures as described in Example 68 were repeated using paramethoxybenzyl 3-chloromethyl-7-{(Z)-2-(2-propyn-1-oxyimino)-2-[2-(triphenylmethyl)aminothiazol-4-yl]acetamido)-3-cephem-4-carboxylate and thiones synthesized in Preparation Examples 6,8 and 16. The produced compounds are listed in Table 7.

Table 7

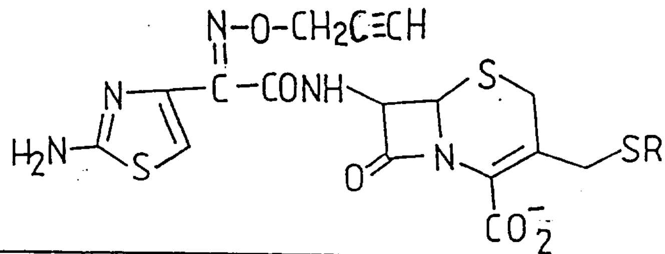

| Ex. | R | MS(FAB, M+1) | NMR(δ, DMSO-$d_6$) | IR(KBr, $cm^{-1}$) |
|---|---|---|---|---|
| 69 | 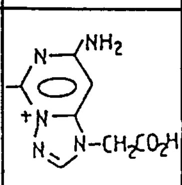 | 633 | * 2.97(s,1H), 3.56(ABq,2H), 4.52 (ABq,2H), 4.83(s,2H), 4.94(s,2H) 5.12(d,1H), 5.70(dd,1H), 6.27 (s,1H), 6.92(s,1H), 8.69(s,1H) | 1769(β-lactam), 1781, 1630, 1525 |
| 70 | 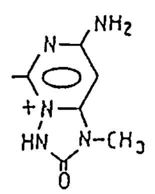 | 605 | 3.02(s,1H), 3.47(ABq,2H), 3.56 (s,3H), 4.56(ABq,2H), 4.80(s,2H) 4.98(d,1H), 5.66(dd,1H), 6.27(s, 1H), 6.76(s,1H), 7.32(s,2H), 7.53(s,2H), 9.46(d,1H) | 1765(β-lactam), 1660, 1620, 1530 |
| 71 | 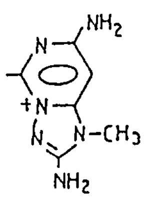 | 604 | 3.04(s,1H), 3.51(s,3H), 3.54 (ABq,2H), 4.47(ABq,2H), 4.86 (s,2H), 5.01(d,1H), 5.72(dd,1H), 6.18(s,1H), 6.72(s,1H), 7.22(s, 2H), 7.76(s,2H), 7.94(s,2H), 9.53(d,1H) | 1770(β-lactam), 1680, 1640, 1520 |

* NMR(δ, $D_2O$ + $NaHCO_3$)

Example 72

Synthesis of 7-[(Z)-2-(5-amino-1,2,4-thiadiazol-3-yl)-2-(ethoxyimino)acetamido]-3-(7-amino-1-methyl[1,2,4] triazolo[1,5-c]pyrimidinium-5-yl)thiomethyl-3-cephem-4-carboxylate

To a solution of 2g of paramethoxybenzyl 3-chloromethyl-7-[(Z)-2-(ethoxyimino)-2-(5-amino-1,2,4-thiadiazol-3-yl) acetamido]-3-cephem-4-carboxylate dissolved in 10ml of dimethyl sulfoxide was added 400mg of 7-amino-1-methyl [1,2,4]triazolo[1,5-c]pyrimidine-5-thione prepared in Preparation Example 4; and the resulting mixture was stirred for 2 hours at a room temperature. To the reaction solution was added 50ml of diethyl ether; and the mixture was stirred vigorously. After the organic solvent layer was removed from the mixture, 20ml of dichloromethane was added to the residue. To the resulting solution was added dropwise slowly 100ml of diethyl ether; and the mixture was filtered. The resulting solid was washed with 50ml of diethyl ether, dried and dissolved in 4ml of anisole. The reaction solution was cooled to 0°C to 4°C; and after 8ml of trifluoroacetic acid was added dropwise thereto, the mixture was stirred for 50

minutes at a room temperature and cooled to -20°C to -30°C; and 50ml of diethyl ether was added dropwise thereto. The resulting mixture was filtered; and the resulting solid was washed with 100ml of acetone and dried to yield 1.1g of an ivory powdery solid. The solid thus obtained was fractionated onto the liquid chromatography(μ-Bondapak $C_{18}$ Steel Column, 19mm x 30cm) using 15%-methanol solution as an eluent to yield 650mg of the title compound as a white solid.

| | |
|---|---|
| MS(FAB, M + 1): | 580 |
| NMR(δ, DMSO-$d_6$): | 1.21(t, 3H), 3.42(ABq, 2H), 3.68(s, 3H), 4.15(q, 2H), 4.47(ABq, 2H), 5.01(d, 1H), 5.62(dd, 1H), 8.23(s, 2H), 9.07(s, 1H), 9.54(d, 1H) |
| IR(KBr, $cm^{-1}$): | 1768(β-lactam), 1690, 1630, 1575 |

Examples 73 to 82

The same procedures as described in Example 72 were repeated using paramethoxybenzyl 3-chloromethyl-7-[(Z)-2-(ethoxyimino)-2-(5-amino-1,2,4-thiadiazol-3-yl) acetamido]-3-cephem-4-carboxylate and thiones synthesized in Preparation Examples 4, 6 to 10, 16, 17, 20, 22 and 23. The produced compounds are listed in Table 8.

Table 8

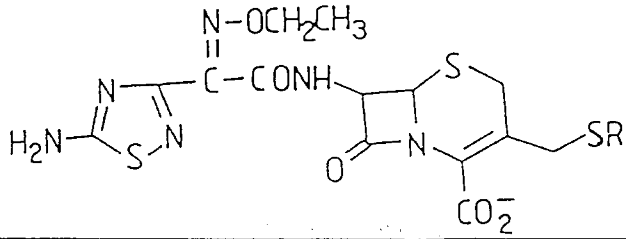

| Ex . | R | MS(FAB, M+1) | NMR( δ , DMSO-$d_6$ ) | IR(KBr, $cm^{-1}$ ) |
|---|---|---|---|---|
| 73 | 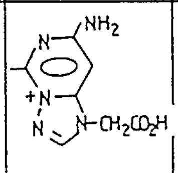 | 624 | * 1.27(t,3H), 3.64(ABq,2H), 4.18 (q,2H), 4.54(ABq,2H), 4.92(s,2H) 5.15(d,1H), 5.72(d,1H), 6.27 (s,1H), 8.69(s,1H) | 1770(β-lactam), 1680, 1640, 1570 |
| 74 | 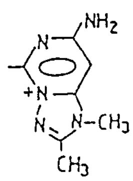 | 594 | 1.25(t,3H), 2.54(s,3H), 3.32 (ABq,2H), 3.84(s,3H), 4.16(q,2H) 4.42(ABq,2H), 5.04(d,1H), 5.67 (dd,1H), 6.17(s,1H), 7.32(s,1H), 7.85(s,2H), 9.56(d,1H) | 1765(β-lactam), 1695, 1630, 1570 |
| 75 | 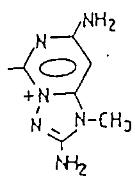 | 595 | 1.26(t,3H), 3.40(s,3H), 3.42 (ABq,2H), 4.12(q,2H), 4.53(ABq, 2H), 5.01(d,1H), 5.54(dd,1H), 6.16(s,1H), 7.76(s,2H), 7.98(s, 2H), 8.17(s,2H), 9.48(d,1H) | 1768(β-lactam), 1680, 1635, 1560 |
| 76 | 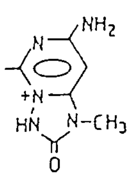 | 596 | 1.27(t,3H), 3.45(ABq,2H), 3.52 (s,3H), 4.13(q,2H), 4.16(ABq,2H) 4.98(d,1H), 5.64(dd,1H), 6.24 (s,1H), 7.24(s,2H), 7.53(s,2H), 9.52(d,1H) | 1770(β-lactam), 1690, 1640, 1580 |
| 77 | 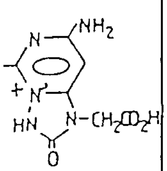 | 640 | 1.26(t,3H), 3.52(ABq,2H), 4.14 (q,2H), 4.46(ABq,2H), 4.94(s,2H) 5.02(d,1H), 5.74(dd,1H), 6.30 (s,1H), 7.21(s,2H), 7.56(s,2H), 9.54(d,1H) | 1765(β-lactam), 1695, 1650, 1570 |
| 78 | 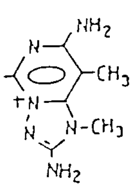 | 609 | 1.25(t,3H), 2.23(s,3H), 3.40(s, 3H), 3.45(ABq,2H), 4.13(q,2H), 4.55(ABq,2H), 5.01(d,1H), 5.67 (dd,1H), 7.76(s,2H), 7.98(s,2H), 8.17(s,2H), 9.52(d,1H) | 1769(β-lactam), 1695, 1630, 1570 |

Table 8(continued)

| Ex . | R | MS(FAB, M+1) | NMR(δ, DMSO-$d_6$) | IR(KBr, $cm^{-1}$) |
|---|---|---|---|---|
| 79 | 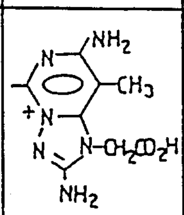 | 653 | 1.26(t,3H), 2.21(s,3H), 3.48 (ABq,2H), 4.13(q,2H), 4.47(ABq, 2H), 4.98(s,2H), 5.01(d,1H), 5.72(dd,1H), 7.22(s,2H), 7.51 (s,2H), 8.16(s,2H), 9.47(d,1H) | 1765(β-lactam), 1680, 1650, 1580 |
| 80 | 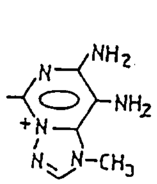 | 595 | 1.27(t,3H), 3.43(ABq,2H), 3.71 (s,3H), 4.15(q,2H), 4.43(ABq,2H) 5.11(d,1H), 5.62(dd,1H), 7.25 (s,2H), 7.62(s,2H), 9.05(s,1H), 9.52(d,1H) | 1768(β-lactam), 1670, 1643, 1515 |
| 81 | 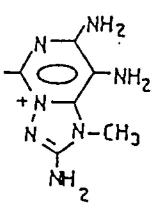 | 610 | 1.25(t,3H), 3.45(s,3H), 3.54 (ABq,2H), 4.12(q,2H), 4.46(ABq, 2H), 5.04(d,1H), 5.68(dd,1H), 7.21(s,2H), 7.72(s,2H), 7.94 (s,2H), 8.05(s,2H), 9.54(d,1H) | 1765(β-lactam), 1690, 1680, 1590 |
| 82 | 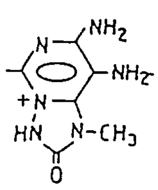 | 611 | 1.25(t,3H), 3.47(ABq,2H), 3.62 (s,3H), 4.11(q,2H), 4.57(ABq,2H) 4.97(d,1H), 5.64(dd,1H), 7.27 (s,2H), 7.45(s,2H), 8.21(s,2H), 9.46(d,1H) | 1770(β-lactam), 1685, 1670, 1650 1590 |

* NMR(δ, $D_2O$ + $NaHCO_3$)

Example 83

Synthesis of 7-[(Z)-2-(2-aminothiazol-4-yl)-2-(1-carboxycyclopentoxyimino)acetamido]-3-(7-amino-1-methyl-[1,2,4] triazolo[1,5-c]pyrimidinium-5-yl)thiomethyl-3-cephem-4-carboxylate

To a solution of 2g of paramethoxybenzyl 3-chloromethyl-7-{(Z)-2-[1-(diphenylmethoxycarbonyl) cyclopentoxyimino)-2-[2-(triphenylmethyl)aminothiazol-4-yl]acetamido}-3-cephem-4-carboxylate dissolved in 10ml of dimethyl sulfoxide was added 400mg of 7-amino-1-methyl [1,2,4]triazolo[1,5-c]pyrimidine-5-thione prepared in Preparation Example 4; and the resulting mixture was stirred for 2 hours at a room temperature. To the reaction solution was added 50ml of diethyl ether; and the mixture was stirred vigorously. After diethyl ether was removed from the mixture, 15ml of dichloromethane was added to the residue. To the resulting solution was added dropwise slowly 100ml of ethyl ether; and the mixture was filtered. The resulting solid was washed with 50ml of ethyl ether, dried and dissolved in 10ml of phenol. To the resulting solution was added 1.0ml of concentrated hydrochloric acid; the mixture was stirred at 50°C for 2 hours and cooled to a room temperature; and 100ml of acetone was added thereto. The resulting mixture was filtered; and the resulting solid was washed with 40ml of acetone and dried to yield 1.4g of an ivory solid. The solid thus obtained was fractionated onto the liquid chromatography(μ-Bondapak $C_{18}$ Steel Column,

19mm x 30cm) using 15%-methanol solution as an eluent to yield 640mg of the title compound as a white solid.

MS(FAB, M + 1): 663

NMR(δ, $D_2O$ + $NaHCO_3$): 1.71(m, 4H), 2.11(m, 4H), 3.63 (ABq, 2H), 3.77(s, 3H), 4.45 (ABq, 2H), 5.17(d, 1H), 5.73 (d, 1H), 6.39(s, 1H), 6.93(s, 1H), 8.67(s, 1H)

IR(KBr, $cm^{-1}$): 1770(β-lactam), 1670, 1610, 1560

Example 84

Synthesis of 7-[(Z)-2-(2-aminothiazol-4-yl)-2-(1-carboxycyclopentoxyimino)acetamido]-3-(2,7-diamino-1-methyl [1,2,4]triazolo[1,5-c]pyrimidinium-5-yl)thiomethyl-3-cephem-4-carboxylate

To a solution of 2g of paramethoxybenzyl 3-chloromethyl-7-{(Z)-2-[2-(triphenylmethyl)aminothiazol-4-yl]-2-[1-(diphenylmethoxycarbonyl)cyclopentoxyimino]acetamido}-3-cephem-4-carboxylate dissolved in 10ml of dimethyl sulfoxide was added 400mg of 2,7-diamino-1-methyl[1,2,4] triazolo[1,5-c]pyrimidine-5-thione prepared in Preparation Example 3; and the resulting mixture was stirred for 2 hours at a room temperature. To the reaction solution was added 50ml of diethyl ether; and the mixture was stirred vigorously. After diethyl ether was removed from the mixture, 15ml of dichloromethane was added to the residue, To the resulting solution was added dropwise slowly 100ml of ethyl ether; and the mixture was filtered. The resulting solid was washed with 50ml of ethyl ether, dried and dissolved in 10ml of phenol. To the resulting solution was added 1.0ml of concentrated hydrochloric acid; the mixture was stirred at 50°C for 2 hours and cooled to a room temperature; and 100ml of acetone was added thereto. The resulting mixture was filtered; and the resulting solid was washed with 40ml of acetone and dried to yield 1.4g of an ivory solid. The solid thus obtained was fractionated onto the liquid chromatography(μ-Bondapak $C_{18}$ Steel Column, 19mm x 30cm) using 15%-methanol solution as an eluent to yield 620mg of the title compound as a white solid.

MS(FAB, M + 1): 678

NMR(δ, $D_2O$ + $NaHCO_3$): 1.72(m, 4H), 2.03(m, 4H), 3.51 (s, 3H), 3.61(ABq, 2H), 4.40 (ABq, 2H), 5.13(d, 1H), 5.71 (d, 1H), 6.21(s, 1H), 6.92(s, 1H)

IR(KBr, $cm^{-1}$): 1765(β-lactam), 1680, 1620, 1550

Example 85

Synthesis of 7-[(Z)-2-(2-aminothiazol-4-yl)-2-((S)-1-carboxyprop-1-oxyimino)acetamido]-3-(7-amino-1-methyl [1,2,4]triazolo[1,5-c]pyrimidinium-5-yl)thiomethyl-3-cephem-4-carboxylate

To a solution of 2g of the compound prepared in Preparation Example 52 dissolved in 15ml of dimethyl sulfoxide was added 420mg of 7-amino-1-methyl[1,2,4] triazolo[1,5-c]pyrimidine-5-thione prepared in Preparation Example 4; and the resulting mixture was stirred for 3 hours at a room temperature and extracted with a mixture of 30ml of tetrahydrofuran and 30ml of ethyl acetate. The extract was washed with 150ml of saturated sodium chloride solution; and the organic phase was separated therefrom, dried over magnesium sulphate, distilled under a reduced pressure to remove the solvent and dried. The resulting solid was dissolved in 6ml of anisole; and the reaction solution was cooled to 0°C to 4°C. After 10ml of trifluoroacetic acid was added dropwise thereto, the mixture was stirred at a room temperature for 40 minutes and cooled to -20°C to -30°C; and 80ml of diethyl ether was added dropwise thereto. The resulting mixture was filtered; and the resulting solid was washed with 70ml of acetone and dried to yield 1.2g of an ivory powdery solid. The solid thus obtained was fractionated onto the liquid chromatography(μ-Bondapak $C_{18}$ Steel Column, 19mm x 30cm) using 10%-methanol solution as an eluent to yield 280mg of the title compound as a white powdered solid.

MS(FAB, M + 1): 637

NMR(δ, $D_2O$ + $NaHCO_3$): 0.96(t, 3H), 1.83(q, 2H), 3.64 (ABq, 2H), 3.72(s, 3H), 4.42 (ABq, 2H), 4.51(t, 1H), 5.15 (d, 1H), 5.74(d, 1H), 6.26 (s, 1H), 6.84(s, 1H), 8.65(s, 1H)

IR(KBr, $cm^{-1}$): 1770(β-lactam), 1670, 1620, 1580

$[\alpha]_D^{20}$: -101.20(C = 0.0098, $H_2O$)

Example 86

Synthesis of 7-[(Z)-2-(2-aminothiazol-4-yl)-2-((R)-1-carboxyprop-1-oxyimino)acetamido]-3-(7-amino-1-methyl [1,2,4]triazolo[1,5-c]pyrimidinium-5-yl)thiomethyl-3-cephem-4-carboxylate

To a solution of 1.5g of the compound prepared in Preparation Example 51 dissolved in 10ml of dimethyl sulfoxide was added 350mg of 7-amino-1-methyl[1,2,4] triazolo[1,5-c)pyrimidine-5-thione prepared in Preparation Example 4; and the resulting mixture was stirred for 3 hours at a room temperature and extracted with a mixture solvent of 20ml of tetrahydrofuran and 20ml of ethyl acetate. The extract was washed with 100ml of saturated sodium chloride solution; and the organic phase was separated therefrom, dried over anhydrous magnesium sulphate, distilled under a reduced pressure to remove the solvent and dried. The resulting solid was dissolved in 4ml of anisole; and the reaction solution was cooled to 0°C to 4°C. After 10ml of trifluoroacetic acid was added dropwise thereto, the mixture was stirred at a room temperature for 40 minutes and cooled to -20°C to -30°C; and 50ml of diethyl ether was added dropwise thereto. The resulting mixture was filtered; and the resulting solid was washed with 50ml of acetone and dried to yield 1.0g of a white powdery solid. The solid thus obtained was fractionated onto the liquid chromatography ($\mu$-Bondapak $C_{18}$ Steel Column, 19mm x 30cm) using 25%-methanol solution as an eluent to yield 480mg of the title compound as a white powdery solid.

| | |
|---|---|
| MS(FAB, M + 1): | 637 |
| NMR($\delta$, $D_2O$ + $NaHCO_3$): | 0.97(t, 3H), 1.82(q, 2H), 3.61 (ABq, 2H), 3.73(s, 3H), 4.39 (ABq, 2H), 4.46(t, 1H), 5.14 (d, 1H), 5.70(d, 1H), 6.29(s, 1H), 6.82(s, 1H), 8.62(s, 1H) |
| IR(KBr, $cm^{-1}$): | 1770($\beta$-lactam), 1670, 1620, 1580 |
| $[\alpha]_D^{20}$: | -67.47(C = 0.0096, $H_2O$) |

Example 87

Synthesis of 7-[(Z)-2-(2-aminothiazol-4-yl)-2-((S)-1-carboxyprop-1-oxyimino)acetamido]-3-(2,7-diamino-1-methyl[1,2,4,]triazolo[1,5-c]pyrimidinium-5-yl) thiomethyl-3-cephem-4-carboxylate

The same procedures as described in Example 85 were repeated using 2,7-diamino-1-methyl[1,2,4]-triazolo[1,5-c] pyrimidine-5-thione prepared in Preparation Example 6 in place of 7-amino-1-methyl[1,2,4]-triazolo[1,5-c] pyrimidine-5-thione to yield 260mg of the title compound as a white solid.

| | |
|---|---|
| MS(FAB, M + 1): | 652 |
| NMR($\delta$, $D_2O$ + $NaHCO_3$): | 0.96(t, 3H), 1.83(q, 2H), 3.49 (s, 3H), 3.57(ABq, 2H), 4.43 (ABq, 2H), 4.49(t, 1H), 5.15 (d, 1H), 5.70(d, 1H), 6.18(s, 1H), 6.86(s, 1H) |
| IR(KBr, $cm^{-1}$): | 1775($\beta$-lactam), 1680, 1620, 1570 |
| $[\alpha]_D^{20}$: | -87.09(C = 0.0098, $H_2O$) |

Example 88

Synthesis of 7-[(Z)-2-(2-aminothiazol-4-yl)-2-((R)-1-carboxyprop-1-oxyimino)acetamido]-3-(2,7-diamino-1-methyl[1,2,4]triazolo[1,5-c]pyrimidinium-5-yl)thiomethyl-3-cephem-4-carboxylate

The same procedures as described in Example 86 were repeated using 2,7-diamino-1-methyl[1,2,4]-triazolo[1,5-c] pyrimidine-5-thione prepared in Preparation Example 6 in place of 7-amino-1-methyl[1,2,4]-triazolo[1,5-c] pyrimidine-5-thione to yield 490mg of the title compound as a white solid.

| | |
|---|---|
| MS(FAB, M + 1): | 652 |
| NMR($\delta$, $D_2O$ + $NaHCO_3$): | 0.96(t, 3H), 1.84(q, 2H), 3.48 (s, 3H), 3.56(ABq, 2H), 4.37 (ABq, 2H), 4.48(t, 1H), 5.16 (d, 1H), 5.68(d, 1H), 6.13(s, 1H), 6.81(s, 1H) |
| IR(KBr, $cm^{-1}$): | 1775($\beta$-lactam), 1680, 1620, 1570 |
| $[\alpha]_D^{20}$: | -57.36(C = 0.0103, $H_2O$) |

Example 89

Synthesis of 7-[(Z)-2-(2-aminothiazol-4-yl)-2-((S)-1-carboxyprop-1-oxyimino)acetamido]-3-(7-amino-1,8-dimethyl[1,2,4]triazolo[1,5-c]pyrimidinium-5-yl) thiomethyl-3-cephem-4-carboxylate

The same procedures as described in Example 85 were repeated using 7-amino-1,8-dimethyl[1,2,4]-triazolo[1,5-c]pyrimidine-5-thione prepared in Preparation Example 5 in place of 7-amino-1-methyl[1,2,4]-triazolo[1,5-c] pyrimidine-5-thione to yield 255mg of the title compound as a white solid.

| | |
|---|---|
| MS(FAB, M + 1): | 651 |
| NMR($\delta$, $D_2O$ + $NaHCO_3$): | 0.95(t, 3H), 1.82(q, 2H), 2.20 (s, 3H), 3.54(ABq, 2H), 4.36 (ABq, 2H), 4.43(t, 1H), 5.12 (d, 1H), 5.67(d, 1H), 6.84(s, 1H), 8.64(s, 1H) |
| IR(KBr, $cm^{-1}$): | 1770($\beta$-lactam), 1675, 1620, 1580 |
| $[\alpha]_D^{20}$: | -102.52(C = 0.0102, $H_2O$) |

Example 90

Synthesis of 7-[(Z)-2-(2-aminothiazol-4-yl)-2-((R)-1-carboxyprop-1-oxyimino)acetamido]-3-(7-amino-1,8-dimethyl[1,2,4]triazolo[1,5-c]pyrimidinium-5-yl) thiomethyl-3-cephem-4-carboxylate

The same procedures as described in Example 86 were repeated using 7-amino-1,8-dimethyl[1,2,4]-triazolo[1,5-c] pyrimidine-5-thione prepared in Preparation Example 5 in place of 7-amino-1-methyl[1,2,4]-triazolo[1,5-c] pyrimidine-5-thione to yield 485mg of the title compound as a white solid.

| | |
|---|---|
| MS(FAB, M + 1): | 651 |
| NMR($\delta$, $D_2O$ + $NaHCO_3$): | 0.96(t, 3H), 1.84(q, 2H), 2.23 (s, 3H), 3.58(ABq, 2H), 4.42 (ABq, 2H), 4.45(t, 1H), 5.15 (d, 1H), 5.69(d, 1H), 6.82(s, 1H), 8.62(s, 1H) |
| IR(KBr, $cm^{-1}$): | 1770($\beta$-lactam), 1675, 1620, 1580 |
| $[\alpha]_D^{20}$: | -69.27(C = 0.0096, $H_2O$) |

Example 91

Synthesis of 7-[(Z)-2-(2-aminothiazol-4-yl)-2-((S)-1-carboxyprop-1-oxyimino)acetamido]-3-(2,7-diamino-1,8-dimethyl[1,2,4]triazolo[1,5-c]pyrimidinium-5-yl) thiomethyl-3-cephem-4-carboxylate

The same procedures described in Example 85 were repeated using 2,7-diamino-1,8-dimethyl[1,2,4]-triazolo [1,5-c]pyrimidine-5-thione prepared in Preparation Example 7 in place of 7-amino-1-methyl[1,2,4]-triazolo [1,5-c]pyrimidine-5-thione to yield 265mg of the title compound as a white solid.

| | |
|---|---|
| MS(FAB, M + 1): | 666 |
| NMR($\delta$, $D_2O$ + $NaHCO_3$): | 0.97(t, 3H), 1.84(q, 2H), 2.23 (s, 3H), 3.52(ABq, 2H), 3.69(s, 3H), 4.42(ABq, 2H), 4.48(t, 1H), 5.13 (d, 1H), 5.67(d, 1H), 6.82(s, 1H) |
| IR(KBr, $cm^{-1}$): | 1780($\beta$-lactam), 1660, 1630, 1590 |
| $[\alpha]_D^{20}$: | -87.25(C = 0.0104, $H_2O$) |

Example 92

Synthesis of 7-[(Z)-2-(2-aminothiazol-4-yl)-2-((R)-1-carboxyprop-1-oxyimino)acetamido]-3-(2,7-diamino-1,8-dimethyl[1,2,4]triazolo[1,5-c]pyrimidinium-5-yl) thiomethyl-3-cephem-4-carboxylate

The same procedures described in Example 86 were repeated using 2,7-diamino-1,8-dimethyl[1,2,4]-triazolo [1,5-c]pyrimidine-5-thione prepared in Preparation Example 7 in place of 7-amino-1-methyl[1,2,4]-triazolo [1,5-c]pyrimidine-5-thione to yield 470mg of the title compound as a white solid.

| | |
|---|---|
| MS(FAB, M + 1): | 666 |
| NMR($\delta$, $D_2O$ + $NaHCO_3$): | 0.96(t, 3H), 1.82(q, 2H), 2.22 (s, 3H), 3.48(ABq, 2H), 3.71(s, 3H), 4.37(ABq, 2H), 4.47(t, 1H), 5.13 (d, 1H), 5.64(d, 1H), 6.80(s, 1H) |
| IR(KBr, $cm^{-1}$): | 1780($\beta$-lactam), 1660, 1630, 1590 |
| $[\alpha]_D^{20}$: | -58.49(C = 0.0095, $H_2O$) |

Example 93

Synthesis of 7-[(Z)-2-(2-aminothiazol-4-yl)-2-((S)-1-carboxyprop-1-oxyimino)acetamido]-3-(7-amino-1,2-dimethyl[1,2,4]triazolo[1,5-c]pyrimidinium-5-yl) thiomethyl-3-cephem-4-carboxylate

The same procedures described in Example 86 were repeated using 7-amino-1,2-dimethyl[1,2,4]-triazolo[1,5-c] pyrimidine-5-thione prepared in Preparation Example 10 in place of 7-amino-1-methyl[1,2,4]-triazolo[1,5-c] pyrimidine-5-thione to yield 260mg of the title compound as a white solid.

| | |
|---|---|
| MS(FAB, M + 1): | 651 |
| NMR($\delta$, $D_2O$ + $NaHCO_3$): | 0.97(t, 3H), 1.81(q, 2H), 2.52 (s, 3H), 3.54(ABq, 2H), 3.63(s, 3H), 4.46(ABq, 2H), 4.47(t, 1H), 5.14 (d, 1H), 5.68(d, 1H), 6.18(s, 1H), 6.91(s, 1H) |
| IR(KBr, $cm^{-1}$): | 1775($\beta$-lactam), 1670, 1620, 1590 |
| $[\alpha]_D^{20}$: | -104.79(C = 0.0097, $H_2O$) |

Example 94

Synthesis of 7-[(Z)-2-(2-aminothiazol-4-yl)-2-((R)-1-carboxyprop-1-oxyimino)acetamido]-3-(7-amino-1,2-dimethyl[1,2,4]triazolo[1,5-c]pyrimidinium-5-yl) thiomethyl-3-cephem-4-carboxylate

The same procedures described in Example 86 were repeated using 7-amino-1,2-dimethyl[1,2,4]-triazolo[1,5-c]pyrimidine-5-thione prepared in Preparation Example 10 in place of 7-amino-1-methyl[1,2,4]-triazolo[1,5-c] pyrimidine-5-thione to yield 465mg of the title compound as a white solid.

| | |
|---|---|
| MS(FAB, M + 1): | 651 |
| NMR($\delta$, $D_2O$ + $NaHCO_3$): | 0.98(t, 3H), 1.83(q, 2H), 2.54 (s, 3H), 3.58(ABq, 2H), 3.61(s, 3H), 4.38(ABq, 2H), 4,46(t, 1H), 5.15 (d, 1H), 5.67(d, 1H), 6.17(s, 1H), 6.89(s, 1H) |
| IR(KBr, $cm^{-1}$): | 1775($\beta$-lactam), 1670, 1620, 1590 |
| $[\alpha]_D^{20}$: | -72.27(C = 0.0095, $H_2O$) |

Example 95

Synthesis of 7-[(Z)-2-(2-aminothiazol-4-yl)-2-(2-carboxybut-2-oxyimino)acetamido]-3-(7-amino-1-methyl [1,2,4]triazolo[1,5-c]pyrimidinium-5-yl)thiomethyl-3-cephem-4-carboxylate(a) and (b)

To a solution of 2g of compound prepared in Preparation Example 53 dissolved in 15ml of dimethyl sulfoxide was added 350mg of 7-amino-1-methyl[1,2,4] triazolo[1,5-c]pyrimidine-5-thione prepared in Preparation Example 4; and the resulting mixture was stirred for 2 hours at a room temperature. To the reaction solution was added 50ml of diethyl ether; and the mixture was stirred vigorously. After the organic phase was removed from the mixture, 20ml of dichloromethane was added to the residue. To the resulting solution was added dropwise slowly 100ml of diethyl ether; and the mixture was filtered. The resulting solid was washed with 50ml of diethyl ether, dried and dissolved in 4ml of anisole. The reaction solution was cooled to 0 to 4°C; and after 80ml of trifluoroacetic acid was added dropwise thereto, the resulting mixture was stirred at a room temperature for 50 minutes and cooled to -20 to -30°C. After 50ml of diethyl ether was added thereto, the resulting mixture was filtered; and the resulting solid was washed with 100ml of acetone and dried to yield 1.2g of an ivory powdery solid which is a mixture of diastereo isomers. The solid thus obtained was fractionated onto the liquid chromatography($\mu$-Bondapak $C_{18}$ Steel Column, 19mm x 30cm) using 20% methanol solution containing 0.5% acetic acid as an eluent to yield 190mg and 180mg of the title compound (a) of short retention time and (b) of long retention time, respectively, as white powdery solids.

| | |
|---|---|
| MS(FAB, M + 1): | 651 |
| NMR($\delta$, $D_2O$ + $NaHCO_3$): | |
| (a): | 0.89(t, 3H), 1.43(s, 3H), 1.86(q, 2H), 3.57 (ABq, 2H), 3.72(s, 3H), 4.43(ABq, 2H), 5.14 (d, 1H), 5.71(d, 1H), 6.21(s, 1H), 6.90(s, 1H), 8.63(s, 1H) |
| (b): | 0.85(t, 3H), 1.42(s, 3H), 1.84(q, 2H), 3.56 (ABq, 2H), 3.72(s, 3H), 4.47(ABq, 2H), 5.12 (d, 1H), 5.73(d, 1H), 6.24(s, 1H), 6.89(s, 1H), 8.65(s, 1H) |
| IR(KBr, $cm^{-1}$): | 1775($\beta$-lactam), 1670, 1620, 1570 |
| $[\alpha]_D^{20}$: | |
| (a): | -93.75(C = 0.0101, $H_2O$) |
| (b): | -74.69(C = 0.0103, $H_2O$) |

Example 96

Synthesis of 7-[(Z)-2-(2-aminothiazol-4-yl)-2-(2-carboxybut-2-oxyimino)acetamido]-3-(2,7-diamino-1-methyl-[1,2,4]triazolo[1,5-c]pyrimidinium-5-yl)thiomethyl-3-cephem-4-carboxylate(a) and (b)

The same procedures as described in Example 95 were repeated using 2,7-diamino-1-methyl[1,2,4]-triazolo[1,5-c]pyrimidine-5-thione prepared in Preparation Example 6 in place of 7-amino-1-methyl[1,2,4]-triazolo[1,5-c] pyrimidine-5-thione to yield 195mg and 200mg of the title compound(a) and (b), respectively, as white solids.

MS(FAB, M + 1): 666

NMR(δ, $D_2O$ + $NaHCO_3$):

(a): 0.86(t, 3H), 1.43(s, 3H), 1.82(q, 2H), 3.48 (s, 3H), 3.56(ABq, 2H), 4.39(ABq, 2H), 5.14 (d, 1H), 5.71(d, 1H), 6.17(s, 1H), 6.87(s, 1H)

(b): 0.83(t, 3H), 1.42(s, 3H), 1.84(q, 2H), 3.63 (ABq, 2H), 3.48(s, 3H), 4.37(ABq, 2H), 5.12 (d, 1H), 5.72(d, 1H), 6.18(s, 1H), 6.88(s, 1H)

IR(KBr, $cm^{-1}$): 1770(β-lactam), 1680, 1620, 1560

$[\alpha]_D^{20}$:

(a): -88.94(C = 0.0096, $H_2O$)

(b): -69.82(C = 0.0102, $H_2O$)

Example 97

Synthesis of 7-[(Z)-2-(2-aminothiazol-4-yl)-2-(2-carboxybut-2-oxyimino)acetamido]-3-(7-amino-1,8-dimethyl-[1,2,4]triazolo[1,5-c]pyrimidinium-5-yl) thiomethyl-3-cephem-4-carboxylate(a) and (b)

The same procedures described in Example 95 were repeated using 7-amino-1,8-dimethyl[1,2,4]-triazolo[1,5-c]pyrimidine-5-thione prepared in Preparation Example 5 in place of 7-amino-1-methyl[1,2,4]-triazolo[1,5-c] pyrimidine-5-thione to yield 200mg and 190mg of the title compound(a) and (b), respectively, as white solids.

MS(FAB, M + 1): 665

NMR(δ, $D_2O$ + $NaHCO_3$):

(a): 0.88(t, 3H), 1.43(s, 3H), 1.82(q, 2H), 2.24 (s, 3H), 3.68(ABq, 2H), 3.69(s, 3H), 4.42 (ABq, 2H), 5.14(d, 1H), 5.73(d, 1H), 6.20(s, 1H), 6.91(s, 1H), 8.63-(s, 1H)

(b): 0.87(t, 3H), 1.44(s, 3H), 1.84(q, 2H), 2.22 (s, 3H), 3.58(ABq, 2H), 3.63(s, 3H), 4.38 (ABq, 2H), 5.12(d, 1H), 5.69(d, 1H), 6.19(s, 1H), 6.89(s, 1H), 8.61-(s, 1H)

IR(KBr, $cm^{-1}$): 1775(β-lactam), 1670, 1620, 1580

$[\alpha]_D^{20}$:

(a): -105.36(C = 0.0099, $H_2O$)

(b): -84.29(C = 0.0101, $H_2O$)

Example 98

Synthesis of 7-[(Z)-2-(2-aminothiazol-4-yl)-2-(2-carboxybut-2-oxyimino)acetamido]-3-(2,7-diamino-1,8-dimethyl[1,2,4]triazolo[1,5-c]pyrimidinium-5-yl) thiomethyl-3-cephem-4-carboxylate(a) and (b)

The same procedures described in Example 95 were repeated using 2,7-diamino-1,8-dimethyl[1,2,4]-triazolo [1,5-c]pyrimidine-5-thione prepared in Preparation Example 7 in place of 7-amino-1-methyl[1,2,4]-triazolo [1,5-c]pyrimidine-5-thione to yield 195mg and 190mg of the title compound(a) and (b), respectively, as white solids.

MS(FAB, M + 1): 680

NMR(δ, $D_2O$ + $NaHCO_3$):

(a): 0.88(t, 3H), 1.44(s, 3H), 1.83(q, 2H), 2.24 (s, 3H), 3.55(ABq, 2H), 3.70(s, 3H), 4.36 (ABq, 2H), 5.10(d, 1H), 5.68(d, 1H), 6.91(s, 1H)

(b): 0.89(t, 3H), 1.43(s, 3H), 1.82(q, 2H), 2.23 (s, 3H), 3.55(ABq, 2H), 3.71(s, 3H), 4.34 (ABq, 2H), 5.10(d, 1H), 5.68(d, 1H), 6.88(s, 1H)

IR(KBr, $cm^{-1}$): 1775(β-lactam), 1680, 1630, 1590

$[\alpha]_D^{20}$:
(a): -103.35(C = 0.0098, $H_2O$)
(b): -79.48(C = 0.0097, $H_2O$)

Example 99

Synthesis of 7-[(Z)-2-(2-aminothiazol-4-yl)-2-(2-carboxybut-2-oxyimino)acetamido]-3-(7-amino-1,2-dimethyl [1,2,4]triazolo[1,5-c]pyrimidinium-5-yl)thiomethyl-3-cephem-4-carboxylate(a) and (b)

The same procedures described in Example 95 were repeated using 7-amino-1,2-dimethyl[1,2,4]-triazolo[1,5-c]pyrimidine-5-thione prepared in Preparation Example 10 in place of 7-amino-1-methyl[1,2,4]-triazolo[1,5-c] pyrimidine-5-thione to yield 180mg and 185mg of the title compound(a) and (b), respectively, as white solids.

MS(FAB, M + 1): 665
NMR(δ, $D_2O$ + $NaHCO_3$):
(a): 0.87(t, 3H), 1.42(s, 3H), 1.84(q, 2H), 2.53 (s, 3H), 3.59(ABq, 2H), 3.61(s, 3H), 4.43 (ABq, 2H), 5.12(d, 1H), 5.69(d, 1H), 6.20(s, 1H), 6.93(s, 1H), 7.41-(d, 1H), 7.52(d, 1H)
(b): 0.88(t, 3H), 1.43(s, 3H), 1.82(q, 2H), 2.51 (s, 3H), 3.58(ABq, 2H), 3.64(s, 3H), 4.47 (ABq, 2H), 5.14(d, 1H), 5.70(d, 1H), 6.21(s, 1H), 6.91(s, 1H), 7.42-(d, 1H), 7.53(d, 1H)
IR(KBr, $cm^{-1}$): 1775(β-lactam), 1670, 1620, 1560
$[\alpha]_D^{20}$:
(a): -96.32(C = 0.0104, $H_2O$)
(b): -80.27(C = 0.0098, $H_2O$)

Example 100

Synthesis of 7-[(Z)-2-(2-aminothiazol-4-yl)-2-(2-carboxyprop-2-oxyimino)acetamido]-3-(1-methyl[1,3] imidazo[1,2-c]pyrimidinium-5-yl)thiomethyl-3-cephem-4-carboxylate

To a solution of 1g of paramethoxybenzyl 3-chloromethyl-7-{(Z)-2-(2-t-butoxycarbonylprop-2-oxyimino)-2-[2-(triphenylmethyl)aminothiazol-4-yl]acetamido)-3-cephem-4-carboxylate dissolved in 5ml of dimethyl sulfoxide was added 150mg of 1-methyl[1,3]imidazo[1,2-c]pyrimidine-5 thione prepared in Preparation Example 29; and the resulting mixture was stirred for 2 hours at a room temperature. To the reaction solution was added 3 x 50ml of ethyl ether; and the mixture was stirred vigorously. After ethyl ether was removed from the mixture, 10ml of dichloromethane was added to the residue. To the resulting solution was added dropwise slowly 100ml of ethyl ether; and the mixture was filtered. The resulting solid was washed with 10ml of ethyl ether, dried and dissolved in 5ml of phenol. To the resulting solution was added 0.5ml of concentrated hydrochloric acid; and the mixture was stirred at 50 °C for 2 hours. The reaction solution was cooled to a room temperature; and 60ml of acetone was added thereto. The resulting mixture was filtered; and the resulting solid was washed with 20ml of acetone and dried to yield 720mg of an ivory solid. The solid thus obtained was fractionated onto the liquid chromatography(μ-Bondapak $C_{18}$ Steel Column, 19mm x 30cm) using 10%-methanol solution as an eluent to yield 340mg of the title compound as a white solid.

MS(FAB, M + 1): 633
NMR(δ, $D_2O$ + $NaHCO_3$): 1.48(d, 6H), 3.62(ABq, 2H), 4.01 (s, 3H), 4.58(ABq, 2H), 5.12(d, 1H), 5.77(d, 1H), 6.91(s, 1H), 7.71 (d, 1H), 7.98(d, 1H), 8.07(d, 1H), 8.32(d, 1H)
IR(KBr, $cm^{-1}$): 1765(β-lactam), 1660, 1610, 1580

Examples 101 to 115

The same procedures as described in Example 100 were repeated using paramethoxybenzyl 3-chloromethyl-{(Z)-2-(2-t-butoxycarbonylprop-2-oxyimino)-2-[2-(triphenylmethyl)aminothiazol-4-yl]acetamido}-3-cephem-4-carboxylate and thiones prepared in Preparations Examples 30 to 42, 48 and 49. The produced compounds are listed in Table 9.

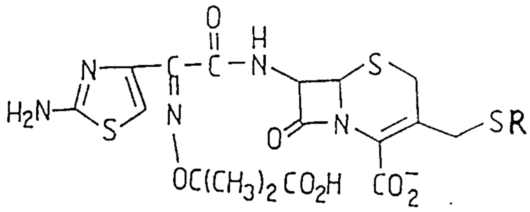

Table 9

| Ex. | R | MS(FAB, M+1) | NMR(δ, $D_2O/NaHCO_3$) | IR(KBr, $cm^{-1}$) |
|---|---|---|---|---|
| 101 | 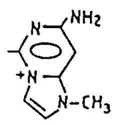 | 648 | 1.52(d,6H), 3.61(ABq,2H), 3.71 (s,3H), 4.53(ABq,2H), 5.15(d,1H), 5.80(d,1H), 6.28(s,1H), 6.95(s, 1H), 7.43(d,1H), 7.55(d,1H) | 1770(β-lactam), 1667, 1620, 1590 |
| 102 | 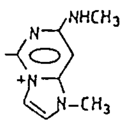 | 662 | 1.50(d,6H), 2.97(s,3H), 3.53 (ABq,2H), 3.73(s,3H), 4.43(ABq, 2H), 5.17(d,1H), 5.80(d,1H), 6.11(s,1H), 6.99(s,1H), 7.41 (d,1H), 7.53(d,1H) | 1765(β-lactam), 1670, 1610, 1595 |
| 103 | 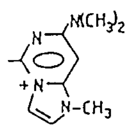 | 676 | 1.50(d,6H), 3.19(s,6H), 3.59 (ABq,2H), 4.52(ABq,2H), 5.23(d, 1H), 5.77(d,1H), 6.09(s,1H), 6.79(s,1H), 7.42(d,1H), 7.49 (d,1H) | 1760(β-lactam), 1670, 1600, 1580 |
| 104 | 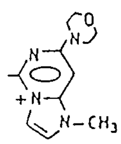 | 718 | 1.48(d,6H), 3.57(ABq,2H), 3.69 (s,3H), 3.67~3.82(m,8H), 4.43 (ABq,2H), 5.20(d,1H), 5.77(d,1H), 6.35(s,1H), 6.79(s,1H), 7.45 (d,1H), 7.54(d,1H) | 1775(β-lactam), 1710, 1630, 1580 |
| 105 | 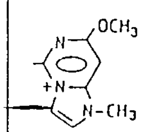 | 663 | 1.51(d,1H), 3.63(ABq,2H), 3.71 (s,3H), 4.07(s,3H), 4.53(ABq,2H), 5.15(d,1H), 5.80(d,1H), 6.21(s, 1H), 6.89(s,1H), 7.51(d,1H), 7.62(d,1H) | 1770(β-lactam), 1705, 1620, 1590 |
| 106 | 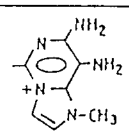 | 663 | 1.50(d,1H), 3.54(ABq,2H), 3.68 (s,3H), 4.41(ABq,2H), 5.11(d,1H), 5.77(d,1H), 6.87(s,1H), 7.42 (d,1H), 7.51(d,1H) | 1760(β-lactam), 1680, 1630, 1570 |

Table 9(continued)

| Ex. | R | MS(FAB, M+1) | NMR(δ, $D_2O/NaHCO_3$) | IR(KBr, $cm^{-1}$) |
|---|---|---|---|---|
| 107 | 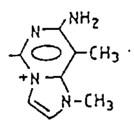 | 662 | 1.49(d,6H), 1.97(s,3H), 3.61 (ABq,2H), 3.70(s,3H), 4.49(ABq, 2H), 5.17(d,1H), 5.79(d,1H), 6.97(s,1H), 7.40(d,1H), 7.49 (d,1H) | 1765(β-lactam), 1695, 1620, 1590 |
| 108 | 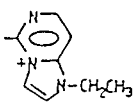 | 647 | 1.29(t,3H), 1.51(d,6H), 3.60 (ABq,2H), 4.19(q,2H), 4.57(ABq, 2H), 5.11(d,1H), 5.80(d,1H), 6.93(s,1H), 7.70(d,1H), 7.93 (d,1H), 8.07(d,1H), 8.32(d,1H) | 1765(β-lactam), 1690, 1610, 1580 |
| 109 | 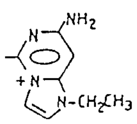 | 662 | 1.25(t,3H), 1.51(d,6H), 3.59 (ABq,2H), 4.21(q,2H), 4.54(ABq, 2H), 5.16(d,1H), 5.79(d,1H), 6.25(s,1H), 6.98(s,1H), 7.42(d, 1H), 7.53(d,1H) | 1780(β-lactam), 1670, 1620, 1595 |
| 110 | 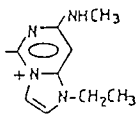 | 676 | 1.27(t,3H), 1.50(d,6H), 2.98(s, 3H), 3.55(ABq,2H), 4.27(q,2H), 4.55(ABq,2H), 5.17(d,1H), 5.81 (d,1H), 6.13(s,1H), 6.98(s,1H), 7.40(d,1H), 7.52(d,1H) | 1775(β-lactam), 1675, 1610, 1590 |
| 111 | 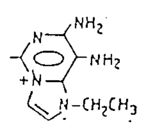 | 677 | 1.26(t,3H), 1.49(d,6H), 3.55 (ABq,2H), 4.19(q,2H), 4.49(ABq, 2H), 5.15(d,1H), 5.78(d,1H), 6.89(s,1H), 7.41(d,1H), 7.52 (d,1H) | 1765(β-lactam), 1690, 1630, 1570 |
| 112 | 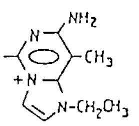 | 676 | 1.26(t,3H), 1.50(d,6H), 1.99(s, 3H), 3.55(ABq,2H), 4.20(q,2H), 5.18(d,1H), 5.81(d,1H), 6.90 (s,1H), 7.43(d,1H), 7.55(d,1H) | 1770(β-lactam), 1695, 1615, 1580 |

Table 9(continued)

| Ex. | R | MS(FAB, M+1) | NMR(δ, $D_2O/NaHCO_3$) | IR(KBr, $cm^{-1}$) |
|---|---|---|---|---|
| 113 | 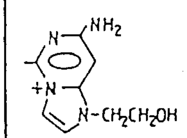 | 678 | 1.50(d,6H), 3.60(ABq,2H), 3.73 (t,2H), 4.23(t,2H), 4.59(ABq,2H), 5.21(d,1H), 5.85(d,1H), 6.27 (s,1H), 6.89(s,1H), 7.42(d,1H) 7.49(d,1H) | 1775(β-lactam), 1680, 1620, 1580 |
| 114 | 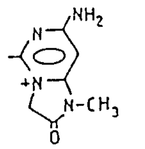 | 664 | 1.46(d,6H), 3.21(s,3H), 3.55 (ABq,2H), 4.42(ABq,2H), 4.80(s, 2H), 5.11(d,1H), 5.75(d,1H), 6.13(s,1H), 6.95(s,1H) | 1780(β-lactam), 1750, 1650, 1610 1590 |
| 115 | 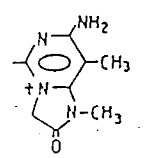 | 678 | 1.50(d,6H), 1.95(s,3H), 3.22 (s,3H), 3.57(ABq,2H), 4.45(ABq, 2H), 4.79(s,2H), 5.10(d,1H), 5.77(d,1H), 6.19(s,1H), 6.95 (s,1H) | 1775(β-lactam), 1740, 1680, 1620 1580 |

Example 116

Synthesis of 7-[(Z)-2-(2-aminothiazol-4-yl)-2-((S)-1-carboxyeth-1-oxyimino)acetamido]-3-(7-amino-1-methyl-[1,3] imidazo[1,2-c]pyrimidinium-5-yl)thiomethyl-3-cephem-4-carboxylate; and 7-[(Z)-2-(2-aminothiazol-4-yl)-2-((R)-1-carboxyeth-1-oxyimino)acetamido]-3-(7-amino-1-methyl[1,3] imidazo[1,2-c]pyrimidinium-5-yl)-thiomethyl-3-cephem-4-carboxylate

To a solution of 1.0g of paramethoxybenzyl 3-chloromethyl-7-{(Z)-2-((S,R)-1-t-butoxycarbonyleth-1-oxyimino)-2-[2-(triphenylmethyl)aminothiazol-4-yl]acetamido}-3-cephem-4-carboxylate dissolved in 5ml of dimethyl sulfoxide was added 150mg of 7-amino-1-methyl[1,3] imidazo[1,2-c]pyrimidine-5-thione prepared in Preparation Example 30; and the resulting mixture was stirred for 2 hours at a room temperature. To the reaction solution was added 3 x 50ml of ethyl ether; and the mixture was stirred vigorously. After ethyl ether was removed from the mixture, 10ml of dichloromethane was added to the residue. To the resulting solution was added dropwise slowly 100ml of ethyl ether; and the mixture was filtered. The resulting solid was washed with 10ml of ethyl ether, dried and dissolved in 5ml of phenol. To the resulting solution was added 0.5ml of concentrated hydrochloric acid; and the mixture was stirred at 50°C for 2 hours. The reaction solution was cooled to a room temperature; and 60ml of acetone was added thereto. The resulting mixture was filtered; and the resulting solid was washed with 20ml of acetone and dried to yield 610mg of a light yellow solid. The solid thus obtained was fractionated onto the liquid chromatography(μ-Bondapak $C_{18}$ Steel Column, 19mm x 30cm) using 5%-methanol solution as an eluent to yield 160mg and 140mg of the title compound as S and R forms, respectively.

| | |
|---|---|
| MS(FAB, M + 1): | 634 |
| NMR(δ, $D_2O$ + $NaHCO_3$): | |
| S: | 1.48(d, 3H), 3.55(ABq, 2H), 3.70(s, 3H), 4.45 (ABq, 2H), 4.63(q, 1H), 5.15(d, 1H), 5.86(d, 1H), 6.23(s, 1H), 6.97(s, 1H); 7.45(s, 1H), 7.56(s, 1H) |
| R: | 1.48(d, 3H), 3.55(ABq, 2H), 3.70(s, 3H), 4.45 (ABq, 2H), 4.63(q, 1H), 5.15(d, 1H), 5.80(d, 1H), 6.23(s, 1H), 6.97(s, 1H), 7.45(s, 1H), 7.56(s, 1H) |
| IR(KBr, $cm^{-1}$): | 1770(β-lactam), 1680, 1620, 1570 |

Examples 117 to 121

The same procedures as described in Example 116 were repeated using paramethoxybenzyl 3-chloromethyl-7-{(Z)-2-((R,S)-1-t-butoxycarbonyleth-1-oxyimino)-2-[2-(triphenylmethyl)aminothiazol-4-yl]-acetamido}-3-cephem-4-carboxylate and thiones synthesized in Preparation Examples 36, 38, 41, 48 and 49. The produced compounds are listed in Table 10.

Table 10

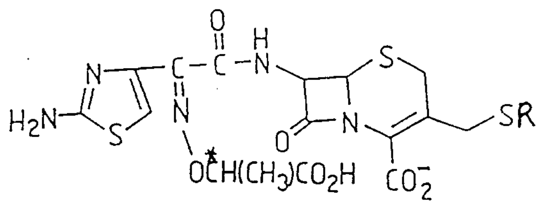

| Ex . | R | MS(FAB, M+1) | NMR(δ , $D_2O/NaHCO_3$) | IR(KBr, $cm^{-1}$) |
|---|---|---|---|---|
| 117 (S) | 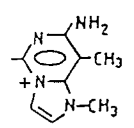 | 648 | 1.47(d,3H), 1.99(s,3H), 3.51 (ABq,2H), 3.71(s,3H), 4.47(ABq, 2H), 4.60(q,1H), 5.11(d,1H), 5.78(d,1H), 6.91(s,1H), 7.41 (d,1H), 7.49(d,1H) | 1770(β-lactam), 1680, 1620, 1570 |
| 117 (R) | | | 1.47(d,3H), 1.99(s,3H), 3.51 (ABq,2H), 3.71(s,3H), 4.47(ABq, 2H), 4.60(q,1H), 5.11(d,1H), 5.73(d,1H), 6.91(s,1H), 7.41 (d,1H), 7.49(d,1H) | |
| 118 (S) | 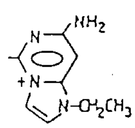 | 648 | 1.29(t,3H), 1.48(s,3H), 3.55 (ABq,2H), 4.21(q,2H), 4.45(ABq, 2H), 4.63(q,1H), 5.18(d,1H), 5.83(d,1H), 6.13(s,1H), 6.89 (s,1H), 7.40(d,1H), 7.48(d,1H) | 1780(β-lactam), 1670, 1620, 1580 |
| 118 (R) | | | 1.29(t,3H), 1.48(s,3H), 3.55 (ABq,2H), 4.21(q,2H), 4.45(ABq, 2H), 4.63(q,1H), 5.18(d,1H), 5.78(d,1H), 6.13(s,1H), 6.89 (s,1H), 7.40(d,1H), 7.48(d,1H) | |
| 119 (S) | 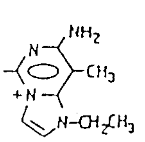 | 662 | 1.27(t,3H), 1.49(d,3H), 1.97(s, 3H), 3.63(ABq,2H), 4.19(q,2H), 4.47(ABq,2H), 4.63(q,1H), 5.18 (d,1H), 5.82(d,1H), 6.89(s,1H), 7.42(d,1H), 7.49(d,1H) | 1780(β-lactam), 1690, 1620, 1580 |
| 119 (R) | | | 1.27(t,3H), 1.49(d,3H), 1.97(s, 3H), 3.63(ABq,2H), 4.19(q,2H), 4.47(ABq,2H), 4.63(q,1H), 5.18 (d,1H), 5.77(d,1H), 6.89(s,1H), 7.42(d,1H), 7.49(d,1H) | |

Table 10(continued)

| Ex . | R | MS(FAB, M+1) | NMR(δ , $D_2O/NaHCO_3$) | IR(KBr, $cm^{-1}$) |
|---|---|---|---|---|
| 120 (S) | 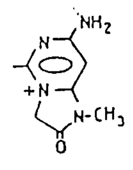 | 650 | 1.48(d,3H), 3.27(s,3H), 3.67 (ABq,2H), 4.45(ABq,2H), 4.61(q, 1H), 4.81(s,2H), 5.12(d,1H), 5.81(d,1H), 6.17(s,1H), 6.97 (s,1H) | 1775(β-lactam), 1740, 1670, 1610 1570 |
| 120 (R) | | | 1.48(d,3H), 3.27(s,3H), 3.67 (ABq,2H), 4.45(ABq,2H), 4.61(q, 1H), 4.81(s,2H), 5.12(d,1H), 5.73(d,1H), 6.17(s,1H), 6.97 (s,1H) | |
| 121 (S) | 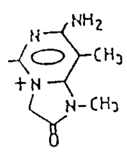 | 664 | 1.50(d,3H), 1.98(s,3H), 3.23 (s,3H), 3.58(ABq,2H), 4.51(ABq, 2H), 4.67(q,1H), 4.84(s,2H), 5.15(d,1H), 5.82(s,1H), 6.99 (s,1H) | 1775(β-lactam), 1730, 1680, 1620 1580 |
| 121 (R) | | | 1.50(d,3H), 1.98(s,3H), 3.23 (s,3H), 3.58(ABq,2H), 4.51(ABq, 2H), 4.67(q,1H), 4.84(s,2H), 5.15(d,1H), 5.77(s,1H), 6.99 (s,1H) | |

Example 122

Synthesis of 7-[(Z)-2-(2-aminothiazol-4-yl)-2-(1-carboxymeth-1-oxyimino)acetamido]-3-(7-amino-1-methyl-[1,3] imidazo[1,2-c]yrimidinium-5-yl)thiomethyl-3-cephem-4-carboxylate

To a solution of 1.0g of paramethoxybenzyl 3-chloromethyl-7-{(Z)-2-(1-t-butoxycarbonylmeth-1-oxyimino)-2-[2-(triphenylmethyl)aminothiazol-4-yl]acetamido}-3-cephem-4-carboxylate dissolved in 5ml of dimethyl sulfoxide was added 150mg of 7-amino-1-methyl[1,3]imidazo[1,2-c] pyrimidine-5-thione prepared in Preparation Example 30; and the resulting mixture was stirred for 2 hours at a room temperature. To the reaction solution was added 3 x 50ml of ethyl ether; and the mixture was stirred vigorously. After ethyl ether was removed from the mixture, 10ml of dichloromethane was added to the residue. To the resulting solution was added dropwise slowly 100ml of ethyl ether; and the mixture was filtered. The resulting solid was washed with 10ml of ethyl ether, dried and dissolved in 5ml of phenol. To the resulting solution was added 0.5ml of concentrated hydrochloric acid; the mixture was stirred at 50 °C for 2 hours and cooled to a room temperature; and 60ml of acetone was added thereto. The resulting mixture was filtered; and the resulting solid was washed with 20ml of acetone and dried to yield 610mg of a light yellow solid. The solid thus obtained was fractionated onto the liquid chromatography($\mu$-Bondapak $C_{18}$ Steel Column, 19mm x 30cm) using 5%-methanol solution as an eluent to yield 310mg of the title compound as a white solid.

MS(FAB, M + 1): 620

NMR(δ, $D_2O$ + $NaHCO_3$): 3.57(ABq, 2H), 3.75(s, 3H), 4.52 (ABq, 2H), 4.81(s, 2H), 5.13(d, 1H), 5.78-(d, 1H), 6.17(s, 1H), 6.92 (s, 1H), 7.42(d, 1H), 7.51(d, 1H)

IR(KBr, $cm^{-1}$): 1760(β-lactam), 1680, 1610, 1570

Examples 123 to 129

The same procedures as described in Example 122 were repeated using paramethoxybenzyl 3-chloromethyl-7-{(Z)-2-(1-t-butoxycarbonylmeth-2-oxyimino)-2-[2-(triphenylmethyl)amino-thiazol-4-yl]-acetamido}-3-cephem-4-carboxylate and thiones prepared in Preparation Examples 31, 36, 38, 39, 41, 42 and 48. The produced compounds are listed in Table 11.

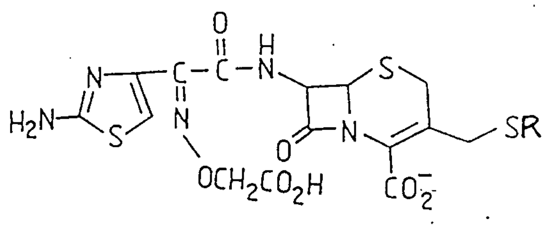

Table 11

| Ex. | R | MS(FAB, M+1) | NMR(δ , $D_2O/NaHCO_3$) | IR(KBr, $cm^{-1}$) |
|---|---|---|---|---|
| 123 | 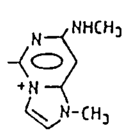 | 634 | 2.99(s,3H), 3.57(ABq,2H), 3.71 (s,3H), 4.50(ABq,2H), 4.81(s,2H), 5.13(d,1H), 5.78(d,1H), 6.13(s, 1H), 7.41(d,1H), 7.48(d,1H) | 1765(β-lactam), 1670, 1610, 1540 |
| 124 | 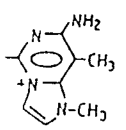 | 634 | 1.97(s,3H), 3.55(ABq,2H), 3.70 (s,3H), 4.47(ABq,2H), 4.79(s,2H), 5.14(d,1H), 5.79(d,1H), 7.40(d, 1H), 7.49(d,1H) | 1770(β-lactam), 1670, 1620, 1530 |
| 125 | 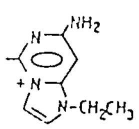 | 634 | 1.27(t,3H), 3.57(ABq,2H), 4.19 (q,2H), 4.53(ABq,2H), 4.80(s,2H), 5.18(d,1H), 5.81(d,1H), 6.17(s, 1H), 6.99(s,1H), 7.42(d,1H), 7.51(d,1H) | 1770(β-lactam), 1680, 1610, 1530 |
| 126 | 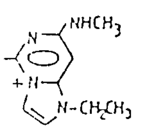 | 648 | 1.27(t,3H), 2.95(s,3H), 3.51 (ABq,2H), 4.13(q,2H), 4.48(ABq, 2H), 4.79(s,2H), 5.15(d,1H), 5.80(d,1H), 6.12(s,1H), 6.93(s, 1H), 7.40(d,1H), 7.48(d,1H) | 1770(β-lactam), 1680, 1620, 1540 |
| 127 | 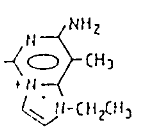 | 648 | 1.27(t,3H), 1.99(s,3H), 3.55 (ABq,2H), 4.20(q,2H), 4.53(ABq, 2H), 4.80(s,2H), 5.17(d,1H), 5.71(d,1H), 6.93(s,1H), 7.41(d, 1H), 7.48(d,1H) | 1765(β-lactam), 1690, 1630, 1540 |

Table 11(continued)

| Ex . | R | MS(FAB, M+1) | NMR($\delta$ , $D_2O/NaHCO_3$) | IR(KBr, $cm^{-1}$) |
|---|---|---|---|---|
| 128 | 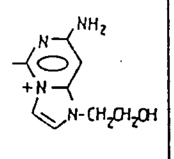 | 650 | 3.58(ABq,2H), 3.77(t,3H), 4.21 (t,3H), 4.51(ABq,2H), 4.80(s,2H), 5.19(d,1H), 5.82(d,1H), 6.23(s, 1H), 6.92(s,1H), 7.43(d,1H), 7.52(d,1H) | 1770($\beta$-lactam), 1680, 1620, 1580 |
| 129 | 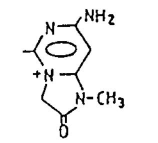 | 636 | 3.19(s,3H), 3.58(ABq,2H), 4.51 (ABq,2H), 4.80(s,2H), 4.81(s,2H), 5.13(d,1H), 5.77(d,1H), 6.15 (s,1H), 6.97(s,1H) | 1780($\beta$-lactam), 1740, 1650, 1620 1570 |

Example 130

Synthesis of 7-{(Z)-2-(2-aminothiazol-4-yl)-2-(methoxyimino)acetamido]-3-(1-methyl[1,3]imidazo[1,2-c] pyrimidinium-5-yl)thiomethyl-3-cephem-4-carboxylate

To a solution of 1.0g of paramethoxybenzyl 3-chloromethyl-7-{(Z)-2-(2-methoxyimino)-2-[2-(triphenylmethyl) aminothiazol-4-yl]acetamido}-3-cephem-4-carboxylate dissolved in 5ml of dimethyl sulfoxide was added 200mg of 1-methyl[1,3]imidazo[1,2-c]pyrimidine-5-thione prepared in Preparation Example 29; and the resulting mixture was stirred for 2 hours at a room temperature. To the reaction solution was added 3 x 50ml of ethyl ehter; and the mixture was stirred vigorously. After ethyl ether was removed from the mixture, 10ml of dichloromethane was added to the residue. To the resulting solution was added dropwise slowly 100ml of ethyl ether; and the mixture was filtered. The resulting solid was washed with 10ml of ethyl ether, dried and dissolved in 3ml of anisole. The reaction solution was cooled to 0°C; and after 4ml of trifluoroacetic acid was added thereto while maintaining at the same temperature, the mixture was warmed to a room temperature and stirred for 80 minutes. The reaction solution was again cooled to -20°C to -30°C; and 50ml of diethyl ether was added dropwise thereto while maintaining at the same temperature. The resulting mixture was filtered; and the resulting solid was washed with 30ml of acetone and dried to yield 670mg of a white solid. The solid thus obtained was fractionated onto the liquid chromatography($\mu$-Bondapak $C_{18}$ Steel Column, 19mm x 30cm) using 20%-methanol solution as an eluent to yield 310mg of the title compound as a white solid.

MS(FAB, M + 1): 561
NMR($\delta$, $D_2O$ + acetone-$d_6$): 3.62(ABq, 2H), 3.95(s, 3H), 4.07(s, 3H), 4.62(ABq, 2H), 5.11(d, 1H), 5.72-(d, 1H), 6.87(s, 1H), 7.80(d, 1H), 7.99(d, 1H), 8.11(d, 1H), 8.32(d, 1H)
IR(KBr, $cm^{-1}$): 1765($\beta$-lactam), 1680, 1620, 1550

Examples 131 to 143

The same procedures as described in Example 130 were repeated using paramethoxybenzyl 3-chloromethyl-7-{(Z)-2-(methoxyimino)-2-[2-(triphenylmethyl)aminothiazol-4-yl] acetamido}-3-cephem-4-carboxylate and thiones prepared in Preparation Examples 30, 31, 34, 36, 37, 41 to 46, 48 and 49. The produced compounds are listed in Table 12.

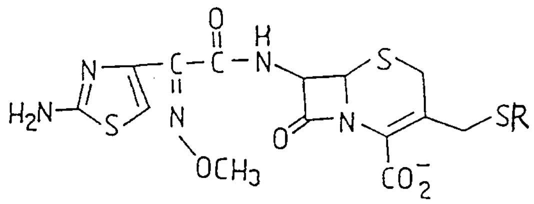

Table 12

| Ex. . | R | MS(FAB, M+1) | NMR($\delta$ , $D_2O$ + acetone-$d_6$) | IR(KBr, cm$^{-1}$) |
|---|---|---|---|---|
| 131 | 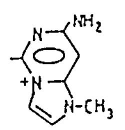 | 576 | 3.55(ABq,2H), 3.71(s,3H), 3.99 (s,3H), 4.49(ABq,2H), 5.08(d,1H), 5.69(d,1H), 6.17(s,1H), 6.83(s, 1H), 7.39(d,1H), 7.48(d,1H) | 1765($\beta$-lactam), 1690, 1580 |
| 132 | 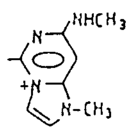 | 590 | 2.98(s,3H), 3.48(ABq,2H), 3.75 (s,3H), 3.92(s,3H), 4.45(ABq,2H), 5.11(d,1H), 5.75(d,1H), 6.15(s, 1H), 6.92(s,1H), 7.42(d,1H), 7.51(d,1H) | 1770($\beta$-lactam), 1690, 1580 |
| 133 | 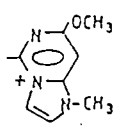 | 591 | 3.61(ABq,2H), 3.71(s,3H), 3.95 (s,3H), 4.11(s,3H), 4.57(ABq,2H), 5.13(d,1H), 5.79(d,1H), 6.27(s, 1H), 6.98(s,1H), 7.50(d,1H), 7.58(d,1H) | 1770($\beta$-lactam), 1680, 1590 |
| 134 | 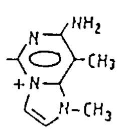 | 590 | 1.98(s,3H), 3.51(ABq,2H), 3.70 (s,3H), 3.99(s,3H), 4.47(ABq,2H), 5.09(d,1H), 5.71(d,1H), 6.93(s, 1H), 7.42(d,1H), 7.49(d,1H) | 1770($\beta$-lactam), 1680, 1565 |
| 135 | 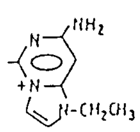 | 590 | 1.30(t,3H), 3.48(ABq,2H), 3.95 (s,3H), 4.11(q,2H), 4.48(ABq,2H), 5.13(d,1H), 5.78(d,1H), 6.11(s, 1H), 6.97(s,1H), 7.41(d,1H), 7.49(d,1H) | 1775($\beta$-lactam), 1690, 1580 |

Table 12(continued)

| Ex. | R | MS(FAB, M+1) | NMR(δ, $D_2O$ +acetone-$d_6$) | IR(KBr, $cm^{-1}$) |
|---|---|---|---|---|
| 136 | 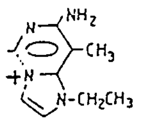 | 604 | 1.29(t,3H), 1.97(s,3H), 3.47 (ABq,2H), 3.99(s,3H), 4.17(q,2H), 4.46(ABq,2H), 5.12(d,1H), 5.76 (d,1H), 6.99(s,1H), 7.41(d,1H), 7.47(d,1H) | 1770(β-lactam), 1690, 1580 |
| 137 | 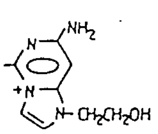 | 606 | 3.61(ABq,2H), 3.78(t,2H), 4.01 (s,3H), 4.28(t,2H), 4.57(ABq,2H), 5.19(d,1H), 5.83(d,1H), 6.23 (s,1H), 6.93(s,1H), 7.43(d,1H) 7.51(d,1H) | 1775(β-lactam), 1680, 1590 |
| 138 | 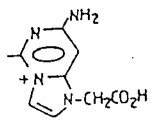 | 620 | 3.59(ABq,2H), 3.98(s,3H), 4.47 (ABq,2H), 4.89(s,2H), 5.17(d,1H), 5.79(d,1H), 6.18(s,1H), 6.98 (s,1H), 7.40(d,1H), 7.47(d,1H) | 1775(β-lactam), 1710, 1680, 1540 |
| 139 | 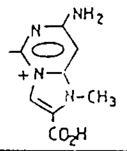 | 620 | 3.68(ABq,2H), 3.81(s,3H), 3.98 (s,3H), 4.53(ABq,2H), 5.13(d,1H), 5.78(d,1H), 6.21(s,1H), 6.97(s, 1H), 7.83(s,1H) | 1770(β-lactam), 1710, 1680, 1550 |
| 140 | 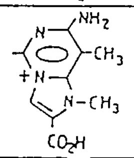 | 634 | 1.98(s,3H), 3.67(ABq,2H), 3.80 (s,3H), 3.98(s,3H), 4.52(ABq,2H), 5.12(d,1H), 5.78(d,1H), 6.98(s, 1H), 7.81(s,1H) | 1765(β-lactam), 1720, 1690, 1570 |
| 141 | 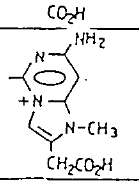 | 634 | 3.52(s,3H), 3.61(ABq,2H), 3.67 (s,2H), 3.97(s,3H), 4.41(ABq,2H), 5.14(d,1H), 5.77(d,1H), 6.19(s, 1H), 6.89(s,1H), 7.41(s,1H) | 1770(β-lactam), 1710, 1680, 1540 |

Table 12(continued)

| Ex. | R | MS(FAB, M+1) | NMR(δ, $D_2O$/$NaHCO_3$) | IR(KBr, $cm^{-1}$) |
|---|---|---|---|---|
| 142 | 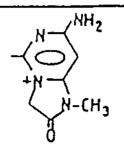 | 592 | 3.20(s,3H), 3.48(ABq,2H), 3.99 (s,3H), 4.47(ABq,2H), 4.82(s,2H), 5.12(d,1H), 5.72(d,1H), 6.13(s, 1H), 6.97(s,1H) | 1765(β-lactam), 1720, 1670, 1550 |
| 143 | 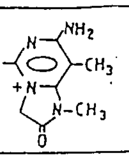 | 606 | 1.98(s,3H), 3.18(s,3H), 3.49 (ABq,2H), 3.97(s,3H), 4.47(ABq, 2H), 4.79(s,2H), 5.14(d,1H), 5.75(d,1H), 6.93(s,1H) | 1770(β-lactam), 1710, 1680, 1570 |

Example 144

Synthesis of 7-[(Z)-2-(2-aminothiazol-4-yl)-2-(ethoxyimino)acetamido]-3-(1-methyl[1,3]imidazo[1,2-c]pyrimidinium-5-yl)thiomethyl-3-cephem-4-carboxylate

To a solution of 1.0g of paramethoxybenzyl 3-chloromethyl-7-{(Z)-2-(ethoxyimino)-2-[2-(triphenylmethyl) aminothiazol-4-yl]acetamido}-3-cephem-4-carboxylate dissolved in 5ml of dimethyl sulfoxide was added 200mg of 1-methyl[1,3]imidazo[1,2-c]pyrimidine-5-thione prepared in Preparation Example 29; and the resulting mixture was stirred for 2 hours at a room temperature. To the reaction solution was added 3 x 50ml of ethyl ehter; and the mixture was stirred vigorously. After ethyl ether was removed from the mixture, 10ml of dichloro-methane was added to the residue. To the resulting solution was added dropwise slowly 100ml of ethyl ether; and the mixture was filtered. The resulting solid was washed with 10ml of ethyl ether, dried and dissolved in 3ml of anisole. The reaction solution was cooled to 0°C; and after 4ml of trifluoroacetic acid was added thereto while maintaining at the some temperature, the mixture was warmed to a room temperature and stirred for 80 minutes. The reaction solution was again cooled to -20°C to -30°C; and 50ml of diethyl ether was added dropwise thereto while maintaining at the same temperature. The resulting mixture was filtered; and the resulting solid was washed with 30ml of acetone and dried to yield 680mg of a white solid. The solid thus obtained was fractionated onto the liquid chromatography($\mu$-Bondapak $C_{18}$ Steel Column, 19mm x 30cm) using 20%-methanol solution as an eluent to yield 270mg of the title compound.

| | |
|---|---|
| MS(FAB, M + 1): | 575 |
| NMR($\delta$, $D_2O$ + acetone-$d_6$): | 1.21(t, 3H), 3.63(ABq, 2H), 3.92(s, 3H), 4.14(q, 2H), 4.57 (ABq, 2H), 5.13-(d, 1H), 5.74 (d, 1H), 6.93(s, 1H), 7.81 (d, 1H), 7.98(d, 1H), 8.11 (d, 1H), 8.33(d, 1H) |
| IR(KBr, $cm^{-1}$): | 1770($\beta$-lactam), 1680, 1610, 1540 |

Examples 145 to 157

The same procedures as described in Example 144 were repeated using paramethoxybenzyl 3-chloromethyl-7-{(Z)-2-(ethoxyimino)-2-[2-(triphenylmethyl)aminothiazol-4-yl] acetamido}-3-cephem-4-carboxylate and thiones prepared in Preparation Examples 30, 32, 33, 38, 39, 41, 42 and 44 to 49. The produced compounds are listed in Table 13.

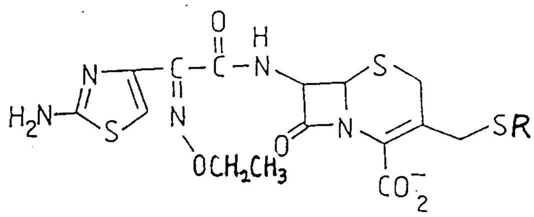

Table 13

| Ex. | R | MS(FAB, M+1) | NMR(δ , $D_2O$ +acetone-$d_6$) | IR(KBr, $cm^{-1}$) |
|---|---|---|---|---|
| 145 | 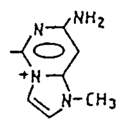 | 590 | 1.28(t,3H), 3.59(ABq,2H), 3.70 (s,3H), 4.15(q,2H), 4.51(ABq,2H), 5.11(d,1H), 5.75(d,1H), 6.17 (s,1H), 6.87(s,1H), 7.41(d,1H), 7.49(d,1H) | 1770(β-lactam), 1680, 1590 |
| 146 | 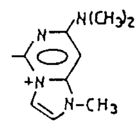 | 604 | 1.27(t,3H), 3.17(s,6H), 3.57 (ABq,2H), 3.71(s,3H), 4.17(q,2H), 4.53(ABq,2H), 5.14(d,1H), 5.76 (d,1H), 6.11(s,1H), 6.91(s,1H), 7.39(d,1H), 7.45(d,1H) | 1770(β-lactam), 1690, 1575 |
| 147 | 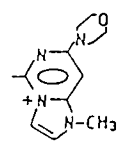 | 660 | 1.25(t,3H), 3.54(ABq,2H), 3.70 (s,3H), 3.68~3.85(m,8H), 4.15 (q,2H), 4.47(ABq,2H), 5.13(d,1H), 5.79(d,1H), 6.29(s,1H), 6.82 (s,1H), 7.43(d,1H), 7.52(d,1H) | 1770(β-lactam), 1680, 1580 |
| 148 | 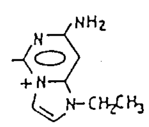 | 604 | 1.27(t,3H), 1.30(t,3H), 3.62 (ABq,2H), 4.17(q,2H), 4.21(q,2H), 4.49(ABq,2H), 5.14(d,1H), 5.73 (d,1H), 6.17(s,1H), 6.92(s,1H), 7.39(d,1H), 7.47(d,1H) | 1775(β-lactam), 1680, 1570 |

Table 13(continued)

| Ex. | R | MS(FAB, M+1) | NMR(δ, $D_2O/NaHCO_3$) | IR(KBr, $cm^{-1}$) |
|---|---|---|---|---|
| 149 | 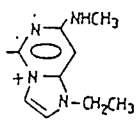 | 618 | 1.27(t,3H), 1.28(t,3H), 2.98(s, 3H), 3.61(ABq,2H), 4.16(q,2H), 4.21(q,2H), 4.52(ABq,2H), 5.11 (d,1H), 5.72(d,1H), 6.12(s,1H), 6.89(s,1H), 7.42(d,1H), 7.51 (d,1H) | 1770(β-lactam), 1680, 1580 |
| 150 | 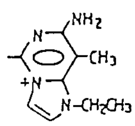 | 618 | 1.29(t,3H), 1.31(t,3H), 1.99 (s,3H), 3.63(ABq,2H), 4.16(q,2H), 4.20(q,2H), 4.51(ABq,2H), 5.17 (d,1H), 5.79(d,1H), 6.93(s,1H), 7.41(d,1H), 7.49(d,1H) | 1770(β-lactam), 1690, 1570 |
| 151 | 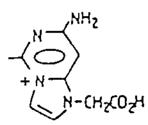 | 634 | 1.28(t,3H), 3.59(ABq,2H), 4.18 (q,2H), 4.52(ABq,2H), 4.93(s,2H), 5.11(d,1H), 5.73(d,1H), 6.12(s, 1H), 6.89(s,1H), 7.42(d,1H), 7.49(d,1H) | 1775(β-lactam), 1705, 1680, 1530 |
| 152 | 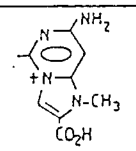 | 634 | 1.27(t,3H), 3.65(ABq,2H), 3.83 (s,3H), 4.11(q,2H), 4.52(ABq,2H), 5.14(d,1H), 5.79(d,1H), 6.23(s, 1H), 6.92(s,1H), 7.87(s,1H) | 1770(β-lactam), 1710, 1680, 1540 |
| 153 | 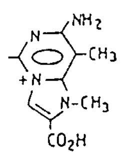 | 648 | 1.27(t,3H), 1.98(s,3H), 3.63 (ABq,2H), 3.82(s,3H), 4.17(q,2H), 4.52(ABq,2H), 5.15(d,1H), 5.76 (d,1H), 6.24(s,1H), 6.93(s,1H), 7.88(s,1H) | 1770(β-lactam), 1710, 1680, 1540 |
| 154 | 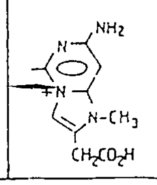 | 648 | 1.28(t,3H), 3.53(s,3H), 3.60 (ABq,2H), 3.68(s,2H), 4.18(q,2H), 4.47(ABq,2H), 5.13(d,1H), 5.75 (d,1H), 6.18(s,1H), 6.92(s,1H), 7.43(s,1H) | 1770(β-lactam), 1720, 1670, 1540 |

Table 13(continued)

| Ex. | R | MS(FAB, M+1) | NMR(δ, $D_2O/NaHCO_3$) | IR(KBr, $cm^{-1}$) |
|---|---|---|---|---|
| 155 | 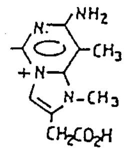 | 662 | 1.27(t,3H), 1.97(s,3H), 3.52(s, 3H), 3.62(ABq,2H), 3.63(s,2H), 4.52(ABq,2H), 5.13(d,1H), 5.72 (d,1H), 6.97(s,1H), 7.40(s,1H), | 1770(β-lactam), 1710, 1680, 1570 |
| 156 | 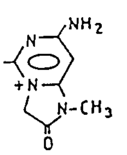 | 606 | 1.29(t,3H), 3.20(s,3H), 3.57 (ABq,2H), 4.18(q,2H), 4.49(ABq, 2H), 4.80(s,2H), 5.13(d,1H), 5.77(d,1H), 6.19(s,1H), 6.91 (s,3H), | 1775(β-lactam), 1715, 1670, 1540 |
| 157 | 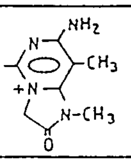 | 620 | 1.28(t,3H), 1.98(s,3H), 3.18(s, 3H), 3.61(ABq,2H), 4.17(q,2H), 4.52(ABq,2H), 4.79(s,2H), 5.11 (d,1H), 5.72(d,1H), 6.89(s,1H) | 1770(β-lactam), 1710, 1680, 1530 |

Example 158

Synthesis of 7-[(Z)-2-(2-aminothiazol-4-yl)-2-(1-carboxycyclopenthoxyimino)acetamido]-3-(7-amino-1-methyl-[1,3] imidazo[1,2-c]pyrimidinium-5-yl)thiomethyl-3-cephem-4-carboxylate

To a solution of 2g of paramethoxybenzyl 3-chloromethyl-7-{(Z)-2-[1-(diphenylmethoxycarbonyl) cyclopentoxyimino)-2-[2-(triphenylmethyl)aminothiazol-4-yl]acetamido}-3-cephem-4-carboxylate dissolved in 10ml of dimethyl sulfoxide was added 400mg of 7-amino-1-methyl [1,3]imidazo[1,2-c]pyrimidine-5-thione prepared in Preparation Example 30; and the resulting mixture was stirred for 2 hours at a room temperature. To the reaction solution was added 50ml of diethyl ether; and the mixture was stirred vigorously. After diethyl ether was removed from the mixture, 5ml of dichloromethane was added to the residue. To the resulting solution was added dropwise slowly 100ml of ethyl ether; and the mixture was filtered. The resulting solid was washed with 50ml of ethyl ether, dried and dissolved in 10ml of phenol. To the resulting solution was added 1.0ml of concentrated hydrochloric acid; and the mixture was stirred at 50°C for 2 hours. The reaction solution was cooled to a room temperature; and 100ml of acetone was added thereto. The resulting mixture was filtered; and the resulting solid was washed with 40ml of acetone and dried to yield 1.4g of an ivory solid. The solid thus obtained was fractionated onto the liquid chromatography (μ-Bondapak $C_{18}$ Steel Column, 19mm x 30cm) using 15%-methanol solution as an eluent to yield 620mg of the title compound.

MS(FAB, M + 1): 680
NMR(δ, $D_2O$ + $NaHCO_3$): 1.61(m, 4H), 2.04(m, 4H), 3.58 (ABq, 2H), 3.68(s, 3H), 4.47 (ABq, 2H), 5.12(d, 1H), 5.74 (d, 1H), 6.27(s, 1H), 6.92(s, 1H), 7.40(s, 1H), 7.52(s, 1H)
IR(KBr, $cm^{-1}$): 1770(β-lactam), 1685, 1595

Example 159

Synthesis of 7-[(Z)-2-(2-aminothiazol-4-yl)-2-((S)-1-carboxyprop-1-oxyimino)acetamido]-3-(7-amino-1-methyl [1,3]imidazo[1,2-c]pyrimidinium-5-yl)thiomethyl-3-cephem-4-carboxylate

To a solution of 2g of the compound prepared in Preparation Example 52 dissolved in 15ml of dimethyl sulfoxide was added 420mg of 7-amino-1-methyl[1,3]imidazo [1,2-c]pyrimidine-5-thione prepared in Preparation Example 30; and the resulting mixture was stirred for 3 hours at a room temperature. The reaction solution was extracted with a mixture of 30ml of tetrahydrofuran and 30ml of ethyl acetate; and the extract was washed with 50ml of saturated sodium chloride solution. The organic phase was separated therefrom,

dried over anhydrous magnesium sulphate, distilled under a reduced pressure to remove the solvent and dried; and the resulting solid was dissolved in 6ml of anisole. The reaction solution was cooled to 0°C to 4°C; and after 10ml of trifluoroacetic acid was added dropwise thereto, the mixture was stirred at a room temperature for 40 minutes and cooled to -20°C to -30°C; and 80ml of diethyl ether was added dropwise thereto. The resulting mixture was filtered; and the resulting solid was washed with 70ml of acetone and dried to yield 1.2g of an ivory powdery solid. The solid thus obtained was fractionated onto the liquid chromatography(μ-Bondapak $C_{18}$ Steel Column, 19mm x 30cm) using 10%-methanol solution as an eluent to yield 255mg of the title compound as a white powdery solid.

| | |
|---|---|
| MS(FAB, M + 1): | 648 |
| NMR(δ, $D_2O$ + $NaHCO_3$): | 0.95(t, 3H), 1.84(q, 2H), 3.61 (ABq, 2H), 3.68(s, 3H), 4.47 (ABq, 2H), 4.49(t, 1H), 5.13(d, 1H), 5.77(d, 1H), 6.96(s, 1H), 6.26 (s, 1H), 7.41(d, 1H), 7.53(d, 1H) |
| IR(KBr, $cm^{-1}$): | 1775(β-lactam), 1670, 1620, 1570 |
| $[\alpha]_D^{20}$ = | -107.55(C = 0.0105, $H_2O$) |

Example 160

Synthesis of 7-[(Z)-2-(2-aminothiazol-4-yl)-2-{(R)-1-carboxyprop-1-oxyimino)acetamido]-3-(7-amino-1-methyl [1,3]imidazo[1,2-c]pyrimidinium-5-yl)thiomethyl-3-cephem-4-carboxylate

To a solution of 1.5g of the compound prepared in Preparation 51 dissolved in 10ml of dimethyl sulfoxide was added 350mg of 7-amino-1-methyl[1,3]imidazo[1,2-c]pyrimidine-5-thione prepared in Preparation 30; and the resulting mixture was stirred for 3 hours at a room temperature. The reaction solution was extracted with a mixture of 20ml of tetrahydrofuran and 20ml of ethyl acetate; and the extract was washed with 100ml of saturated sodium chloride solution. The organic phase was separated therefrom, dried over anhydrous magnesium sulphate, distilled under a reduced pressure to remove the solvent and dried; and the resulting solid was dissolved in 4ml of anisole. The reaction solution was cooled to 0°C to 4°C; after 10ml of trifluoroacetic acid was added dropwise thereto, the mixture was stirred at a room temperature for 40 minutes and cooled to -20°C to - 30°C; and 50ml of diethyl ether was added dropwise thereto. The resulting mixture was filtered; and the resulting solid was washed with 50ml of acetone and dried to yield 1.0g of an ivory powdery solid. The solid thus obtained was fractionated onto the liquid chromatography (μ-Bondapak $C_{18}$ Steel Column, 19mm x 30cm) using 25%-methanol solution as an eluent to yield 470mg of the title compound as a white powdery solid.

| | |
|---|---|
| MS(FAB, M + 1): | 648 |
| NMR(δ, $D_2O$ + $NaHCO_3$): | 0.98(t, 3H), 1.81(q, 2H), 3.61 (ABq, 2H), 3.68(s, 3H), 4.41 (ABq, 2H), 4.46(t, 1H), 5.16(d, 1H), 5.74(s, 1H), 6.27(s, 1H), 6.93 (s, 1H), 7.44(d, 1H), 7.53(d, 1H) |
| IR(KBr, $cm^{-1}$): | 1775(β-lactam), 1670, 1620, 1570 |
| $[\alpha]_D^{20}$ = | -77.05(C = 0.0104, $H_2O$) |

Example 161

Synthesis of 7-[(Z)-2-(2-aminothiazol-4-yl)-2((S)-1-carboxyprop-1-oxyimino)acetamido]-3-(7-amino-1,8-dimethyl[1,3]imidazo[1,2-c]pyrimidinium-5-yl)thiomethyl-3-cephem-4-carboxylate

The same procedures as described in Example 159 were repeated using 7-amino-1,8-dimethyl[1,3]-imidazo[1,2-c] pyrimidine-5-thione prepared in Preparation Example 36 in place of 7-amino-1-methyl[1,3]-imidazo[1,2-c]pyrimidine-5-thione to yield 450mg of the title compound as a white solid.

| | |
|---|---|
| MS(FAB, M + 1): | 662 |
| NMR(δ, $D_2O$ + $NaHCO_3$): | 0.96(t, 3H), 1.82(q, 2H), 2.24 (s, 3H), 3.58(ABq, 2H), 3.63(s, 3H), 4.38(ABq, 2H), 4.43(t, 1H), 5.15 (d, 1H), 5.70(d, 1H), 6.94(s, 1H), 7.41(d, 1H), 7.52(d, 1H) |
| IR(KBr, $cm^{-1}$): | 1770(β-lactam), 1680, 1610, 1580 |
| $[\alpha]_D^{20}$ = | -101.99(C = 0.0097, $H_2O$) |

Example 162

Synthesis of 7-[(Z)-2-(2-aminothiazol-4-yl)-2-((R)-1-carboxyprop-1-oxyimino)acetamido]-3-(7-amino-1,8-dimethyl[1,3]imidazo[1,2-c]pyrimidinium-5-yl)thiomethyl-3-cephem-4-carboxylate

The same procedures as described in Example 160 were repeated using 7-amino-1,8-dimethyl[1,3]-imidazo[1,2-c] pyrimidine-5-thione prepared in Preparation Example 36 in place of 7-amino-1-methyl[1,3]-imidazo[1,2-c]pyrimidine-5-thione to yield 460mg of the title compound as a white solid.

| | |
|---|---|
| MS(FAB, M + 1): | 662 |
| NMR($\delta$, $D_2O$ + $NaHCO_3$): | 0.98(t, 3H), 1.84(q, 2H), 2.23 (s, 3H), 3.56(ABq, 2H), 3.61(s, 3H), 4.42(ABq, 2H), 4.45(t, 1H), 5.16 (d, 1H), 5.69(d, 1H), 6.92(s, 1H), 7.42(d, 1H), 7.51(d, 1H) |
| IR(KBr, $cm^{-1}$): | 1770($\beta$-lactam), 1680, 1610, 1580 |
| $[\alpha]_D^{20}$ = | -70.34(C = 0.0098, $H_2O$) |

Example 163

Synthesis of 7-[(Z)-2-(2-aminothiazol-4-yl)-2-(2-carboxybut-2-oxyimino)acetamido]-3-(7-amino-1-methyl[1,3] imidazo[1,2-c] pyrimidinium-5-yl)thiomethyl-3-cephem-4-carboxylate(a) and (b)

To a solution of 2g of the compound prepared in Preparation Example 53 dissolved in 15ml of dimethyl sulfoxide was added 350mg of 7-amino-1-methyl[1,3]imidazo [1,2-c]pyrimidine-5-thione prepared in Preparation Example 30; and the resulting mixture was stirred for 2 hours at a room temperature. To the reaction solution was added 50ml of diethyl ether; and the mixture was stirred vigorously. After the organic solvent layer was removed from the mixture, 20ml of dichloromethane was added to the residue. To the resulting solution was added dropwise slowly 100ml of diethyl ether; the mixture was filtered; and the resulting solid was washed with 50ml of diethyl ether, dried and dissolved in 4ml of anisole. The reaction solution was cooled to 0°C to 4°C; and after 8ml of trifluoroacetic acid was added dropwise thereto, the mixture was stirred at a room temperature for 50 minutes and cooled to -20°C to -30°C; and 50ml of diethyl ether was added dropwise thereto. The resulting mixture was filtered; and the resulting solid was washed with 100ml of acetone and dried to yield 1.1g of an ivory powdery solid which is a mixture of diastereo isomers. The solid thus obtained was fractionated onto the liquid chromatography($\mu$-Bondapak $C_{18}$ Steel Column, 19mm x 30cm) using 20%-methanol solution containing 0.5% acetic acid as an eluent to yield 200mg and 205mg of the title compound(a) of short retention time and (b) of long retention time, respectively, as white powdery solids.

| | |
|---|---|
| MS(FAB, M + 1): | 662 |
| NMR($\delta$, $D_2O$ + $NaHCO_3$): | |
| (a): | 0.87(t, 3H), 1.44(s, 3H), 1.84(q, 2H), 3.58 (ABq, 2H), 3.63(s, 3H), 4.43(ABq, 2H), 5.12 (d, 1H), 5.68(d, 1H), 6.20(s, 1H), 6.91(s, 1H), 7.42(d, 1H), 7.53(d, 1H) |
| (b): | 0.86(t, 3H), 1.46(s, 3H), 1.85(q, 2H), 3.59 (ABq, 2H), 3.64(s, 3H), 4.45(ABq, 2H), 5.13 (d, 1H), 5.72(d, 1H), 6.23(s, 1H), 6.90(s, 1H), 7.41(d, 1H), 7.51(d, 1H) |
| IR(KBr, $cm^{-1}$): | 1770($\beta$-lactam), 1670, 1640, 1580 |
| $[\alpha]_D^{20}$: | |
| (a): | -109.83(C = 0.0102, $H_2O$) |
| (b): | -92.2(C = 0.0104, $H_2O$) |

Example 164

Synthesis of 7-[(Z)-2-(2-aminothiazol-4-yl)-2-(2-carboxybut-2-oxyimino)acetamido]-3-(7-amino-1,8-dimethyl [1,3]imidazo[1,2-c]pyrimidinium-5-yl)thiomethyl-3-cephem-4-carboxylate(a) and (b)

The same procedures as described in Example 163 were repeated using 7-amino-1,8-dimethyl[1,3]-imidazo[1,2-c] pyrimidine-5-thione prepared in Preparation Example 36 in place of 7-amino-1-methyl[1,3]-imidazo[1,2-c]pyrimidine-5-thione to yield 190mg and 180mg of the title compound(a) and (b), respectively, as white solids.

| | |
|---|---|
| MS(FAB, M + 1): | 676 |

NMR(δ, $D_2O$ + $NaHCO_3$):

(a): 0.90(t, 3H), 1.42(s, 3H), 1.82(q, 2H), 2.23 (s, 3H), 3.59(ABq, 2H), 3.65(s, 3H), 4.45 (ABq, 2H), 5.12(d, 1H), 5.69(d, 1H), 6.17(s, 1H), 6.89(s, 1H), 7.41-(d, 1H), 7.52(d, 1H)

(b): 0.86(t, 3H), 1.46(s, 3H), 1.85(q, 2H), 2.34 (s, 3H), 3.59(ABq, 2H), 3.64(s, 3H), 4.45 (ABq, 2H), 5.13(d, 1H), 5.72(d, 1H), 6.23(s, 1H), 6.90(s, 1H), 7.41-(d, 1H), 7.51(d, 1H)

IR(KBr, $cm^{-1}$): 1770(β-lactam), 1680, 1620, 1570

$[\alpha]_D^{20}$:

(a): -112.82(C = 0.0097, $H_2O$)

(b): -94.37(C = 0.0099, $H_2O$)

In order to illustrate the surprisingly superior antibacterial effectiveness of the compounds of the present invention, the minimal inhibitory concentrations (MIC) of the compounds synthesized in the above Examples against standard strains were determined and compared with Ceftazidime which was used as the control compound.

These MIC values were taken by employing a two-fold dilution method: that is, two-fold serial dilutions of each of the test compounds were made and dispersed in a Müller-Hinton agar medium; 2μl of the standard test strain which had the $10^7$ CFU(Colony Forming Unit) per ml was inoculated on the medium; and these were incubated at 37 °C for 20 hours. The results of the MIC tests are shown in Table 14.

Table 14. MIC values against the standard strains(μg/mℓ)

| Strains | | Compound Prepared in Exp. 1 | Compound Prepared in Exp. 4 | Compound Prepared in Exp. 9 | Compound Prepared in Exp. 11 | Compound Prepared in Exp. 22(S) | Compound Prepared in Exp. 23(R) |
|---|---|---|---|---|---|---|---|
| Bacillus cereus | ATCC 11778 | 128 | 16 | 32 | 64 | 128 | 16 |
| Bacillus megaterium | ATCC 9885 | 0.25 | 0.13 | 0.13 | 0.25 | 0.25 | 0.13 |
| Micrococcus luteus | ATCC 9341 | 0.13 | 0.13 | 0.13 | 0.13 | 0.13 | ≤0.008 |
| Staphylococcus aureus | ATCC 6538p | 4 | 2 | 4 | 2 | 4 | 1 |
| Staphylococcus aureus | ATCC 10537 | 4 | 1 | 2 | 2 | 2 | 2 |
| Staphylococcus epidermidis | ATCC 12228 | 1 | 0.5 | 1 | 1 | 1 | 0.5 |
| Streptococcus faecalis | ATCC 29212 | >128 | >128 | >128 | >128 | >128 | 32 |
| Acinetobacter calcoaceticus | ATCC 15473 | 4 | 2 | 8 | 4 | 4 | 2 |
| Citrobacter freundii | ATCC 8090 | 0.13 | 0.031 | 0.063 | 0.063 | 0.063 | 0.25 |
| Enterobacter aerogenes | ATCC 29751 | 1 | 0.25 | 0.5 | 0.5 | 0.5 | 0.25 |
| Enterobacter cloacae | ATCC 27508 | 0.016 | ≤0.008 | 0.016 | 0.016 | 0.016 | 0.016 |
| Escherichia coli | ATCC 10536 | 0.063 | 0.063 | 0.13 | 0.063 | 0.031 | 0.063 |
| Escherichia coli | ATCC 25922 | 0.13 | 0.063 | 0.063 | 0.063 | 0.13 | 0.13 |
| Klebsiella pneumoniae | ATCC 10031 | 0.063 | 0.016 | 0.063 | 0.13 | 0.031 | 0.063 |
| Morganella morganii | ATCC 8076h | ≤0.008 | ≤0.008 | ≤0.008 | ≤0.008 | ≤0.008 | ≤0.008 |
| Proteus mirabilis | ATCC 25933 | 0.13 | 0.13 | 0.063 | 0.063 | 0.25 | 0.063 |
| Proteus vulgaris | ATCC 6059 | 0.063 | 0.063 | 0.063 | 0.063 | 0.31 | 0.13 |
| Providencia rettgeri | ATCC 9250 | ≤0.008 | ≤0.008 | ≤0.008 | ≤0.008 | ≤0.008 | ≤0.008 |
| Salmonella typhimurium | ATCC 14028 | 0.25 | 0.13 | 0.13 | 0.25 | 0.25 | 0.25 |
| Serratia marcescens | ATCC 27117 | 0.25 | 0.13 | 0.25 | 0.13 | 0.13 | 0.13 |
| Shigella flexneri | ATCC 11836 | 0.031 | 0.016 | 0.031 | 0.031 | 0.13 | 0.031 |
| Shigella sonnei | ATCC 11060 | 0.13 | 0.063 | 0.13 | 0.13 | 0.063 | 0.063 |
| Pseudomonas aeruginosa | ATCC 25619 | 1 | 0.5 | 1 | 1 | 1 | 1 |
| Pseudomonas aeruginosa | ATCC 27853 | 1 | 1 | 2 | 2 | 2 | 2 |
| Pseudomonas aeruginosa | ATCC 10145 | 2 | 1 | 2 | 2 | 2 | 2 |

| Strains | | Compound Prepared in Exp. 40 | Compound Prepared in Exp. 46 | Compound Prepared in Exp. 47 | Compound Prepared in Exp. 51 | Compound Prepared in Exp. 72 |
|---|---|---|---|---|---|---|
| Bacillus cereus | ATCC 11778 | 16 | 8 | 16 | 16 | 16 |
| Bacillus megaterium | ATCC 9885 | 0.031 | 0.063 | 0.25 | 0.25 | 0.063 |
| Micrococcus luteus | ATCC 9341 | ≤0.008 | 0.016 | 0.13 | 0.016 | 0.016 |
| Staphylococcus aureus | ATCC 6538p | 0.13 | 0.13 | 1 | 0.25 | 0.25 |
| Staphylococcus aureus | ATCC 10537 | 0.13 | 0.063 | 1 | 0.25 | 0.13 |
| Staphylococcus epidermidis | ATCC 12228 | 0.031 | 0.063 | 0.25 | 0.13 | 0.25 |
| Streptococcus faecalis | ATCC 29212 | 8 | 16 | 64 | 16 | 8 |
| Acinetobacter calcoaceticus | ATCC 15473 | 2 | 2 | 8 | 4 | 1 |
| Citrobacter freundii | ATCC 8090 | 0.13 | 0.031 | 0.25 | 0.13 | 0.063 |
| Enterobacter aerogenes | ATCC 29751 | 0.25 | 0.13 | 2 | 0.25 | 0.25 |
| Enterobacter cloacae | ATCC 27508 | ≤0.008 | ≤0.008 | ≤0.008 | ≤0.008 | ≤0.008 |
| Escherichia coli | ATCC 10536 | 0.13 | 0.031 | 0.031 | 0.063 | 0.063 |
| Escherichia coli | ATCC 25922 | 0.063 | 0.031 | 0.063 | 0.13 | 0.063 |
| Klebsiella pneumoniae | ATCC 10031 | ≤0.008 | ≤0.008 | 0.031 | ≤0.008 | 0.016 |
| Morganella morganii | ATCC 8076h | ≤0.008 | ≤0.008 | ≤0.008 | ≤0.008 | ≤0.008 |
| Proteus mirabilis | ATCC 25933 | 0.13 | 0.13 | 0.13 | 0.13 | 0.25 |
| Proteus vulgaris | ATCC 6059 | 0.13 | 0.063 | 0.13 | 0.13 | 0.5 |
| Providencia rettgeri | ATCC 9250 | ≤0.008 | ≤0.008 | ≤0.008 | ≤0.008 | ≤0.008 |
| Salmonella typhimurium | ATCC 14028 | 0.25 | 0.063 | 0.13 | 0.25 | 0.25 |
| Serratia marcescens | ATCC 27117 | 0.13 | 0.13 | 0.25 | 0.13 | 0.25 |
| Shigella flexneri | ATCC 11836 | 0.031 | 0.016 | 0.031 | 0.031 | 0.031 |
| Shigella sonnei | ATCC 11060 | 0.031 | 0.063 | 0.013 | 0.13 | 0.063 |
| Pseudomonas aeruginosa | ATCC 25619 | 1 | 2 | 2 | 1 | 1 |
| Pseudomonas aeruginosa | ATCC 27853 | 1 | 2 | 2 | 4 | 4 |
| Pseudomonas aeruginosa | ATCC 10145 | 2 | 4 | 4 | 4 | 4 |

| Strains | | Compound Prepared in Exp. 84 | Compound Prepared in Exp. 86 | Compound Prepared in Exp. 88 | Compound Prepared in Exp. 96(b) |
|---|---|---|---|---|---|
| Bacillus cereus | ATCC 11778 | 32 | 16 | 8 | 16 |
| Bacillus megaterium | ATCC 9885 | 0.25 | 0.031 | 0.031 | 0.25 |
| Micrococcus luteus | ATCC 9341 | 0.016 | ≤0.008 | ≤0.008 | 0.016 |
| Staphylococcus aureus | ATCC 6538p | 0.25 | 0.13 | 0.13 | 1 |
| Staphylococcus aureus | ATCC 10537 | 0.25 | 0.13 | 0.13 | 1 |
| Staphylococcus epidermidis | ATCC 12228 | 0.5 | 0.25 | 0.031 | 0.5 |
| Streptococcus faecalis | ATCC 29212 | 32 | 16 | 8 | 16 |
| Acinetobacter calcoaceticus | ATCC 15473 | 2 | 2 | 1 | 4 |
| Citrobacter freundii | ATCC 8090 | 0.063 | 0.13 | 0.13 | 0.13 |
| Enterobacter aerogenes | ATCC 29751 | 0.25 | 0.25 | 0.13 | 0.5 |
| Enterobacter cloacae | ATCC 27508 | ≤0.008 | ≤0.008 | ≤0.008 | 0.063 |
| Escherichia coli | ATCC 10536 | 0.063 | 0.13 | 0.13 | 0.13 |
| Escherichia coli | ATCC 25922 | 0.063 | 0.063 | 0.063 | 0.13 |
| Klebsiella pneumoniae | ATCC 10031 | 0.063 | ≤0.008 | ≤0.008 | ≤0.008 |
| Morganella morganii | ATCC 8076h | ≤0.008 | ≤0.008 | ≤0.008 | ≤0.008 |
| Proteus mirabilis | ATCC 25933 | 0.063 | 0.13 | 0.13 | 0.13 |
| Proteus vulgaris | ATCC 6059 | 0.063 | 0.13 | 0.063 | 0.063 |
| Providencia rettgeri | ATCC 9250 | ≤0.008 | ≤0.008 | ≤0.008 | ≤0.008 |
| Salmonella typhimurium | ATCC 14028 | 0.13 | 0.25 | 0.13 | 0.25 |
| Serratia marcescens | ATCC 27117 | 0.13 | 0.25 | 0.25 | 0.13 |
| Shigella flexneri | ATCC 11836 | 0.063 | 0.031 | 0.031 | 0.031 |
| Shigella sonnei | ATCC 11060 | 0.13 | 0.063 | 0.063 | 0.13 |
| Pseudomonas aeruginosa | ATCC 25619 | 1 | 1 | 0.5 | 0.5 |
| Pseudomonas aeruginosa | ATCC 27853 | 1 | 1 | 1 | 1 |
| Pseudomonas aeruginosa | ATCC 10145 | 2 | 2 | 1 | 2 |

| | | Compound Prepared in Exp. 101 | Compound Prepared in Exp. 102 | Compound Prepared in Exp. 107 | Compound Prepared in Exp. 116(S) | Compound Prepared in Exp. 116(R) | Compound Prepared in Exp. 117 |
|---|---|---|---|---|---|---|---|
| Bacillus cereus | ATCC 11778 | 128 | >128 | 128 | 128 | 32 | 128 |
| Bacillus megaterium | ATCC 9885 | 0.25 | 0.25 | 0.25 | 0.13 | 0.031 | 0.25 |
| Micrococcus luteus | ATCC 9341 | 0.25 | 0.5 | 0.5 | 0.25 | 0.063 | 0.25 |
| Staphylococcus aureus | ATCC 6538p | 4 | 8 | 4 | 2 | 0.13 | 2 |
| Staphylococcus aureus | ATCC 10537 | 2 | 4 | 2 | 1 | 2 | 1 |
| Staphylococcus epidermidis | ATCC 12228 | 2 | 4 | 2 | 1 | 1 | 1 |
| Streptococcus faecalis | ATCC 29212 | >128 | >128 | >128 | >128 | 16 | >128 |
| Acinetobacter calcoaceticus | ATCC 15473 | 8 | 8 | 8 | 4 | 2 | 4 |
| Citrobacter freundii | ATCC 8090 | 0.063 | 0.13 | 0.063 | 0.031 | 0.031 | 0.031 |
| Enterobacter aerogenes | ATCC 29751 | 1 | 2 | 2 | 1 | 0.5 | 2 |
| Enterobacter cloacae | ATCC 27508 | ≤0.008 | 0.016 | 0.008 | ≤0.008 | 0.016 | ≤0.008 |
| Escherichia coli | ATCC 10536 | 0.063 | 0.13 | 0.063 | 0.016 | 0.063 | 0.031 |
| Escherichia coli | ATCC 25922 | 0.063 | 0.13 | 0.063 | 0.016 | 0.063 | 0.031 |
| Klebsiella pneumoniae | ATCC 10031 | 0.063 | 0.13 | 0.063 | 0.016 | ≤0.008 | 0.031 |
| Morganella morganii | ATCC 8076h | ≤0.008 | ≤0.008 | ≤0.008 | ≤0.008 | ≤0.008 | ≤0.008 |
| Proteus mirabilis | ATCC 25933 | 0.016 | 0.063 | 0.031 | 0.016 | 0.016 | 0.031 |
| Proteus vulgaris | ATCC 6059 | 0.031 | 0.031 | 0.031 | 0.016 | 0.031 | 0.031 |
| Providencia rettgeri | ATCC 9250 | ≤0.008 | ≤0.008 | ≤0.008 | ≤0.008 | 0.063 | ≤0.008 |
| Salmonella typhimurium | ATCC 14028 | 0.13 | 0.25 | 0.25 | 0.063 | 0.25 | 0.13 |
| Serratia marcescens | ATCC 27117 | 0.13 | 0.25 | 0.13 | 0.063 | 0.31 | 0.063 |
| Shigella flexneri | ATCC 11836 | 0.016 | 0.031 | 0.031 | 0.016 | 0.063 | 0.031 |
| Shigella sonnei | ATCC 11060 | 0.13 | 0.13 | 0.13 | 0.063 | 0.031 | 0.063 |
| Pseudomonas aeruginosa | ATCC 25619 | 1 | 1 | 1 | 1 | 2 | 1 |
| Pseudomonas aeruginosa | ATCC 27853 | 1 | 2 | 2 | 1 | 2 | 2 |
| Pseudomonas aeruginosa | ATCC 10145 | 2 | 4 | 2 | 2 | 4 | 2 |

| | | Compound Prepared in Exp. 122 | Compound Prepared in Exp. 131 | Compound Prepared in Exp. 134 | Compound Prepared in Exp. 145 | Compound Prepared in Exp. 158 | Compound Prepared in Exp. 160 | Compound Prepared in Exp. 163(b) | Cefta-zidime |
|---|---|---|---|---|---|---|---|---|---|
| Bacillus cereus | ATCC 11778 | 128 | 16 | 32 | 16 | 16 | 16 | 32 | 128 |
| Bacillus megaterium | ATCC 9885 | 0.5 | 0.031 | 0.031 | 0.031 | 0.031 | 0.063 | 0.25 | 0.25 |
| Micrococcus luteus | ATCC 9341 | 1 | ≤0.008 | ≤0.008 | ≤0.008 | ≤0.008 | ≤0.008 | 0.063 | 1 |
| Staphylococcus aureus | ATCC 6538p | 16 | 0.13 | 0.13 | 0.063 | 0.13 | 0.063 | 0.13 | 16 |
| Staphylococcus aureus | ATCC 10537 | 8 | 0.063 | 0.13 | 0.063 | 0.5 | 1 | 4 | 8 |
| Staphylococcus epidermidis | ATCC 12228 | 8 | 0.063 | 0.13 | 0.063 | 0.25 | 0.25 | 1 | 8 |
| Streptococcus faecalis | ATCC 29212 | >128 | 8 | 8 | 8 | 8 | 8 | 16 | >128 |
| Acinetobacter calcoaceticus | ATCC 15473 | 8 | 1 | 2 | 4 | 1 | 1 | 4 | 4 |
| Citrobacter freundii | ATCC 8090 | 0.13 | 0.016 | 0.031 | 0.063 | 0.063 | 0.063 | 0.031 | 0.25 |
| Enterobacter aerogenes | ATCC 29751 | 4 | 0.031 | 0.031 | 0.063 | 0.25 | 0.25 | 0.5 | 8 |
| Enterobacter cloacae | ATCC 27508 | ≤0.008 | ≤0.008 | ≤0.008 | ≤0.008 | ≤0.008 | ≤0.008 | 0.063 | 0.063 |
| Escherichia coli | ATCC 10536 | 0.13 | 0.031 | 0.031 | 0.063 | 0.063 | 0.063 | 0.016 | 0.13 |
| Escherichia coli | ATCC 25922 | 0.13 | 0.031 | 0.063 | 0.063 | 0.031 | 0.063 | 0.016 | 0.13 |
| Klebsiella pneumoniae | ATCC 10031 | 0.13 | ≤0.008 | ≤0.008 | ≤0.008 | ≤0.008 | ≤0.008 | ≤0.008 | 0.13 |
| Morganella morganii | ATCC 8076h | 0.016 | ≤0.008 | ≤0.008 | ≤0.008 | ≤0.008 | ≤0.008 | ≤0.008 | 0.016 |
| Proteus mirabilis | ATCC 25933 | 0.13 | 0.016 | 0.061 | 0.016 | 0.063 | 0.063 | 0.016 | 0.063 |
| Proteus vulgaris | ATCC 6059 | 0.13 | 0.016 | 0.031 | 0.016 | 0.063 | 0.031 | 0.031 | 0.063 |
| Providencia rettgeri | ATCC 9250 | ≤0.008 | ≤0.008 | ≤0.008 | ≤0.008 | ≤0.008 | ≤0.008 | 0.016 | ≤0.008 |
| Salmonella typhimurium | ATCC 14028 | 0.25 | 0.031 | 0.031 | 0.031 | 0.25 | 0.031 | 0.031 | 0.25 |
| Serratia marcescens | ATCC 27117 | 0.13 | 0.031 | 0.031 | 0.031 | 0.031 | 0.063 | 0.063 | 0.13 |
| Shigella flexneri | ATCC 11836 | 0.063 | 0.016 | 0.016 | 0.031 | 0.016 | 0.063 | 0.016 | 0.063 |
| Shigella sonnei | ATCC 11060 | 0.13 | 0.031 | 0.031 | 0.031 | 0.031 | 0.031 | 0.016 | 0.13 |
| Pseudomonas aeruginosa | ATCC 25619 | 2 | 0.5 | 1 | 0.5 | 1 | 1 | 1 | 1 |
| Pseudomonas aeruginosa | ATCC 27853 | 4 | 2 | 4 | 4 | 2 | 1 | 2 | 1 |
| Pseudomonas aeruginosa | ATCC 10145 | 4 | 4 | 4 | 8 | 2 | 2 | 2 | 2 |

As can be seen from Table 14, the cephalosporin compounds of the present invention possess potent and broad antibacterial activities against Gram-positive and Gram-negative bacteria as compared with the known cephalosporin antibiotics, ceftazidime.

While the invention has been described in connection with the above specific embodiments, it should be recognized that various modifications and changes as may be apparent to those skilled in the art to which the invention pertains may be made and also fall within the scope of the invention as defined by the claims that follow.

## Claims

**1.** A cephalosporin compound, including pharmaceutically acceptable non-toxic salts, physiologically hydrolyzable esters, hydrates and solvates thereof, of the formula(I) and its isomers:

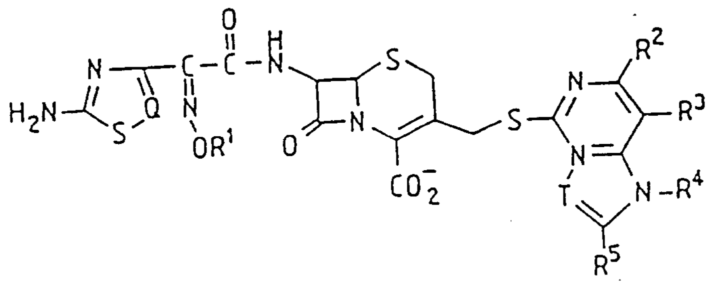

(I)

wherein:

| | |
|---|---|
| $R^1$ is | a $C_{1-4}$ alkyl, $C_{3-4}$ alkenyl, $C_{3-4}$ alkynyl or $-C(R^a)(R^b)-COOH$ wherein $R^a$ and $R^b$ are, independently of each other, a hydrogen or a $C_{1-4}$ alkyl group, respectively, or jointly form a $C_{3-7}$ cycloalkyl group together with the carbon atoms to which they are attached; |
| $R^2$ is | a hydrogen or an amino which may be substituted or unsubstituted, alkoxy or heterocyclic group which is linked with a nitrogen atom; |
| $R^3$ is | a hydrogen or a $C_{1-4}$ alkyl, amino, aminomethyl, hydroxy, hydroxymethyl, carboxy or carboxymethyl group; |
| $R^4$ is | a hydrogen or a $C_{1-4}$ alkyl, amino, hydroxy, hydroxyalkyl, acetamide, carboxyalkyl or sulfonylethyl group; |
| $R^5$ is | a hydrogen or a $C_{1-4}$ alkyl, amino, carboxy, carboxyalkyl or hydroxy group; and |
| Q and T are | independently CH or N, provided that $R^2$ is an amino group when T is N. |

**2.** The cephalosporin compound of claim 1 wherein $R^1$ is a methyl, ethyl, allyl, propargyl or $-C(R^a)(R^b)-COOH$ group wherein $R^a$ and $R^b$ are, independently of each other, a hydrogen or a methyl or ethyl group, respectively, or jointly form a cyclopentyl group together with the carbon atoms to which they are attached; $R^2$ is a hydrogen or an amino group; $R^3$ is a hydrogen or a methyl or amino group; $R^4$ is a methyl, ethyl, amino or carboxymethyl group; and $R^5$ is a hydrogen or a methyl, amino or hydroxy group.

**3.** The cephalosporin compound of claim 1 which is selected from the group consisting of:
7-[(Z)-2-(2-aminothiazol-4-yl)-2-(2-carboxyprop-2-oxyimino) acetamido]-3-(7-amino-1-methyl[1,2,4]-triazolo [1,5-c]pyrimidinium-5-yl)thiomethyl-3-cephem-4-carboxylate;
7-[(Z)-2-(2-aminothiazol-4-yl)-2-(carboxyprop-2-oxyimino)acetamido]-3-(2,7-diamino-1-methyl[1,2,4] triazolo[1,5-c]pyrimidinium-5-yl)thiomethyl-3-cephem-4-carboxylate;
7-[(Z)-2-(2-aminothiazol-4-yl)-2-(2-carboxyprop-2-oxyimino)acetamido]-3-(7-amino-1,8-dimethyl[1,2,4] triazolo[1,5-c]pyrimidinium-5-yl)thiomethyl-3-cephem-4-carboxylate;
7-[(Z)-2-(2-aminothiazol-4-yl)-2-(2-carboxyprop-2-oxyimino)acetamido]-3-(2,7-diamino-1,8-dimethyl-[1,2,4] triazolo[1,5-c]pyrimidinium-5-yl)thiomethyl-3-cephem-4-carboxylate;
7-[(Z)-2-(2-aminothiazol-4-yl)-2-(2-carboxyprop-2-oxyimino)acetamido]-3-(7-amino-1,8-dimethyl[1,2,4] triazol-3-one[1,5-c]pyrimidinium-5-yl)thiomethyl-3-cephem-4-carboxylate;
7-[(Z)-2-(2-aminothiazol-4-yl)-2-((S)-1-carboxyeth-1-oxyimino)acetamido]-3-(7-amino-1-methyl[1,2,4]-triazolo [1,5-c]pyrimidinium-5-yl)thiomethyl-3-cephem-4-carboxylate;
7-[(Z)-2-(2-aminothiazol-4-yl)-2-(ethoxyimino)acetamido]-3-(7-amino-1-methyl[1,2,4]triazolo[1,5-c]-pyrimidinium-5-yl)thiomethyl-3-cephem-4-carboxylate;
7-[(Z)-2-(2-aminothiazol-4-yl)-2-(ethoxyimino)acetamido]-3-(2,7-diamino-1-methyl[1,2,4]triazolo[1,5-c] pyrimidinium-5-yl)thiomethyl-3-cephem-4-carboxylate;
7-[(Z)-2-(2-aminothiazol-4-yl)-2-(methoxyimino) acetamido]-3-(7-amino-1-methyl[1,2,4]triazolo[1,5-c] pyrimidinium-5-yl)thiomethyl-3-cephem-4-carboxylate;
7-[(Z)-2-(2-aminothiazol-4-yl)-2-(methoxyimino) acetamido]-3-(7-amino-1-carboxymethyl[1,2,4]triazolo-[1,5-c]pyrimidinium-5-yl)thiomethyl-3-cephem-4-carboxylate;
7-[(Z)-2-(2-aminothiazol-4-yl)-2-(methoxyimino) acetamido]-3-(2,7-diamino-1-methyl[1,2,4]triazolo[1,5-c]-pyrimidinium-5-yl)thiomethyl-3-cephem-4-carboxylate;
7-[(Z)-2-(2-aminothiazol-4-yl)-2-(methoxyimino) acetamido]-3-(2,7-diamino-1-carboxymethyl[1,2,4]triazolo [1,5-c]pyrimidinium-5-yl)thiomethyl-3-cephem-4-carboxylate;
7-[(Z)-2-(5-amino-1,2,4-thiadiazol-3-yl)-2-(ethoxyimino) acetamido]-3-(7-amino-1-methyl[1,2,4]triazolo-

[1,5-c] pyrimidinium-5-yl)thiomethyl-3-cephem-4-carboxylate;
7-[(Z)-2-(2-aminothiazol-4-yl)-2-((R)-1-carboxyeth-1-oxyimino)acetamido]-3-(7-amino-1-methyl[1,2,4]-triazolo [1,5-c]pyrimidinium-5-yl)thiomethyl-3-cephem-4-carboxylate;
7-[(Z)-2-(2-aminothiazol-4-yl)-2-(1-carboxycyclopentoxyimino)acetamido]-3-(7-amino-1-methyl[1,2,4] triazolo[1,5-c]pyrimidinium-5-yl)thiomethyl-3-cephem-4-carboxylate;
7-[(Z)-2-(2-aminothiazol-4-yl)-2-(1-carboxycyclopentoxyimino)acetamido]-3-(2,7-diamino-1-methyl[1,2,4] triazolo[1,5-c]pyrimidinium-5-yl)thiomethyl-3-cephem-4-carboxylate;
7-[(Z)-2-(2-aminothiazol-4-yl)-2-((R)-1-carboxyprop-1-oxyimino)acetamido]-3-(7-amino-1-methyl[1,2,4]-triazolo [1,5-c]pyrimidinium-5-yl)thiomethyl-3-cephem-4-carboxylate;
7-[(Z)-2-(2-aminothiazol-4-yl)-2-((R)-1-carboxyprop-1-oxyimino)acetamido]-3-(2,7-diamino-1-methyl-[1,2,4] triazolo[1,5-c]pyrimidinium-5-yl)thiomethyl-3-cephem-4-carboxylate;
7-[(Z)-2-(2-aminothiazol-4-yl)-2-((R)-1-carboxyprop-1-oxyimino)acetamido]-3-(7-amino-1,8-dimethyl-[1,2,4] triazolo[1,5-c]pyrimidinium-5-yl)thiomethyl-3-cephem-4-carboxylate;
7-[(Z)-2-(2-aminothiazol-4-yl)-2-(2-carboxybut-2-oxyimino)acetamido]-3-(7-amino-1-methyl[1,2,4]triazolo [1,5-c]pyrimidinium-5-yl)thiomethyl-3-cephem-4-carboxylate;
7-[(Z)-2-(2-aminothiazol-4-yl)-2-(2-carboxybut-2-oxyimino)acetamido]-3-(2,7-diamino-1-methyl[1,2,4] triazolo[1,5-c]pyrimidinium-5-yl)thiomethyl-3-cephem-4-carboxylate;
7-[(Z)-2-(2-aminothiazol-4-yl)-2-(2-carboxybut-2-oxyimino)acetamido]-3-(7-amino-1,8-dimethyl[1,2,4] triazolo[1,5-c]pyrimidinium-5-yl)thiomethyl-3-cephem-4-carboxylate;
7-[(Z)-2-(2-aminothiazol-4-yl)-2-((R)-1-carboxyeth-1-oxyimino)acetamido]-3-(2,7-diamino-1-methyl[1,2,4] triazolo[1,5-c]pyrimidinium-5-yl)thiomethyl-3-cephem-4-carboxylate;
7-[(Z)-2-(2-aminothiazol-4-yl)-2-((R)-1-carboxyeth-1-oxyimino)acetamido]-3-(7-amino-1,8-dimethyl[1,2,4] triazolo[1,5-c]pyrimidinium-5-yl)thiomethyl-3-cephem-4-carboxylate;
7-[(Z)-2-(2-aminothiazol-4-yl)-2-((R)-1-carboxyeth-1-oxyimino)acetamido]-3-(2,7-diamino-1,8-dimethyl-[1,2,4] triazolo[1,5-c]pyrimidinium-5-yl)thiomethyl-3-cephem-4-carboxylate;
7-[(Z)-2-(2-aminothiazol-4-yl)-2-(2-carboxyprop-2-oxyimino)acetamido]-3-(7-amino-1-methyl[1,3]imidazo-[1,2-c]pyrimidinium-5-yl)thiomethyl-3-cephem-4-carboxylate;
7-[(Z)-2-(2-aminothiazol-4-yl)-2-(2-carboxyprop-2-oxyimino)acetamido]-3-(7-methylamino-1-methyl[1,3] imidazo[1,2-c]pyrimidinium-5-yl)thiomethyl-3-cephem-4-carboxylate;
7-[(Z)-2-(2-aminothiazol-4-yl)-2-(2-carboxyprop-2-oxyimino)acetamido]-3-(7,8-diamino-1-methyl[1,3]-imidazo [1,2-c]pyrimidinium-5-yl)thiomethyl-3-cephem-4-carboxylate;
7-[(Z)-2-(2-aminothiazol-4-yl)-2-(2-carboxyprop-2-oxyimino)acetamido]-3-(7-amino-1,8-dimethyl[1,3]-imidazo [1,2-c]pyrimidinium-5-yl)thiomethyl-3-cephem-4-carboxylate;
7-[(Z)-2-(2-aminothiazol-4-yl)-2-(2-carboxyprop-2-oxyimino)acetamido]-3-(7-amino-1-ethyl[1,3]imidazo-[1,2-c]pyrimidinium-5-yl)thiomethyl-3-cephem-4-carboxylate;
7-[(Z)-2-(2-aminothiazol-4-yl)-2-(2-carboxyprop-2-oxyimino)acetamido]-3-(7,8-diamino-1-ethyl[1,3]-imidazo [1,2-c]pyrimidinium-5-yl)thiomethyl-3-cephem-4-carboxylate;
7-[(Z)-2-(2-aminothiazol-4-yl)-2-(2-carboxyprop-2-oxyimino)acetamido]-3-(7-amino-8-methyl-1-ethyl[1,3] imidazo[1,2-c]pyrimidinium-5-yl)thiomethyl-3-cephem-4-carboxylate;
7-[(Z)-2-(2-aminothiazol-4-yl)-2-(2-carboxyprop-2-oxyimino)acetamido]-3-(7-amino-1-methyl[1,3]imidazol-4-one[1,2-c]pyrimidinium-5-yl)thiomethyl-3-cephem-4-carboxylate;
7-[(Z)-2-(2-aminothiazol-4-yl)-2-((S)-1-carboxyeth-1-oxyimino)acetamido]-3-(7-amino-1-methyl[1,3]-imidazo[1,2-c]pyrimidinium-5-yl)thiomethyl-3-cephem-4-carboxylate;
7-[(Z)-2-(2-aminothiazol-4-yl)-2-((R)-1-carboxyeth-1-oxyimino)acetamido]-3-(7-amino-1-methyl[1,3]-imidazo[1,2-c]pyrimidinium-5-yl)thiomethyl-3-cephem-4-carboxylate;
7-[(Z)-2-(2-aminothiazol-4-yl)-2-((S)-1-carboxyeth-1-oxyimino)acetamido]-3-(7-amino-1,8-dimethyl[1,3]-imidazo [1,2-c]pyrimidinium-5-yl)thiomethyl-3-cephem-4-carboxylate;
7-[(Z)-2-(2-aminothiazol-4-yl)-2-((R)-1-carboxyeth-1-oxyimino)acetamido]-3-(7-amino-1,8-dimethyl[1,3]-imidazo [1,2-c]pyrimidinium-5-yl)thiomethyl-3-cephem-4-carboxylate;
7-[(Z)-2-(2-aminothiazol-4-yl)-2-((S)-1-carboxyeth-1-oxyimino)acetamido]-3-(7-amino-1-ethyl[1,3]imidazo-[1,2-c]pyrimidinium-5-yl)thiomethyl-3-cephem-4-carboxylate;
7-[(Z)-2-(2-aminothiazol-4-yl)-2-((R)-1-carboxyeth-1-oxyimino)acetamido]-3-(7-amino-1-ethyl[1,3]imidazo-[1,2-c]pyrimidinium-5-yl)thiomethyl-3-cephem-4-carboxylate;
7-[(Z)-2-(2-aminothiazol-4-yl)-2-((S)-1-carboxyeth-1-oxyimino)acetamido]-3-(7-amino-1-methyl[1,3]-imidazol-4-one[1,2-c]pyrimidinium-5-yl)thiomethyl-3-cephem-4-carboxylate;
7-[(Z)-2-(2-aminothiazol-4-yl)-2-((R)-1-carboxyeth-1-oxyimino)acetamido]-3-(7-amino-1-methyl[1,3]-imidazol-4-one[1,2-c]pyrimidinium-5-yl)thiomethyl-3-cephem-4-carboxylate;
7-[(Z)-2-(2-aminothiazol-4-yl)-2-(1-carboxymeth-1-oxyimino)acetamido]-3-(7-amino-1-methyl[1,3]imidazo-

[1,2-c]pyrimidinium-5-yl)thiomethyl-3-cephem-4-carboxylate;

7-[(Z)-2-(2-aminothiazol-4-yl)-2-(1-carboxymeth-1-oxyimino)acetamido]-3-(7-amino-1,8-dimethyl[1,3]-imidazo [1,2-c]pyrimidinium-5-yl)thiomethyl-3-cephem-4-carboxylate;

7-[(Z)-2-(2-aminothiazol-4-yl)-2-(1-carboxymeth-1-oxyimino)acetamido]-3-(7-amino-1-ethyl[1,3]imidazo-[1,2-c]pyrimidinium-5-yl)thiomethyl-3-cephem-4-carboxylate;

7-[(Z)-2-(2-aminothiazol-4-yl)-2-(1-carboxymeth-1-oxyimino)acetamido]-3-(7-amino-1-methyl[1,3]-imidazol-4-one[1,2-c]pyrimidinium-5-yl)thiomethyl-3-cephem-4-carboxylate;

7-[(Z)-2-(2-aminothiazol-4-yl)-2-(methoxyimino) acetamido]-3-(7-amino-1-methyl[1,3]imidazo[1,2-c] pyrimidinium-5-yl)thiomethyl-3-cephem-4-carboxylate;

7-[(Z)-2-(2-aminothiazol-4-yl)-2-(methoxyimino) acetamido]-3-(7-amino-1,8-dimethyl[1,3]imidazo[1,2-c] pyrimidinium-5-yl)thiomethyl-3-cephem-4-carboxylate;

7-[(Z)-2-(2-aminothiazol-4-yl)-2-(methoxyimino) acetamido]-3-(7-amino-1-ethyl[1,3]imidazo[1,2-c] pyrimidinium-5-yl)thiomethyl-3-cephem-4-carboxylate;

7-[(Z)-2-(2-aminothiazol-4-yl)-2-(methoxyimino) acetamido]-3-(7-amino-1-methyl[1,3]imidazol-4-one[1,2-c] pyrimidinium-5-yl)thiomethyl-3-cephem-4-carboxylate;

7-[(Z)-2-(2-aminothiazol-4-yl)-2-(ethoxyimino)acetamido]-3-(7-amino-1-methyl[1,3]imidazo[1,2-c]-pyrimidinium-5-yl) thiomethyl-3-cephem-4-carboxylate;

7-[(Z)-2-(2-aminothiazol-4-yl)-2-(ethoxyimino)acetamido]-3-(7-amino-1-ethyl[1,3]imidazo[1,2-c]-pyrimidinium-5-yl) thiomethyl-3-cephem-4-carboxylate;

7-[(Z)-2-(2-aminothiazol-4-yl)-2-(ethoxyimino)acetamido]-3-(7-amino-1-methyl[1,3]imidazol-4-one[1,2-c] pyrimidinium-5-yl)thiomethyl-3-cephem-4-carboxylate;

7-[(Z)-2-(2-aminothiazol-4-yl)-2-((R)-1-carboxyeth-1-oxyimino)acetamido]-3-(7-amino-1-methyl[1,3]-imidazo[1,2-c]pyrimidinium-5-yl)thiomethyl-3-cephem-4-carboxylate;

7-[(Z)-2-(2-aminothiazol-4-yl)-2-(1-carboxycyclopentoxyimino)acetamido]-3-(7-amino-1-methyl[1,3]-imidazo [1,2-c]pyrimidinium-5-yl)thiomethyl-3-cephem-4-carboxylate;

7-[(Z)-2-(2-aminothiazol-4-yl)-2-((R)-1-carboxyprop-1-oxyimino)acetamido]-3-(7-amino-1-methyl[1,3]-imidazo[1,2-c]pyrimidinium-5-yl)thiomethyl-3-cephem-4-carboxylate;

and

7-[(Z)-2-(2-aminothiazol-4-yl)-2-(2-carboxybut-2-oxyimino)acetamido]-3-(7-amino-1-methyl[1,3]imidazo-[1,2-c]pyrimidinium-5-yl)thiomethyl-3-cephem-4-carboxylate.

**4.** A process for preparing a compound of formula(I) which comprises reacting a compound of formula(II) with a compound of formula(III) in the presence of a solvent:

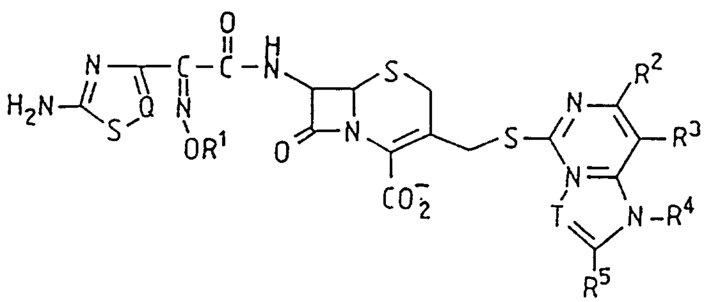

(I)

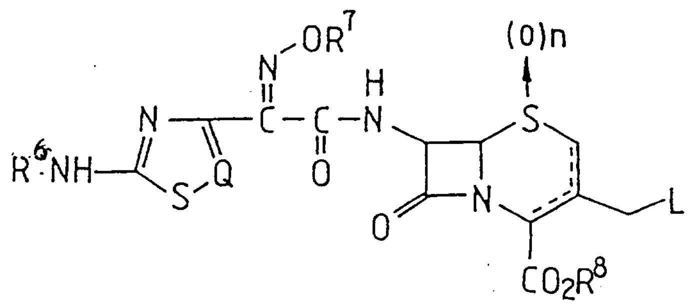

(II)

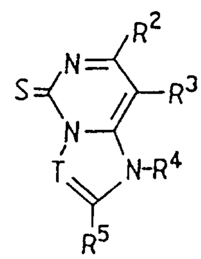

(III)

wherein:

| | |
|---|---|
| $R^1$, $R^2$, $R^3$, $R^4$, $R^5$, Q and T | have the same meanings as defined in claim 1; |
| $R^6$ is | a hydrogen or an amino protecting group; |
| $R^7$ is | a $C_{1-4}$ alkyl, $C_{3-4}$ alkenyl, $C_{3-4}$ alkynyl or $-C(R^a)(R^b)COO(R^c)$ wherein $R^a$ and $R^b$ are, independently of each other, a hydrogen or a $C_{1-4}$ alkyl group, respectively, or jointly, form a $C_{3-7}$ cycloalkyl group together with the carbon atoms to which they are attached and $R^c$ is a hydrogen or a carboxy protecting group, |
| $R^8$ is | a hydrogen or a carboxy protecting group; |
| n is | 0 or 1; and |
| L is | a leaving group. |

**5.** The process of claim 4 wherein the solvent is selected from the group consisting of N,N-dimethylformamide, N,N-dimethylacetamide, dimethylsulfoxide and methanol.

**6.** The process of claim 4 wherein the compound of the formula(III) is employed in an amount ranging from 0.5 to 2 molar equivalents based on the compound of the formula(II).

**7.** The process of claim 4 wherein the amino and/or carboxy protecting group is removed and/or the S-oxide is reduced before or after reacting the compound of formula(II) with the compound of formula(III).

**8.** A pharmaceutical composition comprising a therapeutically effective amount of the cephalosporin compound of claim 1 and a pharmaceutically acceptable carrier.

European Patent Office

# EUROPEAN SEARCH REPORT

Application Number

EP 93 11 2374

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,D | EP-A-0 397 511 (LUCKY LTD.)<br>* the whole document * | 1-3,8 | C07D501/00<br>A61K31/545 |
| Y | WO-A-9 203 445 (LUCKY LTD.)<br>* the whole document * | 1-3,8 | |
| Y,D | EP-A-0 150 507 (MOCHIDA PHARMACEUTICAL CO. LTD.)<br>* page 128, ex. 54-57; page 138, ex. 58 * | 1-3,8 | |
| A,P | EP-A-0 544 166 (F. HOFFMANN-LA ROCHE A.G.)<br>* examples * | 1-3,8 | |
| A,P | EP-A-0 508 375 (LUCKY LTD)<br>* the whole document * | 1-3,8 | |
| A | CHEMICAL ABSTRACTS, vol. 117, no. 9, 31 August 1992, Columbus, Ohio, US; abstract no. 90007u, Y. YOSHIMURA ET AL.<br>* abstract *<br>& J. ANTIBIOT. vol. 45, no. 5, 1992, pages 721 - 734 | 1-3,8 | TECHNICAL FIELDS SEARCHED (Int. Cl.5)<br>C07D<br>A61K |
| A | J. ANTIBIOT. vol. 31, no. 6, 1978, pages 546 - 560<br>H. BREUER ET AL.<br>* table I, compounds 1n, 1o * | 1-3,8 | |
| A,D | US-A-4 390 534 (TERAJI ET AL.)<br>* see, e.g., col. 41(6), col. 53(6), col. 106(106), col. 143(15), etc * | 1-3,8 | |

The present search report has been drawn up for all claims

| Place of search | Date of completion of the search | Examiner |
|---|---|---|
| BERLIN | 26 OCTOBER 1993 | FRELON D. L. M. G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)